# EUROPEAN PATENT APPLICATION

(11) **EP 1 717 227 A1**
(43) Date of publication of application: **02.11.2006**
(21) Application number: 04021314.2
(22) Date of filing: 08.09.2004
(51) Int. Cl.: C07D 209/18, C07D 401/12, C07D 405/12, C07D 409/12, C07D 413/12, C07D 513/04, A61K 31/404, A61K 31/422, A61K 31/429, A61K 31/4709, A61P 3/00, A61P 25/00

(54) **Substituted indole compounds, their preparation and use in medicaments**

(30) Priority: 10.08.2004 ES 200402007
(71) Applicant: LABORATORIOS DEL DR. ESTEVE, S.A., 08041 Barcelona (ES)
(72) Inventor: Merce Vidal, Ramon, 08021 Barcelona (ES); Dordl Zueras, Alberto, 08016 Barcelona (ES); Codony Soler, Xavier, 08301 Mataro (Barcelona) (ES)
(74) Representative: Davila Baz, Angel

(57) **Abstract**

The present invention relates to substituted indole compounds of general formula I, 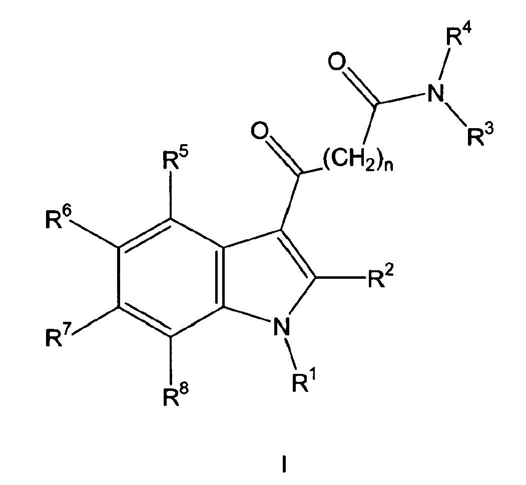 a process for their preparation, medicaments comprising substituted indole compounds as well as the use of substituted indole compounds for the preparation of medicaments, which are suitable e.g. for the prophylaxis and/or treatment of disorders or diseases that are at least partially mediated via 5-HT₆ receptors.

## Description

The present invention relates to substituted indole compounds of general formula I, 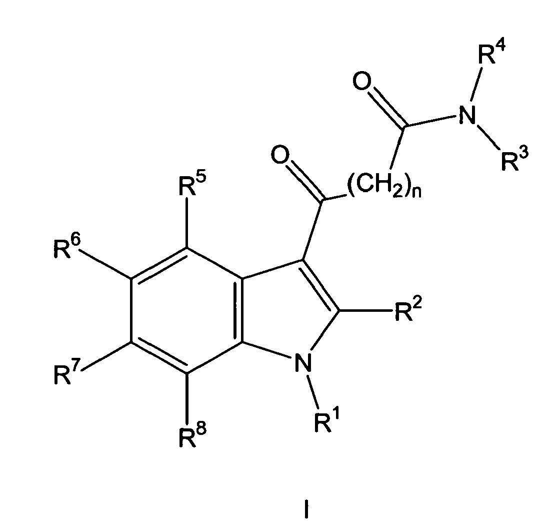 a process for their preparation, medicaments comprising substituted indole compounds as well as the use of substituted indole compounds for the preparation of medicaments, which are suitable e.g. for the prophylaxis and/or treatment of disorders or diseases that are at least partially mediated via 5-HT₆ receptors.

The superfamily of serotonin receptors (5-HT) includes 7 classes (5-HT₁-5-HT₇) encompassing 14 human subclasses [D. Hoyer, et al., Neuropharmacology, 1997, 36, 419]. The 5-HT₆ receptor is the latest serotonin receptor identified by molecular cloning both in rats [F.J. Monsma et al., Mol. Pharmacol., 1993, 43, 320; M. Ruat et al., Biochem. Biophys. Res. Commun., 1993, 193, 268] and in humans [R. Kohen, et al., J. Neurochem., 1996, 66, 47].

Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka et al., Ann. NY Acad. Sci., 1998, 861, 244; A. Bourson et al., Br. J. Pharmacol. , 1998, 125, 1562; D.C. Rogers et al., Br. J. Pharmacol. Suppl., 1999, 127, 22P; A. Bourson et al., J. Pharmacol. Exp. Ther. , 1995, 274, 173; A.J. Sleight, et al., Behav. Brain Res. , 1996, 73, 245; T.A. Branchek et al., Annu. Rev. Pharmacol. Toxicol. , 2000, 40, 319; C. Routledge et al., Br. J. Pharmacol. , 2000, 130, 1606]. It has been shown that typical and atypical antipsychotic drugs for treating schizophrenia have a high affinity for 5-HT₆ receptors [B.L. Roth et al., J. Pharmacol. Exp. Ther. , 1994, 268, 1403; C.E. Glatt et al., Mol. Med. , 1995, 1, 398; F.J. Mosma,et al., Mol. Pharmacol. , 1993, 43, 320; T. Shinkai et al., Am. J. Med. Genet. , 1999, 88, 120]. Compounds with 5-HT₆ receptor affinity are useful for treating infant hyperkinesia (ADHD, attention deficit / hyperactivity disorder) [W.D. Hirst et al., Br. J. Pharmacol. , 2000, 130, 1597; C. Gérard et al., Brain Research , 1997, 746, 207; M.R. Pranzatelli, Drugs of Today, 1997, 33, 379].

Moreover, it has been shown that the 5-HT₆ receptor also plays a role in food ingestion [Neuropharmacology, 41, 2001, 210-219].

Food ingestion disorders, particularly obesity, are a serious, fast growing threat to the health of humans of all age groups, since they increase the risk of developing other serious, even life-threatening diseases such as diabetes or coronary diseases as well.

US 2003/0181482 A1 discloses substituted indol-3-yl-oxoacetamido compounds, wherein different substituents like substituted aryl or heteroaryl radicals are bonded to the nitrogen atom of the indole ring system via a methylene group. These compounds reportedly show cytotoxic and anticancer activity as well as angiogenesis inhibitory activity.

An object of the present invention was to provide compounds that are suitable as active ingredients in medicaments, particularly in medicaments for the prophylaxis and/or treatment of disorders or diseases related to 5-HT₆ receptors.

Surprisingly, it has been found that the substituted indole compounds of general formulas I, Ia, Ib and Ic given below show good to excellent affinity for 5-HT₆₋receptors. These compounds are therefore particularly suitable as pharmacologically active agents in a medicament for the prophylaxis and/or treatment of disorders or diseases related to 5-HT₆-receptors.

Thus, in one aspect the present invention relates to substituted indole compounds of general formula I 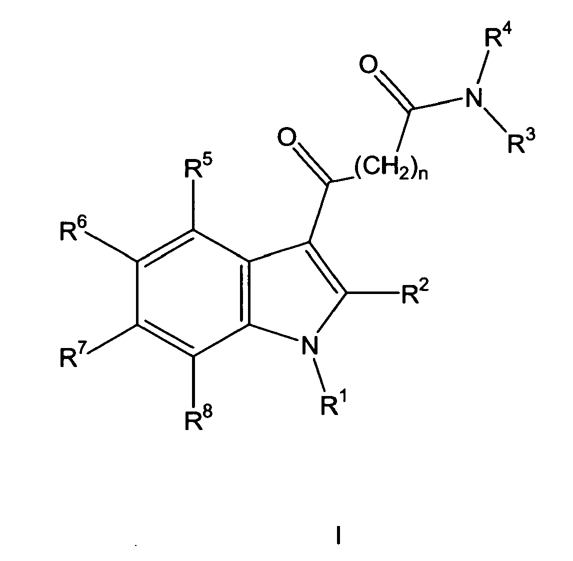 wherein
n represents 0, 1, 2, 3 or 4,
R¹ represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; an optionally at least mono-substituted aryl or heteroaryl radical, which may be bonded via a linear or branched alkylene group; -S(=O)₂-R⁹; or -C(=O)-R¹⁰,
for n = 0: R² represents -NO₂; -NH₂; -SH; -OH; -CN; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
for n = 1, 2, 3 or 4: R² represents -H, -NO₂; -NH₂; -SH; -OH; -CN; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R³ and R⁴, identical or different, represent a hydrogen atom; a linear or branched, saturated or unsaturated aliphatic radical, an optionally at least mono-substituted aryl or heteroaryl radical, which may be bonded via a linear or branched alkylene group; a saturated or unsaturated, optionally at least mono-substituted, optionally at least one heteroatom as a ring member containing cycloaliphatic radical, which may be bonded via a linear or branched alkylene group and/or which may be condensed with an optionally at least mono-substituted mono- or polycyclic ring system, or
R³ and R⁴ together with the bridging nitrogen form an optionally at least mono-substituted, saturated, unsaturated or aromatic heterocyclic ring that may contain at least one further heteroatom as a ring member and/or that may be condensed with an optionally at least mono-substituted mono- or polycyclic ring-system,
R⁵, R⁶, R⁷ and R⁸, identical or different, represent -H; -NO₂; -CN; -N(R¹¹)-S(=O)₂-R¹²; -OR¹³; -SR¹⁴; -C(=O)-OR¹⁵; -NR¹⁶R¹⁷; -C(=O)-R¹⁸; -(C=O)-NR¹⁹R²⁰; -O-(C=O)-R²¹; -S(=O)₂-R²²; -S(=O)₂-NR²³R²⁴; -NR²⁵-C(=O)-NR²⁶R²⁷; -NR²⁸⁻(C=O)-R²⁹; -NR³⁰-(C=O)-OR³¹; -NR³²-S(=O)NR³³R³⁴; or -S(=O)₂-OR³⁵; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
with the proviso that at least one of the substituents R⁵, R⁶, R⁷ and R⁸ represents an -N(R¹¹)-S(=O)₂-R¹² moiety,
R⁹ and R¹⁰, independent from one another, represent a linear or branched, saturated or unsaturated, optionally at least mono-substituted aliphatic radical; or an optionally at least mono-substituted aryl or heteroaryl radical, which may be bonded via a linear or branched alkylene group,
R¹¹ represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group; an optionally at least mono-substituted aryl or heteroaryl radical, which may be bonded via a linear or branched, optionally at least mono-substituted alkylene, alkenylene or alkinylene group; or an -S(=O)₂-R³⁶ moiety,
R¹² represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system; or a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system,
R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰, R³¹, R³², R³³, R³⁴ and R³⁵, independent from one another, represent a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R³⁶ represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system; or a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system,
optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Preferred are compounds of general formula I given above, wherein
n represents 0, 1, 2, 3 or 4,
R¹ represents a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆-alkylene group; an optionally at least mono-substituted 5- to 14- membered aryl or heteroaryl radical, which may be bonded via a linear or branched C₁₋₆-alkylene group; -S(=O)₂-R⁹; or -C(=O)-R¹⁰,
for n = 0: R² represents -NO₂; -NH₂; -SH; -OH; -CN; a halogen atom; a linear or branched, saturated or unsaturated, optionally at least mono-substituted, optionally at least one heteroatom as a chain member containing C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆-alkylene group; or an optionally at least mono-substituted 5- to 14-membered aryl or heteroaryl radical, which may be bonded via a linear or branched C₁₋₆-alkylene group,
for n = 1, 2, 3 or 4: R² represents -H; -NO₂; -NH₂; -SH; -OH; -CN; a halogen atom; a linear or branched, saturated or unsaturated, optionally at least mono-substituted, optionally at least one heteroatom as a chain member containing C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆-alkylene group; or an optionally at least mono-substituted 5- to 14-membered aryl or heteroaryl radical, which may be bonded via a linear or branched C₁₋₆-alkylene group,
R³ and R⁴, identical or different, represent a hydrogen atom; a linear or branched, saturated or unsaturated C₁₋₁₀ aliphatic radical; an optionally at least mono-substituted 5- to 14-membered aryl or heteroaryl radical, which may be bonded via a linear or branched C₁₋₆ alkylene group; a saturated or unsaturated, optionally at least mono-substituted, optionally at least one heteroatom as a ring member containg 3- to 9-membered cycloaliphatic radical, which may be bonded via a linear or branched C₁₋₆ alkylene group and/or which may be condensed with an optionally at least mono-substituted mono- or polycyclic ring system, or
R³ and R⁴ together with the bridging nitrogen form an optionally at least mono-substituted, saturated, unsaturated or aromatic 4- to 9-membered heterocyclic ring that may contain at least one further heteroatom as a ring member and/or that may be condensed with an optionally at least mono-substituted mono- or polycyclic ring-system,
R⁵, R⁶, R⁷ and R⁸, identical or different, represent -H; -NO₂; -CN; -N(R¹¹)-S(=O)₂-R¹²; -OR¹³; -SR¹⁴; -C(=O)-OR¹⁵; -NR¹⁶R¹⁷; -C(=O)-R¹⁸; -(C=O) -NR¹⁹R²⁰; -O-(C=O)-R²¹; -S(=O)₂-R²²; -S(=O)₂-NR²³R²⁴; -NR²⁵-C(=O)-NR²⁶R²⁷; -NR²⁸ -(C=O)-R²⁹; -NR³⁰-(C=O)-OR³¹; -NR³²-S(=O)NR³³R³⁴; or -S(=O)₂-OR³⁵; a halogen atom; a linear or branched, saturated or unsaturated, optionally at least mono-substituted, optionally at least one heteroatom as a chain member containing C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆-alkylene group,
with the proviso that at least one of the substituents R⁵, R⁶, R⁷ and R⁸ represents an -N(R¹¹)-S(=O)₂-R¹² moiety,
R⁹ and R¹⁰, independent from one another, represent a linear or branched, saturated or unsaturated, optionally at least mono-substituted C₁₋₁₀ aliphatic radical; or an optionally at least mono-substituted, 5- to 14-membered aryl or heteroaryl radical, which may be bonded via a linear or branched C₁₋₆-alkylene group,
R¹¹ represents a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group; an optionally at least mono-substituted 5- to 14 membered aryl or heteroaryl radical, which may be bonded via a linear or branched, optionally at least mono-substituted C₁₋₆ alkylene, C₂₋₆ alkenylene or C₂₋₆ alkinylene group; or an -S(=O)₂-R³⁶ moiety,
R¹² represents an optionally at least mono-substituted 5- to 14-membered aryl or heteroaryl radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system; or a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system,
R¹³-R³⁵, independent from one another, represent a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆ alkylene group,
R³⁶ represents an optionally at least mono-substituted 5- to 14-membered aryl or heteroaryl radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system; or a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system,
optionally in form of one of their stereoisomers, preferably enantiomers or diasteromers, a racemate or in form of a mixture of at least two of the stereoisomers, preferably enantiomers and/or diastereomers, in any mixing ratio, or a physiologically acceptable salt thereof, or a corresponding solvate thereof.

Also preferred are compounds of general formula I given above, wherein R¹ represents a hydrogen atom; a linear or branched C₁₋₁₀ alkyl radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members; -S(=O)₂-R⁹; or -C(=O)-R¹⁰,
preferably R¹ represents a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl;
an aryl or heteroaryl radical selected from the group consisting of phenyl, naphthyl, furyl (furanyl), thienyl (thiophenyl), pyrrolyl, oxazolyl,
isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl;

-S(=O)₂-R⁹; or -C(=O)-R¹⁰,

and n and R²-R³⁶ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such substituted indole compounds of general formula I given above are preferred, wherein
for n = 0
R² represents -NO₂; -NH₂; -SH; -OH; -CN; -CF₃; -OCH₃; -O-CH₂-CH₃; F; Cl; Br; I; a linear or branched, saturated or unsaturated C₁₋₁₀ aliphatic radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R² represents -NO₂; -NH₂; -SH; -OH; -CN; -CF₃; -OCH₃; -O-CH₂-CH₃; F; Cl; Br; I; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl, and
forn=1,2,3or4
R² represents -H; -NO₂; -NH₂; -SH; -OH; -CN; -CF₃; -OCH₃; -O-CH₂-CH₃; F; CI; Br; I; a linear or branched, saturated or unsaturated C₁₋₁₀ aliphatic radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R² represents -H; -NO₂; -NH₂; -SH; -OH; -CN; -CF₃; -OCH₃; -O-CH₂-CH₃; F; Cl; Br; I; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of linear or branched C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R² represents a hydrogen atom,
and n, R¹ and R³-R³⁶ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are substituted indole compounds of general formula I given above, wherein R³ and R⁴, identical or different, represent a hydrogen atom or a linear or branched C₁₋₈ alkyl radical, or
R³ and R⁴ together with the bridging nitrogen form an optionally at least mono-substituted, saturated, unsaturated or aromatic 4- to 9-membered heterocyclic ring that may be condensed with an optionally at least mono-substituted mono- or bicyclic ring-system,
whereby the rings of the ring system are 5- 6- or 7-membered and whereby the heterocyclic ring and one or both of the rings of the ring system may contain 1, 2 or 3 heteroatoms independently selected from the group consisting of nitrogen, oxygen and sulphur,
preferably
R³ and R⁴, identical or different, represent a hydrogen atom or an alkyl radical selected from the group consisting of methyl, ethyl, n-propyl, iso-propyl, n-butyl, sec-butyl, iso-butyl and tert-butyl; or
R³ and R⁴ together with the bridging nitrogen atom form a moiety selected from the group consisting of 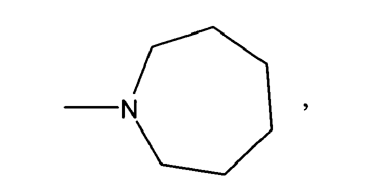 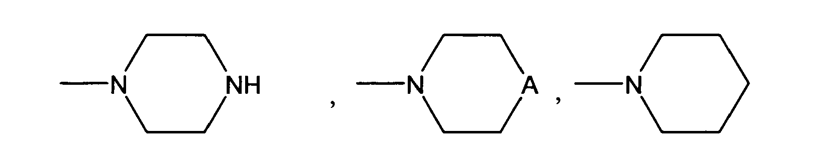 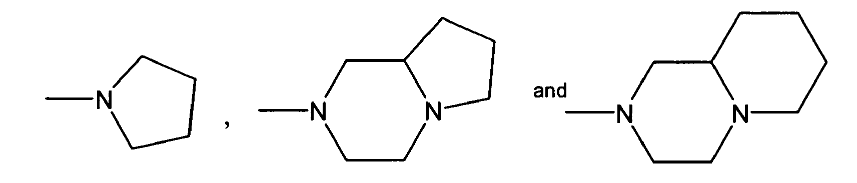 whereby A represents an oxygen atom or a sulphur atom and whereby each of these afore mentioned cyclic moieties may be substituted with 1, 2 or 3 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl in any position including the nitrogen atoms of the piperazine ring,
more preferably R³ and R⁴, identical or different, represent a hydrogen atom or an alkyl radical selected from the group consisting of methyl, ethyl, n-propyl, iso-propyl, n-butyl, sec-butyl, iso-butyl and tert-butyl,
and n, R¹, R² and R⁵-R³⁶ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such substituted indole compounds of general formula I given above are preferred, wherein
R⁵, R⁶, R⁷ and R⁸, identical or different, represent-H; -NO₂; -CN; -N(R¹¹)-S(=O)₂₋R¹²; -OR¹³; -SR¹⁴; -C(=O)-OR¹⁵; -NR¹⁶R¹⁷; -C(=O)-R¹⁸; -(C=O)-NR¹⁹R²⁰; -O-(C=O)-R²¹; -S(=O)₂-R²²; -S(=O)₂-NR²³R²⁴; -NR²⁵-C(=O)-NR²⁶R²⁷; -NR²⁸-(C=O)-R²⁹; -NR³⁰⁻(C=O)-OR³¹; -NR³²-S(=O)NR³³R³⁴; or -S(=O)₂-OR³⁵; F, Cl, Br, I; -CF₃, -CHF₂, -CH₂F, a linear or branched, saturated or unsaturated C₁₋₁₀ aliphatic radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R⁵, R⁶, R⁷ and R⁸, identical or different, represent -H; -NO₂; -CN; -N(R¹¹)-SO₂-R¹²; -OR¹³; -SR¹⁴; -C(=O)-OR¹⁵; -NR¹⁶R¹⁷; -C(=O)-R¹⁸; -(C=O)-NR¹⁹R²⁰; -O--(C=O)-R²¹; -S(=O)₂-R²²; -S(=O)₂-NR²³R²⁴; -NR²⁵-C(=O)-NR²⁶R²⁷; -NR²⁸-(C=O)-R²⁹; -NR³⁰-(C=O)-OR³¹; -NR³²-S(=O)NR³³R³⁴; or -S(=O)₂-OR³⁵; F, Cl, Br, I; -CF₃, -CHF₂, CH₂F, an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R⁵, R⁶, R⁷ and R⁸, identical or different, each represent -H; -NO₂; -CN; -N(R¹¹)-SO₂-R¹²; -OR¹³; -SR¹⁴; -C(=O)-OR¹⁵; -NR¹⁶R¹⁷; - F, Cl, Br, I; -CF₃, -CHF₂, -CH₂F, an alkyl radical selected from the group consisting of methyl, ethyl, n-propyl, iso-propyl, n-butyl, sec-butyl, iso-butyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
in each case with the proviso that at least one of the substituents R⁵, R⁶, R⁷ and R⁸ represents an -N(R¹¹)-S(=O)₂-R¹² moiety,
and n, R¹-R⁴ and R⁹-R³⁶ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Further preferred compounds of general formula I given above are such compounds, wherein one of the substituents R⁵, R⁶, R⁷ and R⁸ represents an -N(R¹¹)-S(=O)₂-R¹² moiety and the other three of these substituents have the meaning given above, preferably one of the substituents R⁵, R⁶, R⁷ and R⁸ represents an -N(R¹¹)-S(=O)₂₋R¹² moiety while the other three of these substituents each represent a hydrogen atom, and n, R¹-R⁴ and R⁹-R³⁶ - if present - have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such substituted indole compounds of general formula I given above are preferred, wherein
R⁹ and R¹⁰, independent from one another, represent a linear or branched, saturated or unsaturated C₁₋₁₀ aliphatic radical; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R⁹ and R¹⁰, independent from one another, represent an alkyl radical selected from the group consisting of methyl, ethyl, n-propyl, iso-propyl, n-butyl, sec-butyl, iso-butyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -0-(C=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
and n, R¹-R⁸ and R¹¹-R³⁶ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such substituted indole compounds of general formula I given above are preferred, wherein R¹¹ represents a hydrogen atom; a linear or branched, saturated or unsaturated, optionally at least mono-substituted C₁₋₁₀-aliphatic radical; a saturated or unsaturated, optionally at least mono-substituted, 3- to 9-membered cycloaliphatic radical, which may be bonded via a linear or branched C₁₋₆-alkylene, C₂₋₆-alkenylene or C₂₋₆-alkinylene group and/or which may contain 1, 2 or 3 heteroatoms independently selected from the group consisting of nitrogen, oxygen and sulphur as ring members; an optionally at least mono-substituted 5- to 10-membered aryl or heteroaryl radical, which may be bonded via a linear or branched C₁₋₆-alkylene, C₂₋₆-alkenylene or C₂₋₆-alkinylene group and wherein the heteroaryl radical contains 1, 2 or 3 heteroatoms independently selected from the group consisting of nitrogen, oxygen and sulphur as ring members; or an -S(=O)₂-R³⁶ moiety,
preferably R¹¹ represents a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or an -S(=O)₂-R³⁶-moiety,
more preferably R¹¹ represents a hydrogen atom,
and n, R¹-R¹⁰ and R¹²-R³⁶ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such substituted indole compounds of general formula I given above are preferred, wherein R¹² represents an optionally at least mono-substituted 5- to 10-membered aryl or heteroaryl radical, which may be bonded via a linear or branched, optionally at least mono-substituted C₁₋₆-alkylene, C₂₋₆-alkenylene or C₂₋₆-alkinylene group and/or which may be condensed with an optionally at least mono-substituted, saturated, unsaturated or aromatic, mono- or bicyclic ring system,
whereby the rings of the ring system are 5- 6- or 7-membered and whereby the heteroaryl radical and optionally one or both of the rings of the ring system contain 1, 2 or 3 heteroatoms independently selected from the group consisting of nitrogen, oxygen and sulphur,
or R¹² represents a saturated or unsaturated, optionally at least mono-substituted, 3-to 9-membered cycloaliphatic radical, which may be bonded via a linear or branched C₁₋₆-alkylene, C₂₋₆-alkenylene or C₂₋₆-alkinylene group and/or which may be condensed with an optionally at least mono-substituted mono- or polycyclic ring system,
whereby the rings of the ring system are 5- 6- or 7-membered and whereby the cycloaliphatic radical and optionally one or both of the rings of the ring system may contain 1, 2 or 3 heteroatoms independently selected from the group consisting of nitrogen, oxygen and sulphur,
preferably R¹² represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, 2-oxo-2,3-dihydro-benzooxazolyl, 2-oxo-2,3-dihydrobenzo[d]thiazolyl, benzooxazolinyl, benzothiazolinyl, benzimidazolidinyl, imidazo[2,1-b]thiazolyl, chromenyl and chromanyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅₋alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -CO-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R¹² represents an aryl or heteroaryl radical selected from the group consisting of phenyl, 1-naphthyl, 2-naphthyl, benzo[b]-thiophenyl, benzo[b]furanyl, quinolinyl, isoquinolinyl, imidazo[2,1-b]thiazolyl, 2-oxo-2,3-dihydro-benzooxazolyl and 2-oxo-2,3-dihydrobenzo[d]thiazolyl, whereby said aryl or heteroaryl radical may be substituted by 1, 2, 3, 4 or 5 substituents independently selected from the group consisting of methyl, ethyl, n-propyl, iso-propyl, n-butyl, sec-butyl, iso-butyl, tert-butyl, F, Cl, Br, I, -CN, -CF₃, -CF₂H, CFH₂, -C(=O)-O-CH₃, C(=O)-O-CH₂-CH₃, cyclopropyl, cyclobutyl, cyclopentyl, cyclohexyl, phenyl, phenoxy and benzyl,
and n, R¹-R¹¹ and R¹³-R³⁶ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such substituted indole compounds of general formula I given above, wherein R¹³-R³⁵ independent from one another, each represent a hydrogen atom; a linear or branched C₁₋₁₀ alkyl radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10-membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members;
preferably R¹³-R³⁵ independent from one another, represent a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or an aryl or heteroaryl radical selected from the group consisting of phenyl, naphthyl, furyl- (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
and n, R¹-R¹² and R³⁶ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such substituted indole compounds of general formula I given above are preferred, wherein R³⁶ represents an optionally at least mono-substituted 5- to 10- membered aryl or heteroaryl radical, which may be bonded via a linear or branched, optionally at least mono-substituted C₁₋₆-alkylene, C₂₋₆-alkenylene or C₂₋₆₋alkinylene group and/or which may be condensed with an optionally at least mono-substituted, saturated, unsaturated or aromatic, mono- or bicyclic ring system,
whereby the rings of the ring system are 5- 6- or 7-membered and whereby the heteroaryl radical and optionally one or both of the rings of the ring system contain 1, 2 or 3 heteroatoms independently selected from the group consisting of nitrogen, oxygen and sulphur,
or R³⁶ represents a saturated or unsaturated, optionally at least mono-substituted, 3-to 9-membered cycloaliphatic radical, which may be bonded via a linear or branched C₁₋₆-alkylene, C₂₋₆-alkenylene or C₂₋₆-alkinylene group and/or which may be condensed with an optionally at least mono-substituted mono- or polycyclic ring system,
whereby the rings of the ring system are 5- 6- or 7-membered and whereby the cycloaliphatic radical and optionally one or both of the rings of the ring system may contain 1, 2 or 3 heteroatoms independently selected from the group consisting of nitrogen, oxygen and sulphur,
preferably R³⁶ represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, 2-oxo-2,3-dihydro-benzooxazolyl, 2-oxo-2,3-dihydrobenzo[d]thiazolyl, benzooxazolinyl, benzothiazolinyl, benzimidazolidinyl, imidazo[2,1-b]thiazolyl, chromenyl and chromanyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅₋alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -CO-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1,2 or 3}- group and/or substituted with 1, 2, 3, 4 or 5 substituents independently selected from the group consisting C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
and n and R¹-R³⁵ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

In any of the foregoing definitions the following proviso may apply, namely that R³ and R⁴ do not both identically represent a hydrogen atom.

Another aspect of the present invention relates to a process for the preparation of a substituted indole compound of general formula I given above, according to which at least one compound of general formula II, 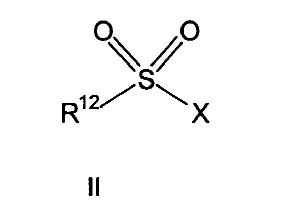 wherein R¹² has the meaning given above and X represents a leaving group, preferably a halogen atom, particularly preferably a chlorine atom, is reacted with at least one compound of general formula III, 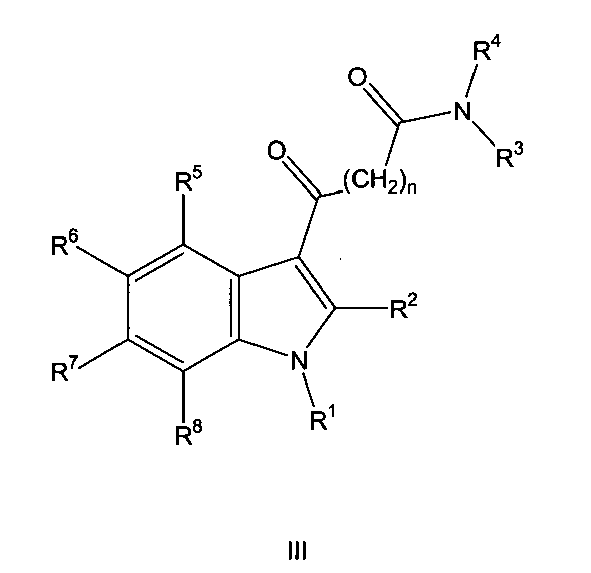 wherein R¹ to R⁴ and n have the meaning given above and R⁵, R⁶, R⁷ and R⁸ have the meaning given above with the proviso that at least one substituent of the group consisting of R⁵, R⁶, R⁷ and R⁸ represents an -N(R¹¹)(H) moiety or a protected derivative thereof, in a suitable reaction medium, preferably in the presence of at least one base and/or at least one auxiliary agent.

If a protected derivative is used, the respective protective group has to be removed after the reaction to obtain the desired substituted indole compound of general formula I. Suitable protecting groups as well as methods for introducing and removing such protecting groups are well known to those skilled in the art as described below.

Suitable reaction media for the reaction between compounds of general formulas (II) and (III) include organic solvents, such as dialkyl ether, preferably diethyl ether, or a cyclic ether, preferably tetrahydrofurane or dioxane; or a halogenated hydrocarbon, preferably dichloromethane or chloroform; an alcohol, preferably methanol or ethanol; a dipolar aprotic solvent, preferably acetonitrile, pyridine or dimethylformamide, or any other suitable reaction medium. Of course, mixtures of at least two classes of solvents or of at least two solvents of one class may also be used.

The reaction is preferably carried out in the presence of at least one suitable base, for example, an inorganic base such as a hydroxide or a carbonate of an alkali metal and/or an organic base, preferably triethylamine or pyridine.

The reaction is preferably carried out at a temperature between -10 °C and ambient temperature, i.e. approximately 25 °C and the reaction time is preferably between 5 minutes and 24 hours.

The compounds of general formula (I) given above may be purified and/or isolated according to methods well known to those skilled in the art. Preferably, the compounds of general formula (I) may be isolated by evaporating the reaction medium, addition of water and adjusting the pH value to obtain the compound in form of a solid that can be isolated by filtration, or by extraction with a solvent that is not miscible with water such as chloroform and purification by chromatography or recrystallisation from a suitable solvent.

The compounds of general formula (II) are commercially available or may be prepared according to methods well known in the art, for example, analogous to the methods described in the bibliography of E.E. Gilbert, Synthesis, 1969, 1, 3.

The compounds of general formula III are also either commercially available or can be produced according to methods known to those skilled in the art as for example described in Dillard et al., Journal of Medicinal Chemistry 1996, 39(26), 5119-5136 by reduction of the corresponding nitro derivatives which are either commercially available or can be produced according to methods known to those skilled in the art as - for example - described in the literature publications of Primofiore et al., Journal of Medicinal Chemistry 2001, 44(14), 2286-2297, Da Settimo et al. Generoso. Farmaco 1993, 48(2), 285-295, Da Settimo et al. Journal of Medicinal Chemistry 1996, 39(26), 5083-5091, Macor et al. Synthetic Communications 1993, 23(1), 65-72., Settimo et al. Gazzetta Chimica Italiana 1962, 92, 150-164, Primofiore et al.

Journal of Medicinal Chemistry 1989, 32(12), 2514-2518, Primofiore et al. European Journal of Medicinal Chemistry 1988, 23(4), 397-401.

The respective parts of the literature descriptions cited above are hereby incorporated by reference and form part of the disclosure.

Alternatively, the substituted indole compounds of general formula (I) given above, in which R¹¹ represents a linear or branched, saturated or unsaturated, optionally at least mono-substituted aliphatic radical; a saturated or unsaturated, optionally at least mono-substituted, optionally at least one heteroatom as a ring member containing cycloaliphatic radical, which is bonded via a linear or branched, optionally at least mono-substituted alkylene, alkenylene or alkinylene group; or an optionally at least mono-substituted aryl or heteroaryl radical, which is bonded via a linear or branched, optionally at least mono-substituted alkylene, alkenylene or alkinylene group and all of the other substituents have the above defined meaning, may also be prepared by an alkylation type reaction from the corresponding compound of general formula (I), in wherein R¹¹ represents a hydrogen atom and all of the other substituents have the meaning given above, e.g. by reaction with corresponding halide R¹¹-X, wherein X represents a halogen atom, preferably a chlorine atom, or a sulfate of the general formula IV 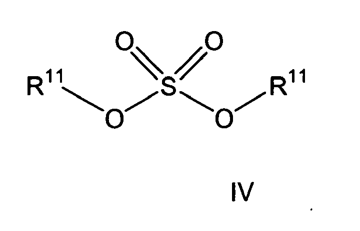 wherein in each case R¹¹ has the afore mentioned meaning. The corresponding halides and sulfates are commercially available or may be prepared according to methods well known to those skilled in the art.

The alkylation type reaction is preferably carried out in the presence of at least one suitable base such as a hydroxide and/or a carbonate of an alkali metal, a metal hydride, alcoxides such as sodium methoxide or potassium tert-butoxid, organometallic compounds such as n-butyllithium or tert-butyllithium, in the presence of a suitable reaction medium such as dialkyl ether, preferably diethyl ether, or a cyclic ether, preferably tetrahydrofurane or dioxane, a hydrocarbon, preferably toluene, an alcohol, preferably methanol or ethanol, a dipolar aprotic solvent, preferably acetonitrile, pyridine or dimethylformamide, or any other suitable reaction medium. Of course, mixtures of at least two classes of solvents or of at least two solvents of one class may also be used.

The reaction is preferably carried out at a temperature between -10 °C and ambient temperature, i.e. approximately 25 °C and the reaction time is preferably 1 and 24 hours.

The compounds of general formula (I) given above may be purified and/or isolated according to methods well known to those skilled in the art. Preferably, the compounds of general formula (I) may be isolated by evaporating the reaction medium, addition of water and then adjusting the pH value to obtain the compound in form of a solid that can be isolated by filtration, or by extraction with a solvent that is not miscible with water such as chloroform and purified by chromatography or recrystallisation from a suitable solvent.

During some synthetic reactions described above or while preparing the compounds of general formulas II or III the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.

If the substituted indole compounds of general formula (I) are obtained in form of a mixture of stereoisomers, particularly enantiomers or diastereomers, said mixtures may be separated by standard procedures known to those skilled in the art, e.g. chromatographic methods or crystallization with chiral reagents.

A further aspect of the present invention relates to a medicament comprising at least one substituted indole compound of general formula I given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, and optionally at least one auxiliary substance.

Said medicament is suitable for 5-HT₆-receptor regulation, particularly for the prophylaxis and/or treatment of a disorder or a disease that is least partially mediated via 5-HT₆-receptors.

Preferably said medicament is suitable for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity, or for the prophylaxis and/or treatment of irritable colon syndrome; disorders of the central nervous system; anxiety; panic attacks; depression; bipolar disorders; cognitive disorders; memory disorders; senile dementia; psychosis; neurodegenerative disorders, preferably selected from the group consisting of Morbus Alzheimer, Morbus Parkinson, Morbus Huntington and Multiple Sclerosis; schizophrenia; psychosis; or hyperactivity disorder (ADHD, attention deficit/hyperactivity disorder) or for the improvement of cognition (cognitive enhancement).

More preferably said medicament is suitable for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

Most preferably, said medicament is suitable for the prophylaxis and/or treatment of obesity and/or disorders or diseases related thereto.

In another aspect the present invention relates to the use of at least one substituted indole compound of general formula I given above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament suitable for 5-HT₆-receptor regulation, preferably for the prophylaxis and/or treatment of a disorder or a disease that is least partially mediated via 5-HT₆-receptors.

The use of at least one substituted indole compound of general formula I given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity, or for the prophylaxis and/or treatment of irritable colon syndrome; disorders of the central nervous system; anxiety; panic attacks; depression; bipolar disorders; cognitive disorders; memory disorders; senile dementia; psychosis; neurodegenerative disorders, preferably selected from the group consisting of Morbus Alzheimer, Morbus Parkinson, Morbus Huntington and Multiple Sclerosis; schizophrenia; psychosis; or hyperactivity disorder (ADHD, attention deficit/hyperactivity disorder) or for improvement of cognition (cognitive enhancement) is preferred.

More preferred is the use of at least one substituted indole compound of general formula I as defined above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

Most preferred is the use of at least one substituted indole compound of general formula I as defined above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for for the prophylaxis and/or treatment of obesity and/or disorders or diseases related thereto.

In yet another aspect the present invention relates to substituted indole compounds of general formula Ic 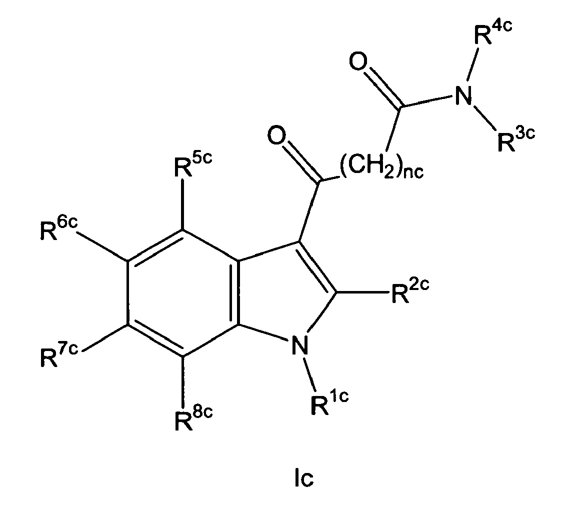 wherein
nc represents 0, 1, 2, 3 or 4,
R^{1c} represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; an optionally at least mono-substituted aryl or heteroaryl radical, which may be bonded via a linear or branched alkylene group; -S(=O)₂-R^{9c}; or -C(=O)-R^{10c},
R^{2c} represents -H, -NO₂; -NH₂; -SH; -OH; -CN; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R^{3c} and R^{4c}, identical or different, represent a hydrogen atom; a linear or branched, saturated or unsaturated aliphatic radical, an optionally at least mono-substituted aryl or heteroaryl radical, which may be bonded via a linear or branched alkylene group; a saturated or unsaturated, optionally at least mono-substituted, optionally at least one heteroatom as a ring member containing cycloaliphatic radical, which may be bonded via a linear or branched alkylene group and/or which may be condensed with an optionally at least mono-substituted mono- or polycyclic ring system, or
R^{3c} and R^{4c} together with the bridging nitrogen form an optionally at least mono-substituted, saturated, unsaturated or aromatic heterocyclic ring that may contain at least one further heteroatom as a ring member and/or that may be condensed with an optionally at least mono-substituted mono- or polycyclic ring-system,
R^{5c}, R^{6c}, R^{7c} and R^{8c}, identical or different, represent -H; -NO₂; -CN; -N(R^{11c})-S(=O)₂-R^{12c}; -OR^{13c}; -SR^{14c}; -C(=O)-OR^{15c}; -NR^{16c}R^{17c}; -C(=O)-R^{18c}; -(C=O)-NR^{19c}R^{20c}; -O-(C=O)-R^{21c}; -S(=O)₂-R^{22c}; -S(=O)₂-NR^{23c}R^{24c}; -NR^{25c}-C(=O)-NR^{26c}R^{27c}; -NR^{28c}-(C=O)-R^{29c}; -NR^{30c}-(C=O)-OR^{31c}; -NR^{32c}-S(=O)NR^{33c}R^{34c}; or -S(=O)₂-OR^{35c}; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
with the proviso that one of the substituents R^{5c}, R^{6c}, R^{7c} and R^{8c} represents an -N(R^{11c})-S(=O)₂-R^{12c} moiety,
R^{9c} and R^{10c}, independent from one another, represent a linear or branched, saturated or unsaturated, optionally at least mono-substituted aliphatic radical; or an optionally at least mono-substituted aryl or heteroaryl radical, which may be bonded via a linear or branched alkylene group,
R^{11c} represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group; an optionally at least mono-substituted aryl or heteroaryl radical, which may be bonded via a linear or branched, optionally at least mono-substituted alkylene, alkenylene or alkinylene group; or an -S(=O)₂-R^{36c} moiety,
R^{12c} represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system; or a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system,
R^{13c}, R^{14c}, R^{15c}, R^{16c}, R^{17c}, R^{18c}, R^{19c}, R^{20c}, R^{21c}, R^{22c}, R^{23c}, R^{24c}, R^{25c}, R^{26c}, R^{27c}, R^{28c}, R^{29c}, R^{30c}, R^{31c}, R^{32c}, R^{33c}, R^{34c} and R^{35c}, independent from one another, represent a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R^{36c} represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system; or a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system,
optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Preferred are compounds of general formula Ic given above, wherein
nc represents 0, 1, 2, 3 or 4,
R^{1c} represents a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆-alkylene group; an optionally at least mono-substituted 5- to 14- membered aryl or heteroaryl radical, which may be bonded via a linear or branched C₁₋₆-alkylene group; -S(=O)₂-R^{9c}; or -C(=O)-R^{10c},
R^{2c} represents -H; -NO₂; -NH₂; -SH; -OH; -CN; a halogen atom; a linear or branched, saturated or unsaturated, optionally at least mono-substituted, optionally at least one heteroatom as a chain member containing C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆-alkylene group; or an optionally at least mono-substituted 5- to 14-membered aryl or heteroaryl radical, which may be bonded via a linear or branched C₁₋₆-alkylene group,
R^{3c} and R^{4c}, identical or different, represent a hydrogen atom; a linear or branched, saturated or unsaturated C₁₋₁₀ aliphatic radical; an optionally at least mono-substituted 5- to 14-membered aryl or heteroaryl radical, which may be bonded via a linear or branched C₁₋₆ alkylene group; a saturated or unsaturated, optionally at least mono-substituted, optionally at least one heteroatom as a ring member containg 3- to 9-membered cycloaliphatic radical, which may be bonded via a linear or branched C₁₋₆ alkylene group and/or which may be condensed with an optionally at least mono-substituted mono- or polycyclic ring system, or
R^{3c} and R^{4c} together with the bridging nitrogen form an optionally at least mono-substituted, saturated, unsaturated or aromatic 4- to 9-membered heterocyclic ring that may contain at least one further heteroatom as a ring member and/or that may be condensed with an optionally at least mono-substituted mono- or polycyclic ring-system,
R^{5c} R^{6c}, R^{7c} and R^{8c}, identical or different, represent-H; -NO₂; -CN; -N(R^{11c})-S(=O)₂-R^{12c}; -OR^{13c}; -SR^{14c}; -C(=O)-OR^{15c}; -NR^{16c}R^{17c}; -C(=O)-R^{18c}; -(C=O)-NR^{19c}R^{20c}; -O-(C=O)-R^{21c}; -S(=O)₂-R^{22c}; -S(=O)₂-NR^{23c}R^{24c}; -NR^{25c}-C(=O)-NR^{26c}R^{27c}; -NR^{28c}-(C=O)-R^{29c}; -NR^{30c}-(C=O)-OR^{31c}; -NR^{32C}-S(=O)NR^{33c}R^{34c}; or -S(=O)₂-OR^{35c}; a halogen atom; a linear or branched, saturated or unsaturated, optionally at least mono-substituted, optionally at least one heteroatom as a chain member containing C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆-alkylene group,
with the proviso that one of the substituents R^{5c}, R^{6c}, R^{7c} and R^{8c} represents an -N(R^{11c})-S(=O)₂-R^{12c} moiety,
R^{9c} and R^{10c}, independent from one another, represent a linear or branched, saturated or unsaturated, optionally at least mono-substituted C₁₋₁₀ aliphatic radical; or an optionally at least mono-substituted, 5- to 14-membered aryl or heteroaryl radical, which may be bonded via a linear or branched C₁₋₆-alkylene group,
R^{11c} represents a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group; an optionally at least mono-substituted 5- to 14 membered aryl or heteroaryl radical, which may be bonded via a linear or branched, optionally at least mono-substituted C₁₋₆ alkylene, C₂₋₆ alkenylene or C₂₋₆ alkinylene group; or an -S(=O)₂-R^{36c} moiety,
R^{12c} represents an optionally at least mono-substituted 5- to 14-membered aryl or heteroaryl radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system; or a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system,
R^{13c}-R^{35c}, independent from one another, represent a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆ alkylene group,
R^{36c} represents an optionally at least mono-substituted 5- to 14-membered aryl or heteroaryl radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system; or a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system,
optionally in form of one of their stereoisomers, preferably enantiomers or diasteromers, a racemate or in form of a mixture of at least two of the stereoisomers, preferably enantiomers and/or diastereomers, in any mixing ratio, or a physiologically acceptable salt thereof, or a corresponding solvate thereof.

Also preferred are compounds of general formula Ic given above, wherein R^{1c} represents a hydrogen atom; a linear or branched C₁₋₁₀ alkyl radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members; -S(=O)₂-R^{9c}; or -C(=O)-R^{10c},
preferably R^{1c} represents a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; -S(=O)₂-R^{9c}; or -C(=O)-R^{10c},
more preferably R^{1c} represents a hydrogen atom,
and nc and R^{2c}-R^{36c} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.
Furthermore, such substituted indole compounds of general formula Ic given above are preferred, wherein
R^{2c} represents -H; -NO₂; -NH₂; -SH; -OH; -CN; -CF₃; -OCH₃; -O-CH₂-CH₃; F; Cl; Br; I; a linear or branched, saturated or unsaturated C₁₋₁₀ aliphatic radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R^{2c} represents -H; -NO₂; -NH₂; -SH; -OH; -CN; -CF₃; -OCH₃; -O-CH₂-CH₃; F; CI; Br; I; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of linear or branched C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R^{2c} represents a hydrogen atom,
and nc, R^{1c} and R^{3c}-R^{36c} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are substituted indole compounds of general formula Ic given above, wherein R^{3c} and R^{4c}, identical or different, represent a hydrogen atom or a linear or branched C₁₋₈ alkyl radical, or
R^{3c} and R^{4c} together with the bridging nitrogen form an optionally at least mono-substituted, saturated, unsaturated or aromatic 4- to 9-membered heterocyclic ring that may be condensed with an optionally at least mono-substituted mono- or bicyclic ring-system,
whereby the rings of the ring system are 5- 6- or 7-membered and whereby the heterocyclic ring and one or both of the rings of the ring system may contain 1, 2 or 3 heteroatoms independently selected from the group consisting of nitrogen, oxygen and sulphur,
preferably
R^{3c} and R^{4c}, identical or different, represent a hydrogen atom or an alkyl radical selected from the group consisting of methyl, ethyl, n-propyl, iso-propyl, n-butyl, sec-butyl, iso-butyl and tert-butyl; or
R^{3c} and R^{4c} together with the bridging nitrogen atom form a moiety selected from the group consisting of 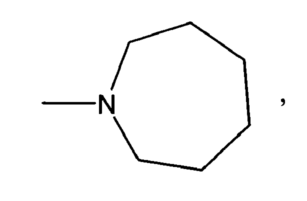 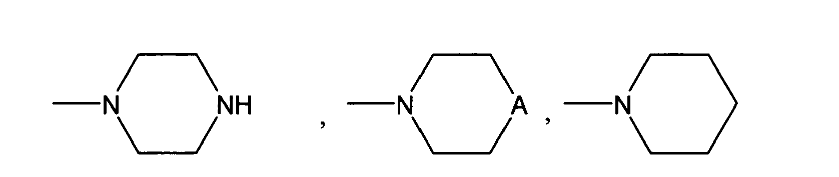 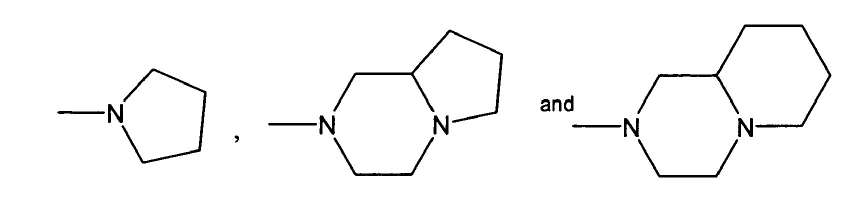 whereby A represents an oxygen atom or a sulphur atom and whereby each of these afore mentioned cyclic moieties may be substituted with 1, 2 or 3 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl in any position including the nitrogen atoms of the piperazine ring,
more preferably R^{3c} and R^{4c}, identical or different, represent a hydrogen atom or an alkyl radical selected from the group consisting of methyl, ethyl, n-propyl, iso-propyl, n-butyl, sec-butyl, iso-butyl and tert-butyl,
and nc, R^{1c}, R^{2c} and R^{5c}-R^{36c} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such substituted indole compounds of general formula Ic given above are preferred, wherein
R^{5c}, R^{6c}, R^{7c} and R^{8c}, identical or different, represent -H; -NO₂; -CN; -N(R^{11c})-S(=O)₂-R^{12c}; -OR^{13c}; -SR^{14c}; -C(=O)-OR^{15c}; -NR^{16c}R^{17c}; -C(=O)-R^{18c}; -(C=O)-NR^{19c}R^{20c}; -O-(C=O)-R^{21c}; -S(=O)₂-R^{22c}; -S(=O)₂-NR^{23c}R^{24c}; -NR^{25c}C(=O)-NR^{26c}R^{27c}; -NR^{28c}-(C=O)-R^{29c}; -NR^{30c}-(C=O)-OR^{31c}; -NR^{32c}-S(=O)NR^{33c}R^{34c}; or -S(=O)₂-OR^{35c}; F, Cl, Br, I; -CF₃, -CHF₂, -CH₂F, a linear or branched, saturated or unsaturated C₁₋₁₀ aliphatic radical; a saturated or unsaturated, optionally at least mono-substituted, 3-to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R^{5c}, R^{6c}, R^{7c} and R^{8c}, identical or different, represent -H; -NO₂; -CN; -N(R^{11c})-SO₂-R^{12c}; -OR^{13c}; -SR^{14c}; -C(=O)-OR^{15c}; -NR^{16c}R^{17c}; -C(=O)-R^{18c}; -(C=O)-NR^{19c}R^{20c}; -O-(C=O)-R^{21c}; -S(=O)₂-R^{22c}; -S(=O)₂-NR^{23c}R^{24c}; -NR^{25c}-C(=O)-NR^{26c}R^{27c}; -NR^{28c}-(C=O)-R^{29c}; -NR^{30c}-(C=O)-OR^{31c}; -NR^{32c}-S(=O)NR^{33c}R^{34c}; or -S(=O)₂-OR^{35c}; F, Cl, Br, I; -CF₃, -CHF₂, -CH₂F, an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R^{5c}, R^{6c}, R^{7c} and R^{8c}, identical or different, each represent -H; -NO₂; -CN; -N(R^{11c})-SO₂-R^{12c}; -OR^{13c}; -SR^{14c}; -C(=O)-OR^{15c}; -NR^{16c}R^{17c}; - F, Cl, Br, I; -CF₃, -CHF₂, -CH₂F, an alkyl radical selected from the group consisting of methyl, ethyl, n-propyl, iso-propyl, n-butyl, sec-butyl, iso-butyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
in each case with the proviso that one of the substituents R^{5c}, R^{6c}, R^{7c} and R^{8c} represents an -N(R^{11c})-S(=O)₂-R^{12c} moiety,
and nc, R^{1c}-R^{4c} and R^{9c}-R^{36c} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Further preferred substituted indole compounds of general formula Ic given above are such compounds, wherein one of the substituents R^{5c}, R^{6c}, R^{7c} and R^{8c} represents an -N(R^{11c})-S(=O)₂-R^{12c} moiety while the other three of these substituents each represent a hydrogen atom, and nc, R^{1c}-R^{4c}, R^{9c}-R^{12c} and R^{36c} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such substituted indole compounds of general formula Ic given above are preferred, wherein
R^{9c} and R^{10c}, independent from one another, represent a linear or branched, saturated or unsaturated C₁₋₁₀ aliphatic radical; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R^{9c} and R^{10c}, independent from one another, represent an alkyl radical selected from the group consisting of methyl, ethyl, n-propyl, iso-propyl, n-butyl, sec-butyl, iso-butyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
and nc, R^{1c}-R^{8c} and R^{11c}-R^{36c} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such substituted indole compounds of general formula Ic given above are preferred, wherein R^{11c} represents a hydrogen atom; a linear or branched, saturated or unsaturated, optionally at least mono-substituted C₁₋₁₀-aliphatic radical; a saturated or unsaturated, optionally at least mono-substituted, 3- to 9-membered cycloaliphatic radical, which may be bonded via a linear or branched C₁₋₆-alkylene, C₂₋₆-alkenylene or C₂₋₆-alkinylene group and/or which may contain 1, 2 or 3 heteroatoms independently selected from the group consisting of nitrogen, oxygen and sulphur as ring members; an optionally at least mono-substituted 5- to 10-membered aryl or heteroaryl radical, which may be bonded via a linear or branched C₁₋₆-alkylene, C₂₋₆-alkenylene or C₂₋₆-alkinylene group and wherein the heteroaryl radical contains 1, 2 or 3 heteroatoms independently selected from the group consisting of nitrogen, oxygen and sulphur as ring members; or an -S(=O)₂-R^{36c} moiety,
preferably R^{11c} represents a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or an -S(=O)₂-R^{36c}-moiety,
more preferably R^{11c} represents a hydrogen atom,
and nc, R^{1c}-R^{10c} and R^{12c}-R^{36c} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such substituted indole compounds of general formula Ic given above are preferred, wherein R^{12c} represents an optionally at least mono-substituted 5- to 10-membered aryl or heteroaryl radical, which may be bonded via a linear or branched, optionally at least mono-substituted C₁₋₆-alkylene, C₂₋₆-alkenylene or C₂₋₆-alkinylene group and/or which may be condensed with an optionally at least mono-substituted, saturated, unsaturated or aromatic, mono- or bicyclic ring system,
whereby the rings of the ring system are 5- 6- or 7-membered and whereby the heteroaryl radical and optionally one or both of the rings of the ring system contain 1, 2 or 3 heteroatoms independently selected from the group consisting of nitrogen, oxygen and sulphur,
or R^{12c} represents a saturated or unsaturated, optionally at least mono-substituted, 3-to 9-membered cycloaliphatic radical, which may be bonded via a linear or branched C₁₋₆-alkylene, C₂₋₆-alkenylene or C₂₋₆-alkinylene group and/or which may be condensed with an optionally at least mono-substituted mono- or polycyclic ring system,
whereby the rings of the ring system are 5- 6- or 7-membered and whereby the cycloaliphatic radical and optionally one or both of the rings of the ring system may contain 1, 2 or 3 heteroatoms independently selected from the group consisting of nitrogen, oxygen and sulphur,
preferably R^{12c} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, 2-oxo-2,3-dihydro-benzooxazolyl, 2-oxo-2,3-dihydrobenzo[d]thiazolyl, benzooxazolinyl, benzothiazolinyl, benzimidazolidinyl, imidazo[2,1-b]thiazolyl, chromenyl and chromanyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅₋alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -CO-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R^{12c} represents an aryl or heteroaryl radical selected from the group consisting of phenyl, 1-naphthyl, 2-naphthyl, benzo[b]-thiophenyl, benzo[b]furanyl, quinolinyl, isoquinolinyl, imidazo[2,1-b]thiazolyl, 2-oxo-2,3-dihydro-benzooxazolyl and 2-oxo-2,3-dihydrobenzo[d]thiazolyl, whereby said aryl or heteroaryl radical may be substituted by 1, 2, 3, 4 or 5 substituents independently selected from the group consisting of methyl, ethyl, n-propyl, iso-propyl, n-butyl, sec-butyl, iso-butyl, tert-butyl, F, Cl, Br, I, -CN, -CF₃, -CF₂H, CFH₂, -C(=O)-O-CH₃, C(=O)-O-CH₂-CH₃, cyclopropyl, cyclobutyl, cyclopentyl, cyclohexyl, phenyl, phenoxy and benzyl,
and nc, R^{1c}-R^{11c} and R^{13c}-R^{36c} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such substituted indole compounds of general formula Ic given above, wherein R^{13c}-R^{35c} independent from one another, each represent a hydrogen atom; a linear or branched C₁₋₁₀ alkyl radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10-membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members;
preferably R^{13c}-R^{35c} independent from one another, represent a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl- (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
and nc, R^{1c}-R^{12c} and R^{36c} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such substituted indole compounds of general formula Ic given above are preferred, wherein R^{36c} represents an optionally at least mono-substituted 5- to 10- membered aryl or heteroaryl radical, which may be bonded via a linear or branched, optionally at least mono-substituted C₁₋₆-alkylene, C₂₋₆-alkenylene or C₂₋₆₋alkinylene group and/or which may be condensed with an optionally at least mono-substituted, saturated, unsaturated or aromatic, mono- or bicyclic ring system,
whereby the rings of the ring system are 5- 6- or 7-membered and whereby the heteroaryl radical and optionally one or both of the rings of the ring system contain 1, 2 or 3 heteroatoms independently selected from the group consisting of nitrogen, oxygen and sulphur,
or R^{36c} represents a saturated or unsaturated, optionally at least mono-substituted, 3-to 9-membered cycloaliphatic radical, which may be bonded via a linear or branched C₁₋₆-alkylene, C₂₋₆-alkenylene or C₂₋₆-alkinylene group and/or which may be condensed with an optionally at least mono-substituted mono- or polycyclic ring system,
whereby the rings of the ring system are 5- 6- or 7-membered and whereby the cycloaliphatic radical and optionally one or both of the rings of the ring system may contain 1, 2 or 3 heteroatoms independently selected from the group consisting of nitrogen, oxygen and sulphur,
preferably R^{36c} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, 2-oxo-2,3-dihydro-benzooxazolyl, 2-oxo-2,3-dihydrobenzo[d]thiazolyl, benzooxazolinyl, benzothiazolinyl, benzimidazolidinyl, imidazo[2,1-b]thiazolyl, chromenyl and chromanyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅₋alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -CO-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
and nc and R^{1c}-R^{35c} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such substituted indole compounds of general formula Ic given above are preferred, wherein nc is 0 and R^{1c}-R^{36c} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

In any of the foregoing definitions the following proviso may apply, namely that R^{3c} and R^{4c} do not both identically represent a hydrogen atom.

Particularly preferred are substituted indole compound of general formula Ic, wherein
nc is 0,
R^{1c} represents a hydrogen atom,
R^{2c} represents a hydrogen atom,
R^{3c} and R^{4c}, identical or different, represent an alkyl radical selected from the group consisting of methyl, ethyl, n-propyl, iso-propyl, n-butyl, sec-butyl, iso-butyl and tert-butyl,
one of the substituents R^{5c}, R^{6c}, R^{7c} and R^{8c} represents an -N(R^{11c})-S(=O)-R^{12c-}moiety while the other three of these substituents each represent a hydrogen atom,
R^{11c} represents a hydrogen atom,
R^{12c} represents an aryl or heteroaryl radical selected from the group consisting of phenyl, 1-naphthyl, 2-naphthyl, benzo[b]-thiophenyl, benzo[b]furanyl, quinolinyl, isoquinolinyl, imidazo[2,1-b]thiazolyl, 2-oxo-2,3-dihydro-benzooxazolyl and 2-oxo-2,3-dihydrobenzo[d]thiazolyl, whereby said aryl or heteroaryl radical may be substituted by 1, 2, 3, 4 or 5 substituents independently selected from the group consisting of methyl, ethyl, n-propyl, iso-propyl, n-butyl, sec-butyl, iso-butyl, tert-butyl, F, CI, Br, I, -CN, -CF₃, -CF₂H, CFH₂, -C(=O)-O-CH₃, C(=O)-O-CH₂-CH₃, cyclopropyl, cyclobutyl, cyclopentyl, cyclohexyl, phenyl, phenoxy and benzyl.

Most preferred are substituted indole compound of general formula Ic selected from the group consisting of
[1] 2-[5-(5-Chloro-3-methyl-benzo[b]thiophene-2-sulfonylamino)-1H-indol-3-yl]-N, N-diethyl-2-oxoacetamide,
[2] N,N-Diethyl-2-[5-(naphthalene-2-sulfonylamino)-1H-indol-3-yl]-2-oxo-acetamide,
[3] N,N-Diethyl-2-[5-(naphthalene-1-sulfonylamino)-1H-indol-3-yl]-2-oxo-acetamide,
[4] 2-[5-(Biphenyl-4-sulfonylamino)-1H-indol-3-yl]-N,N-diethyl-2-oxo-acetamide,
[5] N,N-Diethyl-2-oxo-2-[5-(quinoline-8-sulfonylamino)-1H-indol-3-yl]-acetamide,
[6] N,N-Dimethyl-2-[5-(naphthalene-2-sulfonylamino)-1H-indol-3-yl]-2-oxo-acetamide,
[7] N,N-Dimethyl-2-[5-(naphthalene-1-sulfonylamino)-1H-indol-3-yl]-2-oxo-acetamide,
[8] 2-[5-(5-Chloro-3-methyl-benzo[b]thiophene-2-sulfonylamino)-1H-indol-3-yl]-N,N-dimethyl-2-oxo-acetamide,
[9] 2-[5-(6-Chloro-imidazo[2,1-b]thiazole-5-sulfonylamino)-1H-indol-3-yl]-N, N-diethyl-2-oxo-acetamide,
[10] 2-[5-(6-Chloro-imidazo[2,1-b]thiazole-5-sulfonylamino)-1H-indol-3-yl]-N,N-dimethyl-2-oxo-acetamide,
[11] N,N-Dimethyl-2-[4-(naphthalene-1-sulfonylamino)-1H-indol-3-yl]-2-oxo-acetamide,
[12] 2-[4-(5-Chloro-3-methyl-benzo[b]thiophene-2-sulfonylamino)-1H-indole-3-yl]-N, N-dimethyl-2-oxo-acetamide,
[13] 2-[4-(6-Chloro-imidazo[2,1-b]thiazole-5-sulfonylamino)-1H-indol-3-yl]-N,N-dimethyl-2-oxo-acetamide,
[14] N,N-Dimethyl-2-[5-[(4-fluoro-3-methyl-phenyl)-1-sulfonylamino]-1H-indol-3-yl]-2-oxo-acetamide,
[15] 5-(3-Dimethylaminooxalyl-1H-indol-5-ylsulfamoyl)-3-methyl-benzofuran-2-carboxylic acid ethyl ester,
[16] 2-[5-(Biphenyl-4-sulfonylamino)-1H-indol-3-yl]-N,N-dimethyl-2-oxo-acetamide,
[17] N,N-Dimethyl-2-oxo-2-[5-(2-oxo-2,3-dihydro-benzoxazole-6-sulfonylamino)-1H-indol-3-yl]-acetamide,
[18] N,N-Dimethyl-2-oxo-2-[5-(2-oxo-2,3-dihydrobenzo[d]thiazole-6-sulfonamido)-1H-indol-3-yl]acetamide,
[19] 2-[5-[(4-Cyclohexyl-phenyl)-1-sulfonylamino]-1H-indol-3-yl]-N,N-dimethyl-2-oxo-acetamide and
[20] N,N-Dimethyl-2-[5-[(4-phenoxy-phenyl)-1-sulfonylamino]-1H-indol-3-yl]-2-oxo-acetamide.

Another aspect of the present invention relates to a process for the preparation of a substituted indole compound of general formula Ic given above, according to which at least one compound of general formula IIc, 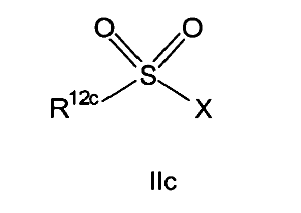 wherein R^{12c} has the meaning given above and X represents a leaving group, preferably a halogen atom, particularly preferably a chlorine atom, is reacted with at least one compound of general formula IIIc, 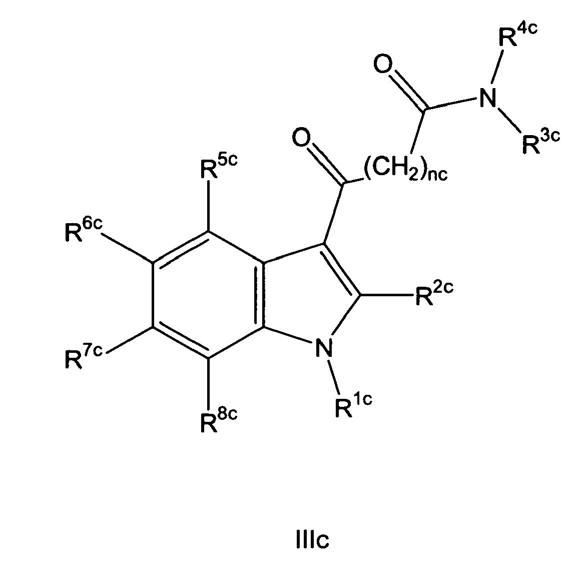 wherein R^{1c} to R^{4c} and nc have the meaning given above and R^{5c}, R^{6c}, R^{7c} and R^{8c} have the meaning given above with the proviso that at least one substituent of the group consisting of R^{5c}, R^{6c}, R^{7c} and R^{8c} represents an -N(R^{11c})(H) moiety or a protected derivative thereof, in a suitable reaction medium, preferably in the presence of at least one base and/or at least one auxiliary agent.

If a protected derivative is used, the respective protective group has to be removed after the reaction to obtain the desired substituted indole compound of general formula Ic. Suitable protecting groups as well as methods for introducing and removing such protecting groups are well known to those skilled in the art as described below.

Suitable reaction media for the reaction between compounds of general formulas IIc and IIIc include organic solvents, such as dialkyl ether, preferably diethyl ether, or a cyclic ether, preferably tetrahydrofurane or dioxane; or a halogenated hydrocarbon, preferably dichloromethane or chloroform; an alcohol, preferably methanol or ethanol; a dipolar aprotic solvent, preferably acetonitrile, pyridine or dimethylformamide, or any other suitable reaction medium. Of course, mixtures of at least two classes of solvents or of at least two solvents of one class may also be used.

The reaction is preferably carried out in the presence of at least one suitable base, for example, an inorganic base such as a hydroxide or a carbonate of an alkali metal and/or an organic base, preferably triethylamine or pyridine.

The reaction is preferably carried out at a temperature between -10 °C and ambient temperature, i.e. approximately 25 °C and the reaction time is preferably between 5 minutes and 24 hours.

The compounds of general formula Ic given above may be purified and/or isolated according to methods well known to those skilled in the art. Preferably, the compounds of general formula Ic may be isolated by evaporating the reaction medium, addition of water and adjusting the pH value to obtain the compound in form of a solid that can be isolated by filtration, or by extraction with a solvent that is not miscible with water such as chloroform and purification by chromatography or recrystallisation from a suitable solvent.

The compounds of general formula Ilc are commercially available or may be prepared according to methods well known in the art, for example, analogous to the methods described in the bibliography of E.E. Gilbert, Synthesis, 1969, 1, 3.

The compounds of general formula IIIc are also either commercially available or can be produced according to methods known to those skilled in the art as for example described in Dillard et al., Journal of Medicinal Chemistry 1996, 39(26), 5119-5136 by reduction of the corresponding nitro derivatives which are either commercially available or can be produced according to methods known to those skilled in the art as - for example - described in the literature publications of Primofiore et al., Journal of Medicinal Chemistry 2001, 44(14), 2286-2297, Da Settimo et al. Generoso. Farmaco 1993, 48(2), 285-295, Da Settimo et al. Journal of Medicinal Chemistry 1996, 39(26), 5083-5091, Macor et al. Synthetic Communications 1993, 23(1), 65-72., Settimo et al. Gazzetta Chimica ltaliana 1962, 92, 150-164, Primofiore et al. Journal of Medicinal Chemistry 1989, 32(12), 2514-2518, Primofiore et al. European Journal of Medicinal Chemistry 1988, 23(4), 397-401.

The respective parts of the literature descriptions cited above are hereby incorporated by reference and form part of the disclosure.

Altematively, the substituted indole compounds of general formula Ic given above, in which R^{11c} represents a linear or branched, saturated or unsaturated, optionally at least mono-substituted aliphatic radical; a saturated or unsaturated, optionally at least mono-substituted, optionally at least one heteroatom as a ring member containing cycloaliphatic radical, which is bonded via a linear or branched, optionally at least mono-substituted alkylene, alkenylene or alkinylene group; or an optionally at least mono-substituted aryl or heteroaryl radical, which is bonded via a linear or branched, optionally at least mono-substituted alkylene, alkenylene or alkinylene group and all of the other substituents have the above defined meaning, may also be prepared by an alkylation type reaction from the corresponding compound of general formula Ic, in wherein R^{11c} represents a hydrogen atom and all of the other substituents have the meaning given above, e.g. by reaction with corresponding halide R^{11c}-X, wherein X represents a halogen atom, preferably a chlorine atom, or a sulfate of the general formula lVc 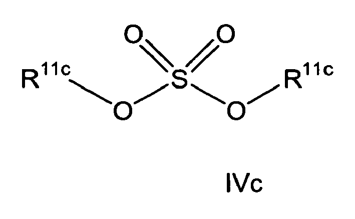 wherein in each case R^{11c} has the afore mentioned meaning. The corresponding halides and sulfates are commercially available or may be prepared according to methods well known to those skilled in the art.

The alkylation type reaction is preferably carried out in the presence of at least one suitable base such as a hydroxide and/or a carbonate of an alkali metal, a metal hydride, alcoxides such as sodium methoxide or potassium tert-butoxid, organometallic compounds such as n-butyllithium or tert-butyllithium, in the presence of a suitable reaction medium such as dialkyl ether, preferably diethyl ether, or a cyclic ether, preferably tetrahydrofurane or dioxane, a hydrocarbon, preferably toluene, an alcohol, preferably methanol or ethanol, a dipolar aprotic solvent, preferably acetonitrile, pyridine or dimethylformamide, or any other suitable reaction medium. Of course, mixtures of at least two classes of solvents or of at least two solvents of one class may also be used.

The reaction is preferably carried out at a temperature between -10 °C and ambient temperature, i.e. approximately 25 °C and the reaction time is preferably 1 and 24 hours.

The compounds of general formula Ic given above may be purified and/or isolated according to methods well known to those skilled in the art. Preferably, the compounds of general formula lc may be isolated by evaporating the reaction medium, addition of water and then adjusting the pH value to obtain the compound in form of a solid that can be isolated by filtration, or by extraction with a solvent that is not miscible with water such as chloroform and purified by chromatography or recrystallisation from a suitable solvent.

During some synthetic reactions described above or while preparing the compounds of general formulas IIc or IIIc the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.

If the substituted indole compounds of general formula Ic are obtained in form of a mixture of stereoisomers, particularly enantiomers or diastereomers, said mixtures may be separated by standard procedures known to those skilled in the art, e.g. chromatographic methods or crystallization with chiral reagents.

A further aspect of the present invention relates to a medicament comprising at least one substituted indole compound of general formula Ic given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, and optionally at least one auxiliary substance.

Said medicament is suitable for 5-HT₆-receptor regulation, particularly for the prophylaxis and/or treatment of a disorder or a disease that is least partially mediated via 5-HT₆-receptors.

Preferably said medicament is suitable for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity, or for the prophylaxis and/or treatment of irritable colon syndrome; disorders of the central nervous system; anxiety; panic attacks; depression; bipolar disorders; cognitive disorders; memory disorders; senile dementia; psychosis; neurodegenerative disorders, preferably selected from the group consisting of Morbus Alzheimer, Morbus Parkinson, Morbus Huntington and Multiple Sclerosis; schizophrenia; psychosis; hyperactivity disorder (ADHD, attention deficit/hyperactivity disorder) or for improvement of cognition (cognitive enhancement).

More preferably said medicament is suitable for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

Most preferably, said medicament is suitable for the prophylaxis and/or treatment of obesity and/or disorders or diseases related thereto.

In another aspect the present invention relates to the use of at least one substituted indole compound of general formula Ic given above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament suitable for 5-HT₆-receptor regulation, preferably for the prophylaxis and/or treatment of a disorder or a disease that is least partially mediated via 5-HT₆-receptors.

The use of at least one substituted indole compound of general formula Ic given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity, or for the prophylaxis and/or treatment of irritable colon syndrome; disorders of the central nervous system; anxiety; panic attacks; depression; bipolar disorders; cognitive disorders; memory disorders; senile dementia; psychosis; neurodegenerative disorders, preferably selected from the group consisting of Morbus Alzheimer, Morbus Parkinson, Morbus Huntington and Multiple Sclerosis; schizophrenia; psychosis; hyperactivity disorder (ADHD, attention deficit/hyperactivity disorder) or for improvement of cognition (cognitive enhancement) is preferred.

More preferred is the use of at least one substituted indole compound of general formula Ic as defined above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

Most preferred is the use of at least one substituted indole compound of general formula Ic as defined above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for for the prophylaxis and/or treatment of obesity and/or disorders or diseases related thereto.

In yet another aspect the present invention relates to a medicament comprising at least one substituted indole compound of general formula Ia*,* 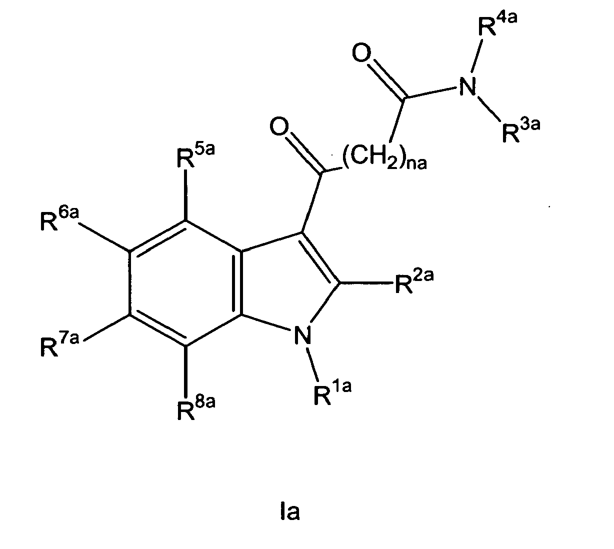 wherein
na represents 0, 1, 2, 3 or 4,
for na = 0: R^{1a} represents a hydrogen atom; a saturated or unsaturated, optionally at least mono-substituted, optionally at least one heteroatom as a ring member containing cycloaliphatic radical; an optionally at least mono-substituted aryl or heteroaryl radical; -S(=O)₂-R^{9a}; or -C(=O)-R^{10a},
for na = 1, 2, 3 or 4: R^{1a} represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; an optionally at least mono-substituted aryl or heteroaryl radical, which may be bonded via a linear or branched alkylene group; -S(=O)₂-R^{9a}; or -C(=O)-R^{10a},
R^{2a} represents -H, -NO₂; -NH₂; -SH; -OH; -CN; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R^{3a} and R^{4a}, identical or different, represent a hydrogen atom; a linear or branched, saturated or unsaturated aliphatic radical, an optionally at least mono-substituted aryl or heteroaryl radical, which may be bonded via a linear or branched alkylene group; a saturated or unsaturated, optionally at least mono-substituted, optionally at least one heteroatom as a ring member containing cycloaliphatic radical, which may be bonded via a linear or branched alkylene group and/or which may be condensed with an optionally at least mono-substituted mono- or polycyclic ring system, or
R^{3a} and R^{4a} together with the bridging nitrogen form an optionally at least mono-substituted, saturated, unsaturated or aromatic heterocyclic ring that may contain at least one further heteroatom as a ring member and/or that may be condensed with an optionally at least mono-substituted mono- or polycyclic ring-system,
R^{5a}, R^{6a}, R^{7a} and R^{8a}, identical or different, represent -H; -NO₂; -CN; -N(R^{11a})-S(=O)₂-R^{12a}; -OR^{13a}; -SR^{14a}; -C(=O)-OR^{15a}; -NR^{16a}R^{17a}; -C(=O)-R^{18a}; -(C=O)-NR^{19a}R^{20a}; -O-(C=O)-R^{21a}; -S(=O)₂-R^{22a}; -S(=O)₂-NR^{23a}R^{24a}; -NR^{25a}-C(=O)-NR^{26a}R^{27a}; -NR^{28a}-(C=O)-R^{29a}; -NR^{30a}-(C=O)-OR^{31a}; -NR^{32a}-S(=O)NR^{33a}R^{34a}; or-S(=O)₂-OR^{35a}; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
with the proviso that at least one of the substituents R^{5a}, R^{6a}, R^{7a} and R^{8a} represents an -N(R^{11a})-S(=O)₂-R^{12a} moiety,
R^{9a} and R^{10a}, independent from one another, represent a linear or branched, saturated or unsaturated, optionally at least mono-substituted aliphatic radical; or an optionally at least mono-substituted aryl or heteroaryl radical, which may be bonded via a linear or branched alkylene group,
R^{11a} represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group; an optionally at least mono-substituted aryl or heteroaryl radical, which may be bonded via a linear or branched, optionally at least mono-substituted alkylene, alkenylene or alkinylene group; or an -S(=O)₂-R^{36a} moiety,
R^{12a} represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system; or a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system,
R^{13a}, R^{14a}, R^{15a}, R^{16a}, R^{17a}, R^{18a}, R^{19a}, R^{20a}, R^{21a}, R^{22a}, R^{23a}, R^{24a}, R^{25a}, R^{26a}, R^{27a}, R^{28a}, R^{29a}, R^{30a}, R^{31a}, R^{32a}, R^{33a}, R^{34a}, R^{35a}, independent from one another, represent a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R^{36a} represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system; or a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system,
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred is a medicament comprising at least one substituted indole compound of general formula la, wherein
na is 0, 1, 2, 3 or 4
for na = 0: R^{1a} represents a hydrogen atom; a saturated or unsaturated, optionally at least mono-substituted, optionally at least one heteroatom as a ring member containing 3- to 9-membered cycloaliphatic radical; an optionally at least mono-substituted 5- to 14- membered aryl or heteroaryl radical; -S(=O)₂-R^{9a}; or -C(=O)-R^{10a},
for na = 1, 2, 3 or 4: R^{1a} represents a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆-alkylene group; an optionally at least mono-substituted 5- to 14- membered aryl or heteroaryl radical, which may be bonded via a linear or branched C₁₋₆-alkylene group; -S(=O)₂-R^{9a}; or -C(=O)-R^{10a},
R^{2a} represents -H, -NO₂; -NH₂; -SH; -OH; -CN; a halogen atom; a linear or branched, saturated or unsaturated, optionally at least mono-substituted, optionally at least one heteroatom as a chain member containing C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆-alkylene group; or an optionally at least mono-substituted 5- to 14-membered aryl or heteroaryl radical, which may be bonded via a linear or branched C₁₋₆-alkylene group,
R^{3a} and R^{4a}, identical or different, represent a hydrogen atom; a linear or branched, saturated or unsaturated C₁₋₁₀ aliphatic radical; an optionally at least mono-substituted 5- to 14-membered aryl or heteroaryl radical, which may be bonded via a linear or branched C₁₋₆ alkylene group; a saturated or unsaturated, optionally at least mono-substituted, optionally at least one heteroatom as a ring member containg 3- to 9-membered cycloaliphatic radical, which may be bonded via a linear or branched C₁₋₆ alkylene group and/or which may be condensed with an optionally at least mono-substituted mono- or polycyclic ring system, or
R^{3a} and R^{4a} together with the bridging nitrogen form an optionally at least mono-substituted, saturated, unsaturated or aromatic 4- to 9-membered heterocyclic ring that may contain at least one further heteroatom as a ring member and/or that may be condensed with an optionally at least mono-substituted mono- or polycyclic ring-system,
R^{5a}, R^{6a}, R^{7a} and R^{8a}, identical or different, represent -H; -NO₂; -CN; -N(R^{11a})-S(=O)₂-R^{12a}; -OR^{13a}; -SR^{14a}; -C(=O)-OR^{15a}; -NR^{16a}R^{17a}; -C(=O)-R^{18a}; -(C=O)-NR^{19a}R^{20a}; -O-(C=O)-R^{21a}; -S(=O)₂-R^{22a}; -S(=O)₂-NR^{23a}R^{24a}; -NR^{25a}-C(=O)-NR^{26a}R^{27a}; -NR^{28a}-(C=O)-R^{29a}; -NR^{30a}-(C=O)-OR^{31a}; -NR^{32a}-S(=O)NR^{33a}R^{34a}; or-S(=O)₂-OR^{35a}; a halogen atom; a linear or branched, saturated or unsaturated, optionally at least mono-substituted, optionally at least one heteroatom as a chain member containing C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆-alkylene group,
with the proviso that at least one of the substituents R^{5a}, R^{6a}, R^{7a} and R^{8a} represents an -N(R^{11a})-S(=O)₂-R^{12a} moiety,
R^{9a} and R^{10a}, independent from one another, represent a linear or branched, saturated or unsaturated, optionally at least mono-substituted C₁₋₁₀ aliphatic radical; or an optionally at least mono-substituted, 5- to 14-membered aryl or heteroaryl radical, which may be bonded via a linear or branched C₁₋₆-alkylene group,
R^{11a} represents a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group; an optionally at least mono-substituted 5- to 14 membered aryl or heteroaryl radical, which may be bonded via a linear or branched, optionally at least mono-substituted C₁₋₆ alkylene, C₂₋₆ alkenylene or C₂₋₆ alkinylene group; or an -S(=O)₂-R^{36a} moiety,
R^{12a} represents an optionally at least mono-substituted 5- to 14-membered aryl or heteroaryl radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system; or a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system,
R^{13a}-R^{35a}, independent from one another, represent a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆ alkylene group,
R^{36a} represents an optionally at least mono-substituted 5- to 14-membered aryl or heteroaryl radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system; or a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system.

Furthermore, preferred is a medicament comprising at least one substituted indole compound of general formula Ia, wherein
for na = 0
R^{1a} represents a hydrogen atom; a saturated or unsaturated, optionally at least mono-substituted, 3- to 9-membered cycloaliphatic radical, which may contain 1, 2 or 3 heteroatoms independently selected from the group consisting of nitrogen, oxygen and sulphur as ring members; an optionally at least mono-substituted 5- to 10-membered aryl or heteroaryl radical, wherein the heteroaryl radical contains 1, 2 or 3 heteroatoms independently selected from the group consisting of nitrogen, oxygen and sulphur as ring members;

-S(=O)₂-R^{9a}; or -C(=O)-R^{10a},

preferably R^{1a} represents a hydrogen atom;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅₋alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; -S(=O)₂-R^{9a}; or -C(=O)-R^{10a},
more preferably R^{1a} represents a hydrogen atom,
for na = 1, 2, 3 or 4
R^{1a} represents a hydrogen atom; a linear or branched C₁₋₁₀ alkyl radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members; -S(=O)₂-R^{9a}; or -C(=O)-R^{10a},
preferably R^{1a} represents a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; -S(=O)₂-R^{9a}; or -C(=O)-R^{10a}
and R^{2a}-R^{36a} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Furthermore, preferred is a medicament comprising at least one substituted indole compound of general formula Ia, wherein R^{2a} represents -H, -NO₂; -NH₂; -SH; -OH; -CN; -CF₃; -OCH₃; -O-CH₂-CH₃; F; Cl; Br; I; a linear or branched, saturated or unsaturated C₁₋₁₀ aliphatic radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10-membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R^{2a} represents -H, -NO₂; -NH₂; -SH; -OH; -CN; -CF₃; -OCH₃; -O-CH₂₋CH₃; F; Cl; Br; I; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R^{2a} represents a hydrogen atom,
and na, R^{1a} and R^{3a}-R^{36a} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Furthermore, preferred is a medicament comprising at least one substituted indole compound of general formula Ia, wherein
R^{3a} and R^{4a}, identical or different, represent a hydrogen atom or a linear or branched C₁₋₈ alkyl radical, or
R^{3a} and R^{4a} together with the bridging nitrogen form an optionally at least mono-substituted, saturated, unsaturated or aromatic 4- to 9-membered heterocyclic ring that may be condensed with an optionally at least mono-substituted mono- or bicyclic ring-system,
whereby the rings of the ring system are 5- 6- or 7-membered and whereby the heterocyclic ring and one or both of the rings of the ring system may contain 1, 2 or 3 heteroatoms independently selected from the group consisting of nitrogen, oxygen and sulphur,
preferably
R^{3a} and R^{4a}, identical or different, represent a hydrogen atom or an alkyl radical selected from the group consisting of methyl, ethyl, n-propyl, iso-propyl, n-butyl, sec-butyl, iso-butyl and tert-butyl; or
R^{3a} and R^{4a} together with the bridging nitrogen atom form a moiety selected from the group consisting of 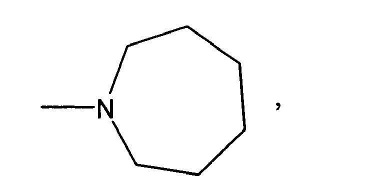 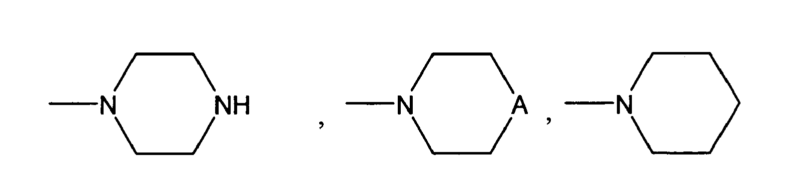 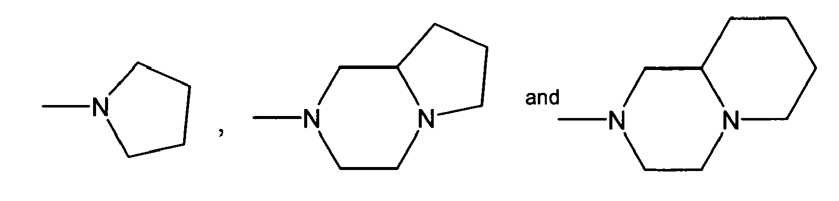 whereby A represents an oxygen atom or a sulphur atom and whereby each of these afore mentioned cyclic moieties may be substituted with 1, 2 or 3 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl in any position including the nitrogen atoms of the piperazine ring,
more preferably R^{3a} and R^{4a}, identical or different, represent a hydrogen atom or an alkyl radical selected from the group consisting of methyl, ethyl, n-propyl, iso-propyl, n-butyl, sec-butyl, iso-butyl and tert-butyl
and
na, R^{1a}, R^{2a} and R^{5a}-R^{36a} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Furthermore, preferred is a medicament comprising at least one substituted indole compound of general formula la, wherein
R^{5a}, R^{6a}, R^{7a} and R^{8a} identical or different, represent -H; -NO₂; -CN; -N(R^{11a})-S(=O)₂-R^{12a}; -OR^{13a}; -SR^{14a}; -C(=O)-OR^{15a}; -NR^{16a}R^{17a}; -C(=O)-R^{18a}; -(C=O)-NR^{19a}R^{20a}; -O-(C=O)-R^{21a}; -S(=O)₂-R^{22a}; -S(=O)₂-NR^{23a}R^{24a}; -NR^{25a}-C(=O)-NR^{26a}R^{27a}; -NR^{28a}-(C=O)-R^{29a}; -NR^{30a}-(C=O)-OR^{31a}; -NR^{32a}-S(=O)NR^{33a}R^{34a}; or -S(=O)₂-OR^{35a}; F, Cl, Br, I; -CF₃, -CHF₂, -CH₂F, a linear or branched, saturated or unsaturated C₁₋₁₀ aliphatic radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10-membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R^{5a}, R^{6a}, R^{7a} and R^{8a}, identical or different, represent -H; -NO₂; -CN; -N(R^{11a})-S(=O)₂-R^{12a}; -OR^{13a}; -SR^{14a}; -C(=O)-OR^{15a}; -NR^{16a}R^{17a}; -C(=O)-R^{18a}; -(C=O)-NR^{19a}R^{20a}; -O-(C=O)-R^{21a}; -S(=O)₂-R^{22a}; -S(=O)₂-NR^{23a}R^{24a}; -NR^{25a}-C(=O)-NR^{26a}R^{27a}; -NR^{28a}-(C=O)-R^{29a}; -NR^{30a}-(C=O)-OR^{31a}; -NR^{32a}-S(=O)NR^{33a}R^{34a}; or -S(=O)₂-OR^{35a}; F, Cl, Br, I; -CF₃, -CHF₂, -CH₂F, an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R^{5a}, R^{6a}, R^{7a} and R^{8a}, identical or different, each represent -H; -NO₂; -CN; -N(R^{11a})-S(=O)₂-R^{12a}; -OR^{13a}; -SR^{14a}; -C(=O)-OR^{15a}; -NR^{16a}R^{17a}; - F, Cl, Br, I; -CF₃, -CHF₂, -CH₂F, an alkyl radical selected from the group consisting of methyl, ethyl, n-propyl, iso-propyl, n-butyl, sec-butyl, iso-butyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
in each case with the proviso that at least one of the substituents R^{5a}, R^{6a}, R^{7a} and R^{8a} represents an -N(R^{11a})-S(=O)₂-R^{12a} moiety,
and na, R^{1a}-R^{4a} and R^{9a}-R^{36a} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Furthermore, preferred is a medicament comprising at least one substituted indole compound of general formula la, wherein one of the substituents R^{5a}, R^{6a}, R^{7a} and R^{8a} represents an -N(R^{11a})-S(=O)₂-R^{12a} moiety, preferably one of the substituents R^{5a}, R^{6a}, R^{7a} and R^{8a} represents an -N(R^{11a})-S(=O)₂-R^{12a} moiety while the other three of these substituents each represent a hydrogen atom, and na, R^{1a}-R^{4a} and R^{9a}-R^{36a} - if present - have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Furthermore, preferred is a medicament comprising at least one substituted indole compound of general formula Ia, wherein R^{9a} and R^{10a}, independent from one another, represent a linear or branched, saturated or unsaturated C₁₋₁₀ aliphatic radical; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R^{9a} and R^{10a}, independent from one another, represent an alkyl radical selected from the group consisting of methyl, ethyl, n-propyl, iso-propyl, n-butyl, sec-butyl, iso-butyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -0-(C=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
and na, R^{1a}-R^{8a} and R^{11a}-R^{36a} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Furthermore, preferred is a medicament comprising at least one substituted indole compound of general formula la, wherein R^{11a} represents a hydrogen atom; a linear or branched, saturated or unsaturated, optionally at least mono-substituted C₁₋₁₀₋aliphatic radical; a saturated or unsaturated, optionally at least mono-substituted, 3-to 9-membered cycloaliphatic radical, which may be bonded via a linear or branched C₁₋₆-alkylene, C₂₋₆-alkenylene or C₂₋₆-alkinylene group and/or which may contain 1, 2 or 3 heteroatoms independently selected from the group consisting of nitrogen, oxygen and sulphur as ring members; an optionally at least mono-substituted 5- to 10- membered aryl or heteroaryl radical, which may be bonded via a linear or branched C₁₋₆-alkylene, C₂₋₆-alkenylene or C₂₋₆-alkinylene group and wherein the heteroaryl radical contains 1, 2 or 3 heteroatoms independently selected from the group consisting of nitrogen, oxygen and sulphur as ring members; or an -S(=O)₂₋R^{36a} moiety,
preferably R^{11a} represents a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or an -S(=O)₂-R^{36a}-moiety,
more preferably R^{11a} represents a hydrogen atom,
and na, R^{1a}-R^{10a} and R^{12a}-R^{36a} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Furthermore, preferred is a medicament comprising at least one substituted indole compound of general formula Ia, wherein R^{12a} represents an optionally at least mono-substituted 5- to 10- membered aryl or heteroaryl radical, which may be bonded via a linear or branched, optionally at least mono-substituted C₁₋₆-alkylene, C₂₋₆-alkenylene or C₂₋₆-alkinylene group and/or which may be condensed with an optionally at least mono-substituted, saturated, unsaturated or aromatic, mono- or bicyclic ring system,
whereby the rings of the ring system are 5- 6- or 7-membered and whereby the heteroaryl radical and optionally one or both of the rings of the ring system contain 1, 2 or 3 heteroatoms independently selected from the group consisting of nitrogen, oxygen and sulphur,
or R^{12a} represents a saturated or unsaturated, optionally at least mono-substituted, 3-to 9-membered cycloaliphatic radical, which may be bonded via a linear or branched C₁₋₆-alkylene, C₂₋₆-alkenylene or C₂₋₆-alkinylene group and/or which may be condensed with an optionally at least mono-substituted mono- or polycyclic ring system,
whereby the rings of the ring system are 5- 6- or 7-membered and whereby the cycloaliphatic radical and optionally one or both of the rings of the ring system may contain 1, 2 or 3 heteroatoms independently selected from the group consisting of nitrogen, oxygen and sulphur,
preferably R^{12a} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, 2-oxo-2,3-dihydro-benzooxazolyl, 2-oxo-2,3-dihydrobenzo[d]thiazolyl, benzooxazolinyl, benzothiazolinyl, benzimidazolidinyl, imidazo[2,1-b]thiazolyl, chromenyl and chromanyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅₋alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -CO-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R^{12a} represents an aryl or heteroaryl radical selected from the group consisting of phenyl, 1-naphthyl, 2-naphthyl, benzo[b]-thiophenyl, benzo[b]furanyl, quinolinyl, isoquinolinyl, imidazo[2,1-b]thiazolyl, 2-oxo-2,3-dihydro-benzooxazolyl and 2-oxo-2,3-dihydrobenzo[d]thiazolyl, whereby said aryl or heteroaryl radical may be substituted by 1, 2, 3, 4 or 5 substituents independently selected from the group consisting of methyl, ethyl, n-propyl, iso-propyl, n-butyl, sec-butyl, iso-butyl, tert-butyl, F, CI, Br, I, -CN, -CF₃, -CF₂H, CFH₂, -C(=O)-O-CH₃, C(=O)-O-CH₂-CH₃, cyclopropyl, cyclobutyl, cyclopentyl, cyclohexyl, phenyl, phenoxy and benzyl,
and na, R^{1a}-R^{11a} and R^{13a}-R^{36a} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Furthermore, preferred is a medicament comprising at least one substituted indole compound of general formula la, wherein R^{13a}-R^{35a} independent from one another, each represent a hydrogen atom; a linear or branched C₁₋₁₀ alkyl radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members;
preferably R^{13a}-R^{35a} independent from one another, represent a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl- (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
and na, R^{1a}-R^{12a} and R^{36a} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Furthermore, preferred is a medicament comprising at least one substituted indole compound of general formula la, wherein R^{36a} represents an optionally at least mono-substituted 5- to 10- membered aryl or heteroaryl radical, which may be bonded via a linear or branched, optionally at least mono-substituted C₁₋₆-alkylene, C₂₋₆-alkenylene or C₂₋₆-alkinylene group and/or which may be condensed with an optionally at least mono-substituted, saturated, unsaturated or aromatic, mono- or bicyclic ring system,
whereby the rings of the ring system are 5- 6- or 7-membered and whereby the heteroaryl radical and optionally one or both of the rings of the ring system contain 1, 2 or 3 heteroatoms independently selected from the group consisting of nitrogen, oxygen and sulphur,
or R^{36a} represents a saturated or unsaturated, optionally at least mono-substituted, 3-to 9-membered cycloaliphatic radical, which may be bonded via a linear or branched C₁₋₆-alkylene, C₂₋₆-alkenylene or C₂₋₆-alkinylene group and/or which may be condensed with an optionally at least mono-substituted mono- or polycyclic ring system,
whereby the rings of the ring system are 5- 6- or 7-membered and whereby the cycloaliphatic radical and optionally one or both of the rings of the ring system may contain 1, 2 or 3 heteroatoms independently selected from the group consisting of nitrogen, oxygen and sulphur,
preferably R^{36a} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, 2-oxo-2,3-dihydro-benzooxazolyl, 2-oxo-2,3-dihydrobenzo[d]thiazolyl, benzooxazolinyl, benzothiazolinyl, benzimidazolidinyl, imidazo[2,1-b]thiazolyl, chromenyl and chromanyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅₋alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -CO-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl.
and na and R^{1a} -R^{35a} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Furthermore, preferred is a medicament comprising at least one substituted indole compound of general formula la, wherein na is 0 and R^{1a}-R^{36a} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

In any of the foregoing definitions the following proviso may apply, namely that R^{3a} and R^{4a} do not both identically represent a hydrogen atom.

Particularly preferred is a medicament comprising at least one substituted indole compound of general formula Ia,
na is 0,
R^{1a} represents a hydrogen atom,
R^{2a} represents a hydrogen atom,
R^{3a} and R^{4a}, identical or different, represent an alkyl radical selected from the group consisting of methyl, ethyl, n-propyl, iso-propyl, n-butyl, sec-butyl, iso-butyl and tert-butyl,
one of the substituents R^{5a}, R^{6a}, R^{7a} and R^{8a} represents an -N(R^{11a})-S(=O)-R^{12a-}moiety while the other three of these substituents each represent a hydrogen atom,
R^{11a} represents a hydrogen atom,
R^{12a} represents an aryl or heteroaryl radical selected from the group consisting of phenyl, 1-naphthyl, 2-naphthyl, benzo[b]-thiophenyl, benzo[b]furanyl, quinolinyl, isoquinolinyl, imidazo[2,1-b]thiazolyl, 2-oxo-2,3-dihydro-benzooxazolyl and 2-oxo-2,3-dihydrobenzo[d]thiazolyl, whereby said aryl or heteroaryl radical may be substituted by 1, 2, 3, 4 or 5 substituents independently selected from the group consisting of methyl, ethyl, n-propyl, iso-propyl, n-butyl, sec-butyl, iso-butyl, tert-butyl, F, CI, Br, I, -CN, -CF₃, -CF₂H, CFH₂, -C(=O)-O-CH₃, C(=O)-O-CH₂-CH₃, cyclopropyl, cyclobutyl, cyclopentyl, cyclohexyl, phenyl, phenoxy and benzyl.

Most particularly preferred is a medicament comprising at least one substituted indole compound of general formula la selected from the group consisting of
[1] 2-[5-(5-Chloro-3-methyl-benzo[b]thiophene-2-sulfonylamino)-1H-indol-3-yl]-N,N-diethyl-2-oxoacetamide,
[2] N,N-Diethyl-2-[5-(naphthalene-2-sulfonylamino)-1H-indol-3-yl]-2-oxo-acetamide,
[3] N,N-Diethyl-2-[5-(naphthalene-1-sulfonylamino)-1H-indol-3-yl]-2-oxo-acetamide,
[4] 2-[5-(Biphenyl-4-sulfonylamino)-1H-indol-3-yl]-N,N-diethyl-2-oxo-acetamide,
[5] N,N-Diethyl-2-oxo-2-[5-(quinoline-8-sulfonylamino)-1H-indol-3-yl]-acetamide,
[6] N,N-Dimethyl-2-[5-(naphthalene-2-sulfonylamino)-1H-indol-3-yl]-2-oxo-acetamide,
[7] N,N-Dimethyl-2-[5-(naphthalene-1-sulfonylamino)-1H-indol-3-yl]-2-oxo-acetamide,
[8] 2-[5-(5-Chloro-3-methyl-benzo[b]thiophene-2-sulfonylamino)-1H-indol-3-yl]-N,N-dimethyl-2-oxo-acetamide,
[9] 2-[5-(6-Chloro-imidazo[2,1-b]thiazole-5-sulfonylamino)-1H-indol-3-yl]-N,N-diethyl-2-oxo-acetamide,
[10] 2-[5-(6-Chloro-imidazo[2,1-b]thiazole-5-sulfonylamino)-1H-indol-3-yl]-N,N-dimethyl-2-oxo-acetamide,
[11] N,N-Dimethyl-2-[4-(naphthalene-1-sulfonylamino)-1H-indol-3-yl]-2-oxo-acetamide,
[12] 2-[4-(5-Chloro-3-methyl-benzo[b]thiophene-2-sulfonylamino)-1H-indole-3-yl]-N,N-dimethyl-2-oxo-acetamide,
[13] 2-[4-(6-Chloro-imidazo[2,1-b]thiazole-5-sulfonylamino)-1H-indol-3-yl]-N,N-dimethyl-2-oxo-acetamide,
[14] N,N-Dimethyl-2-[5-[(4-fluoro-3-methyl-phenyl)-1-sulfonylamino]-1H-indol-3-yl]-2-oxo-acetamide,
[15] 5-(3-Dimethylaminooxalyl-1H-indol-5-ylsulfamoyl)-3-methyl-benzofuran-2-carboxylic acid ethyl ester,
[16] 2-[5-(Biphenyl-4-sulfonylamino)-1 H-indol-3-yl]-N,N-dimethyl-2-oxo-acetamide,
[17] N,N-Dimethyl-2-oxo-2-[5-(2-oxo-2,3-dihydro-benzoxazole-6-sulfonylamino)-1 H-indol-3-yl]-acetamide,
[18] N,N-Dimethyl-2-oxo-2-[5-(2-oxo-2,3-dihydrobenzo[d]thiazole-6-sulfonamido)-1 H-indol-3-yl]acetamide,
[19] 2-[5-[(4-Cyclohexyl-phenyl)-1-sulfonylamino]-1 H-indol-3-yl]-N,N-dimethyl-2-oxo-acetamide and
[20] N,N-Dimethyl-2-[5-[(4-phenoxy-phenyl)-1-sulfonylamino]-1 H-indol-3-yl]-2-oxo-acetamide.

The medicament described above comprising at least one substituted indole compound of general formula la, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, and optionally at least one auxiliary substance, is suitable for 5-HT₆-receptor regulation, particularly for the prophylaxis and/or treatment of a disorder or a disease that is least partially mediated via 5-HT₆-receptors.

Preferably said medicament is suitable for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity, or for the prophylaxis and/or treatment of irritable colon syndrome; disorders of the central nervous system; anxiety; panic attacks; depression; bipolar disorders; cognitive disorders; memory disorders; senile dementia; psychosis; neurodegenerative disorders, preferably selected from the group consisting of Morbus Alzheimer, Morbus Parkinson, Morbus Huntington and Multiple Sclerosis; schizophrenia; psychosis; hyperactivity disorder (ADHD, attention deficit/hyperactivity disorder), or for improvement of cognition (cognitive enhancement).

More preferably said medicament is suitable for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

Most preferably, said medicament is suitable for the prophylaxis and/or treatment of obesity and/or disorders or diseases related thereto.

In yet another aspect the present invention relates to the use of at least one substituted indole compound of general formula Ib, 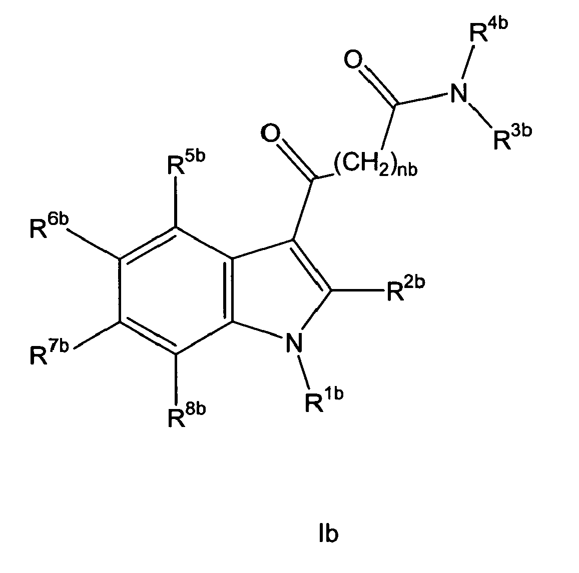 wherein
nb represents 0, 1, 2, 3 or 4,
R^{1b} represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; an optionally at least mono-substituted aryl or heteroaryl radical, which may be bonded via a linear or branched alkylene group; -S(=O)₂-R^{9b}; or -C(=O)-R^{10b},
R^{2b} represents -H, -NO₂; -NH₂; -SH; -OH; -CN; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R^{3b} and R^{4b}, identical or different, represent a hydrogen atom; a linear or branched, saturated or unsaturated aliphatic radical, an optionally at least mono-substituted aryl or heteroaryl radical, which may be bonded via a linear or branched alkylene group; a saturated or unsaturated, optionally at least mono-substituted, optionally at least one heteroatom as a ring member containing cycloaliphatic radical, which may be bonded via a linear or branched alkylene group and/or which may be condensed with an optionally at least mono-substituted mono- or polycyclic ring system, or
R^{3b} and R^{4b} together with the bridging nitrogen form an optionally at least mono-substituted, saturated, unsaturated or aromatic heterocyclic ring that may contain at least one further heteroatom as a ring member and/or that may be condensed with an optionally at least mono-substituted mono- or polycyclic ring-system,
R^{5b}, R^{6b}, R^{7b} and R^{8b}, identical or different, represent -H; -NO₂; -CN; -N(R^{11b})-S(=O)₂-R^{12b}; -OR^{13b}; -SR^{14b}; -C(=O)-OR^{15b}; -NR^{16b}R^{17b}; -C(=O)-R^{18b}; -(C=O)-NR^{19b}R^{20b}; -O-(C=O)-R^{21b}; -S(=O)₂-R^{22b}; -S(=O)₂-NR^{23b}R^{24b}; -NR^{25b}-C(=O)-NR^{26b}R^{27b}; -NR^{28b}-(C=O)-R^{29b}; -NR^{30b}-(C=O)-OR^{31b}; -NR^{32b}-S(=O)NR^{33b}R^{34b}; or -S(=O)₂-OR^{35b}; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
with the proviso that at least one of the substituents R^{5b}, R^{6b}, R^{7b} and R^{8b} represents an -N(R^{11b})-S(=O)₂-R^{12b} moiety,
R^{9b} and R^{10b}, independent from one another, represent a linear or branched, saturated or unsaturated, optionally at least mono-substituted aliphatic radical; or an optionally at least mono-substituted aryl or heteroaryl radical, which may be bonded via a linear or branched alkylene group,
R^{11b} represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group; an optionally at least mono-substituted aryl or heteroaryl radical, which may be bonded via a linear or branched, optionally at least mono-substituted alkylene, alkenylene or alkinylene group; or an -S(=O)₂-R^{36b} moiety,
R^{12b} represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system; or a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system,
R^{13b}, R^{14b}, R^{15b}, R^{16b}, R^{17b}, R^{18b}, R^{19b}, R^{20b}, R^{21b}, R^{22b}, R^{23b}, R^{24b}, R^{25b}, R^{26b}, R^{27b}, R^{28b}, R^{29b}, R^{30b}, R^{31b}, R^{32b}, R^{33b}, R^{34b}, R^{35b}, independent from one another, represent a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R^{36b} represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system; or a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system,
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof,
for the manufacture of a medicament for 5-HT₆ receptor regulation, preferably for the prophylaxis and/or treatment of a disorder or disease that is at least partially mediated via 5-HT₆ receptors.

Preferably compounds of general formula Ib are used, wherein
nb is 0, 1, 2, 3 or 4,
R^{1b} represents a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆-alkylene group; an optionally at least mono-substituted 5- to 14- membered aryl or heteroaryl radical, which may be bonded via a linear or branched C₁₋₆-alkylene group; -S(=O)₂-R^{9b}; or -C(=O)-R^{10b},
R^{2b} represents -H; -NO₂; -NH₂; -SH; -OH; -CN; a halogen atom; a linear or branched, saturated or unsaturated, optionally at least mono-substituted, optionally at least one heteroatom as a chain member containing C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆-alkylene group; or an optionally at least mono-substituted 5- to 14-membered aryl or heteroaryl radical, which may be bonded via a linear or branched C₁₋₆-alkylene group,
R^{3b} and R^{4b}, identical or different, represent a hydrogen atom; a linear or branched, saturated or unsaturated C₁₋₁₀ aliphatic radical; an optionally at least mono-substituted 5- to 14-membered aryl or heteroaryl radical, which may be bonded via a linear or branched C₁₋₆ alkylene group; a saturated or unsaturated, optionally at least mono-substituted, optionally at least one heteroatom as a ring member containg 3- to 9-membered cycloaliphatic radical, which may be bonded via a linear or branched C₁₋₆ alkylene group and/or which may be condensed with an optionally at least mono-substituted mono- or polycyclic ring system, or
R^{3b} and R^{4b} together with the bridging nitrogen form an optionally at least mono-substituted, saturated, unsaturated or aromatic 4- to 9-membered heterocyclic ring that may contain at least one further heteroatom as a ring member and/or that may be condensed with an optionally at least mono-substituted mono- or polycyclic ring-system,
R^{5b}, R^{6b}, R^{7b} and R^{8b}, identical or different, represent -H; -NO₂; -CN; -N(R^{11b})-S(=O)₂-R^{12b}; -OR^{13b}; -SR^{14b}; -C(=O)-OR^{15b}; -NR^{16b}R^{17b}; -C(=O)-R^{18b}; -(C=O)-NR^{19b}R^{20b}; -O-(C=O)-R^{21b}; -S(=O)₂-R^{22b}; -S(=O)₂-NR^{23b}R^{24b}; -NR^{2sb}-C(=O)-NR^{26b}R^{27b}; -NR^{28b}-(C=O)-R^{29b}; -NR^{30b}-(C=O)-OR^{31b}; -NR^{32b}-S(=O)NR^{33b}R^{34b}; or -S(=O)₂-OR^{35b}; a halogen atom; a linear or branched, saturated or unsaturated, optionally at least mono-substituted, optionally at least one heteroatom as a chain member containing C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆-alkylene group,
with the proviso that at least one of the substituents R^{5b}, R^{6b}, R^{7b} and R^{8b} represents an -N(R^{11b})-S(=O)₂-R^{12b} moiety,
R^{9b} and R^{10b}, independent from one another, represent a linear or branched, saturated or unsaturated, optionally at least mono-substituted C₁₋₁₀ aliphatic radical; or an optionally at least mono-substituted, 5- to 14-membered aryl or heteroaryl radical, which may be bonded via a linear or branched C₁₋₆-alkylene group,
R^{11b} represents a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group; an optionally at least mono-substituted 5- to 14 membered aryl or heteroaryl radical, which may be bonded via a linear or branched, optionally at least mono-substituted C₁₋₆ alkylene, C₂₋₆ alkenylene or C₂₋₆ alkinylene group; or an -S(=O)₂-R^{36b} moiety,
R^{12b} represents an optionally at least mono-substituted 5- to 14-membered aryl or heteroaryl radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system; or a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system,
R^{13b}-R^{35b}, independent from one another, represent a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆ alkylene group,
R^{36b} represents an optionally at least mono-substituted 5- to 14-membered aryl or heteroaryl radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system; or a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system.

Also preferred is the use of compounds of general formula Ib, wherein R^{1b} represents a hydrogen atom; a linear or branched C₁₋₁₀ alkyl radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members; -S(=O)₂-R^{9b}; or -C(=O)-R^{10b},
preferably R^{1b} represents a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, -CF₂H, - CFH₂, -C(=O)-NH₂, -C(=O)-NH(C₁₋₅-alkyl), -CO-N(C₁₋₅-alkyl)₂, -S(=O)₂-C₁₋₅-alkyl, - S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2} or ₃- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; -S(=O)₂-R^{9b}; or -C(=O)-R^{10b},
more preferably R^{1b} represents a hydrogen atom, and nb and R^{2b}-R^{36b} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Also preferred is the use of compounds of general formula Ib, wherein
R^{2b} represents -H; -NO₂; -NH₂; -SH; -OH; -CN; -CF₃; -OCH₃; -O-CH₂-CH₃; F; CI; Br; I; a linear or branched, saturated or unsaturated C₁₋₁₀ aliphatic radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R^{2b} represents -H; -NO₂; -NH₂; -SH; -OH; -CN; -CF₃; -OCH₃; -O-CH₂-CH₃; F; Cl; Br; I; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1,2 or 3}- group and/or substituted with 1, 2, 3, 4 or 5 substituents independently selected from the group consisting of linear or branched C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, - S(=O)₂-C₁₋₅-alkyl, -S(=O)₂-phenyl, cyclopropyl, cyclobutyl, cyclopentyl, cyclohexyl, phenyl, phenoxy and benzyl,
more preferably R^{2b} represents a hydrogen atom,
and nb, R^{1b} and R^{3b}-R^{36b} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Also preferred is the use of compounds of general formula Ib, wherein
R^{3b} and R^{4b}, identical or different, represent a hydrogen atom or a linear or branched C₁₋₈ alkyl radical, or
R^{3b} and R^{4b} together with the bridging nitrogen form an optionally at least mono-substituted, saturated, unsaturated or aromatic 4- to 9-membered heterocyclic ring that may be condensed with an optionally at least mono-substituted mono- or bicyclic ring-system,
whereby the rings of the ring system are 5- 6- or 7-membered and whereby the heterocyclic ring and one or both of the rings of the ring system may contain 1, 2 or 3 heteroatoms independently selected from the group consisting of nitrogen, oxygen and sulphur, preferably
R^{3b} and R^{4b}, identical or different, represent a hydrogen atom or an alkyl radical selected from the group consisting of methyl, ethyl, n-propyl, iso-propyl, n-butyl, sec-butyl, iso-butyl and tert-butyl; or
R^{3b} and R^{4b} together with the bridging nitrogen atom form a moiety selected from the group consisting of 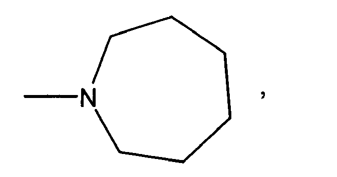 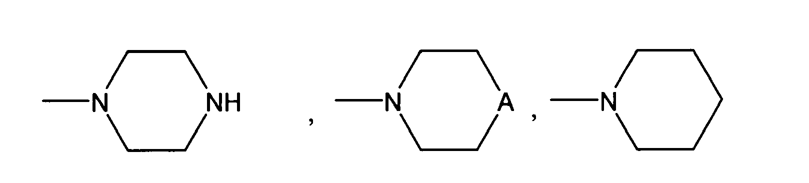 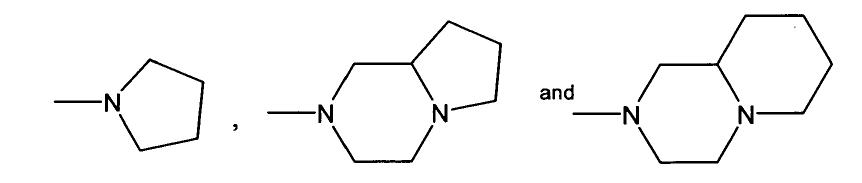 whereby A represents an oxygen atom or a sulphur atom and whereby each of these afore mentioned cyclic moieties may be substituted with 1, 2 or 3 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl in any position including the nitrogen atoms of the piperazine ring,
more preferably R^{3b} and R^{4b}, identical or different, represent a hydrogen atom or an alkyl radical selected from the group consisting of methyl, ethyl, n-propyl, iso-propyl, n-butyl, sec-butyl, iso-butyl and tert-butyl,
and nb, R^{1b}, R^{2b} and R^{5b}-R^{36b} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Also preferred is the use of compounds of general formula Ib, wherein
R^{5b}, R^{6b}, R^{7b} and R^{8b}, identical or different, represent -H; -NO₂; -CN; -N(R^{11b})-S(=O)₂-R^{12b}; -OR^{13b}; -SR^{14b}; -C(=O)-OR^{15b}; -NR^{16b}R^{17b}; -C(=O)-R^{18b}; -(C=O)-NR^{19b}R^{20b}; -O-(C=O)-R^{21b}; -S(=O)₂-R^{22b}; -S(=O)₂-NR^{23b}R^{24b}; -NR^{25b}-C(=O)-NR^{26b}R^{27b}; -NR^{28b}-(C=O)-R^{29b}; -NR^{30b}-(C=O)-OR^{31b}; -NR^{32b}-S(=O)NR^{33b}R^{34b}; or -S(=O)₂-OR^{35b}; F, Cl, Br, I; -CF₃, -CHF₂, -CH₂F, a linear or branched, saturated or unsaturated C₁₋₁₀ aliphatic radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10-membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R^{5b}, R^{6b}, R^{7b} and R^{8b}, identical or different, represent -H; -NO₂; -CN; -N(R^{11b})-SO₂-R^{12b}; -OR^{13b}; -SR^{14b}; -C(=O)-OR^{15b}; -NR^{16b}R^{17b}; -C(=O)-R^{18b}; -(C=O)-NR^{19b}R^{20b}; -O-(C=O)-R^{21b}; -S(=O)₂-R^{22b}; -S(=O)₂-NR^{23b}R^{24b}; -NR^{25b}-C(=O)-NR^{26b}R^{27b}; -NR^{28b}-(C=O)-R^{29b}; -NR^{30b}-(C=O)-OR^{31b}; -NR^{32b}-S(=O)NR^{33b}R^{34b}; or -S(=O)₂-OR^{35b}; F, Cl, Br, I; -CF₃, -CHF₂, -CH₂F, an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R^{5b}, R^{6b}, R^{7b} and R^{8b}, identical or different, each represent -H; -NO₂; -CN; -N(R^{11b})-SO₂-R^{12b}; -OR^{13b}; -SR^{14b}; -C(=O)-OR^{15b}; -NR^{16b}R^{17b}; - F, Cl, Br, I; -CF₃, -CHF₂, -CH₂F, an alkyl radical selected from the group consisting of methyl, ethyl, n-propyl, iso-propyl, n-butyl, sec-butyl, iso-butyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
in each case with the proviso that at least one of the substituents R^{5b}, R^{6b}, R^{7b} and R^{8b} represents a -N(R^{11b})-S(=O)₂-R^{12b} moiety,
and nb, R^{1b}-R^{4b} and R^{9b}-R^{36b} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Also preferred is the use of compounds of general formula Ib, wherein one of the substituents R^{5b}, R^{6b}, R^{7b} and R^{8b} represents an -N(R^{11b})-S(=O)₂-R^{12b} moiety, preferably one of the substituents R^{5b}, R^{6b}, R^{7b} and R^{8b} represents an -N(R^{11b})-S(=O)₂-R^{12b} moiety while the other three of these substituents each represent a hydrogen atom, and nb, R^{1b}-R^{4b} and R^{9b}-R^{36b} - if present - have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Also preferred is the use of compounds of general formula Ib, wherein R^{9b} and R^{10b}, independent from one another, represent a linear or branched, saturated or unsaturated C₁₋₁₀ aliphatic radical; or an optionally at least mono-substituted 5- to 10-membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R^{9b} and R^{10b}, independent from one another, represent an alkyl radical selected from the group consisting of methyl, ethyl, n-propyl, iso-propyl, n-butyl, sec-butyl, iso-butyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1,2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, CI, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
and nb, R^{1b}-R^{8b} and R^{11b}-R^{36b} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Also preferred is the use of compounds of general formula Ib, wherein
R^{11b} represents a hydrogen atom; a linear or branched, saturated or unsaturated, optionally at least mono-substituted C₁₋₁₀-aliphatic radical; a saturated or unsaturated, optionally at least mono-substituted, 3- to 9-membered cycloaliphatic radical, which may be bonded via a linear or branched C₁₋₆-alkylene, C₂₋₆-alkenylene or C₂₋₆-alkinylene group and/or which may contain 1, 2 or 3 heteroatoms independently selected from the group consisting of nitrogen, oxygen and sulphur as ring members; an optionally at least mono-substituted 5- to 10- membered aryl or heteroaryl radical, which may be bonded via a linear or branched C₁₋₆-alkylene, C₂₋₆₋alkenylene or C₂₋₆-alkinylene group and wherein the heteroaryl radical contains 1, 2 or 3 heteroatoms independently selected from the group consisting of nitrogen, oxygen and sulphur as ring members; or an -S(=O)₂-R^{36b} moiety,
preferably R^{11b} represents a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or an -S(=O)₂-R^{36b}-moiety,
more preferably R^{11b} represents a hydrogen atom,
and nb, R^{1b}-R^{10b} and R^{12b}-R^{36b} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Also preferred is the use of compounds of general formula Ib, wherein R^{12b} represents an optionally at least mono-substituted 5- to 10- membered aryl or heteroaryl radical, which may be bonded via a linear or branched, optionally at least mono-substituted C₁₋₆-alkylene, C₂₋₆-alkenylene or C₂₋₆-alkinylene group and/or which may be condensed with an optionally at least mono-substituted, saturated, unsaturated or aromatic, mono- or bicyclic ring system,
whereby the rings of the ring system are 5- 6- or 7-membered and whereby the heteroaryl radical and optionally one or both of the rings of the ring system contain 1, 2 or 3 heteroatoms independently selected from the group consisting of nitrogen, oxygen and sulphur,
or R^{12b} represents a saturated or unsaturated, optionally at least mono-substituted, 3-to 9-membered cycloaliphatic radical, which may be bonded via a linear or branched C₁₋₆-alkylene, C₂₋₆-alkenylene or C₂₋₆-alkinylene group and/or which may be condensed with an optionally at least mono-substituted mono- or polycyclic ring system,
whereby the rings of the ring system are 5- 6- or 7-membered and whereby the cycloaliphatic radical and optionally one or both of the rings of the ring system may contain 1, 2 or 3 heteroatoms independently selected from the group consisting of nitrogen, oxygen and sulphur,
preferably R^{12b} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, 2-oxo-2,3-dihydro-benzooxazolyl, 2-oxo-2,3-dihydrobenzo[d]thiazolyl, benzooxazolinyl, benzothiazolinyl, benzimidazolidinyl, imidazo[2,1-b]thiazolyl, chromenyl and chromanyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅₋alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -CO-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl.
more preferably R^{12b} represents an aryl or heteroaryl radical selected from the group consisting of phenyl, 1-naphthyl, 2-naphthyl, benzo[b]-thiophenyl, benzo[b]furanyl, quinolinyl, isoquinolinyl, imidazo[2,1-b]thiazolyl, 2-oxo-2,3-dihydro-benzooxazolyl and 2-oxo-2,3-dihydrobenzo[d]thiazolyl, whereby said aryl or heteroaryl radical may be substituted by 1, 2, 3, 4 or 5 substituents independently selected from the group consisting of methyl, ethyl, n-propyl, iso-propyl, n-butyl, sec-butyl, iso-butyl, tert-butyl, F, Cl, Br, I, -CN, -CF₃, -CF₂H, CFH₂, -C(=O)-O-CH₃, C(=O)-O-CH₂-CH₃, cyclopropyl, cyclobutyl, cyclopentyl, cyclohexyl, phenyl, phenoxy and benzyl,
and nb, R^{1b}-R^{11b} and R^{13b}-R^{36b} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Also preferred is the use of compounds of general formula Ib, wherein R^{13b}-R^{35b} independent from one another, each represent a hydrogen atom; a linear or branched C₁₋₁₀ alkyl radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10-membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members;
preferably R^{13b}-R^{35b} independent from one another, represent a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl- (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
and nb, R^{1b}-R^{12b} and R^{36b} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Also preferred is the use of compounds of general formula Ib, wherein R^{36b} represents an optionally at least mono-substituted 5- to 10- membered aryl or heteroaryl radical, which may be bonded via a linear or branched, optionally at least mono-substituted C₁₋₆-alkylene, C₂₋₆-alkenylene or C₂₋₆-alkinylene group and/or which may be condensed with an optionally at least mono-substituted, saturated, unsaturated or aromatic, mono- or bicyclic ring system,
whereby the rings of the ring system are 5- 6- or 7-membered and whereby the heteroaryl radical and optionally one or both of the rings of the ring system contain 1, 2 or 3 heteroatoms independently selected from the group consisting of nitrogen, oxygen and sulphur,
or R^{36b} represents a saturated or unsaturated, optionally at least mono-substituted, 3-to 9-membered cycloaliphatic radical, which may be bonded via a linear or branched C₁₋₆-alkylene, C₂₋₆-alkenylene or C₂₋₆-alkinylene group and/or which may be condensed with an optionally at least mono-substituted mono- or polycyclic ring system,
whereby the rings of the ring system are 5- 6- or 7-membered and whereby the cycloaliphatic radical and optionally one or both of the rings of the ring system may contain 1, 2 or 3 heteroatoms independently selected from the group consisting of nitrogen, oxygen and sulphur,
preferably R^{36b} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, 2-oxo-2,3-dihydro-benzooxazolyl, 2-oxo-2,3-dihydrobenzo[d]thiazolyl, benzooxazolinyl, benzothiazolinyl, benzimidazolidinyl, imidazo[2,1-b]thiazolyl, chromenyl and chromanyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅₋alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -CO-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
and nb and R^{1b}-R^{35b} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Also preferred is the use of compounds of general formula lb, wherein nb is 0 and R^{1b}-R^{36b} have the meaning given above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

In any of the foregoing definitions the following proviso may apply, namely that R^{3b} and R^{4b} do not both identically represent a hydrogen atom.

Particularly preferred is the use of compounds of general formula Ib, wherein
nb is 0,
R^{1b} represents a hydrogen atom,
R^{2b} represents a hydrogen atom,
R^{3b} and R^{4b}, identical or different, represent an alkyl radical selected from the group consisting of methyl, ethyl, n-propyl, iso-propyl, n-butyl, sec-butyl, iso-butyl and tert-butyl,
one of the substituents R^{5b}, R^{6b}, R^{7b} and R^{8b} represents an -N(R^{11b})-S(=O)₂-R^{12b-}moiety while the other three of these substituents each represent a hydrogen atom,
R¹¹ represents a hydrogen atom,
R¹² represents an aryl or heteroaryl radical selected from the group consisting of phenyl, 1-naphthyl, 2-naphthyl, benzo[b]-thiophenyl, benzo[b]furanyl, quinolinyl, isoquinolinyl, imidazo[2,1-b]thiazolyl, 2-oxo-2,3-dihydro-benzooxazolyl and 2-oxo-2,3-dihydrobenzo[d]thiazolyl, whereby said aryl or heteroaryl radical may be substituted by 1, 2, 3, 4 or 5 substituents independently selected from the group consisting of methyl, ethyl, n-propyl, iso-propyl, n-butyl, sec-butyl, iso-butyl, tert-butyl, F, CI, Br, I, -CN, -CF₃, -CF₂H, CFH₂, -C(=O)-O-CH₃, C(=O)-O-CH₂-CH₃, cyclopropyl, cyclobutyl, cyclopentyl, cyclohexyl, phenyl, phenoxy and benzyl.

Most particularly preferred is the use of compounds of general formula Ib selected from the group consisting of
[1] 2-[5-(5-Chloro-3-methyl-benzo[b]thiophene-2-sulfonylamino)-1H-indol-3-yl]-N,N-diethyl-2-oxoacetamide,
[2] N,N-Diethyl-2-[5-(naphthalene-2-sulfonylamino)-1H-indol-3-yl]-2-oxo-acetamide,
[3] N,N-Diethyl-2-[5-(naphthalene-1-sulfonylamino)-1H-indol-3-yl]-2-oxo-acetamide,
[4] 2-[5-(Biphenyl-4-sulfonylamino)-1H-indol-3-yl]-N,N-diethyl-2-oxo-acetamide,
[5] N,N-Diethyl-2-oxo-2-[5-(quinoline-8-sulfonylamino)-1H-indol-3-yl]-acetamide,
[6] N,N-Dimethyl-2-[5-(naphthalene-2-sulfonylamino)-1H-indol-3-yl]-2-oxo-acetamide,
[7] N,N-Dimethyl-2-[5-(naphthalene-1-sulfonylamino)-1H-indol-3-yl]-2-oxo-acetamide,
[8] 2-[5-(5-Chloro-3-methyl-benzo[b]thiophene-2-sulfonylamino)-1H-indol-3-yl]-N,N-dimethyl-2-oxo-acetamide,
[9] 2-[5-(6-Chloro-imidazo[2,1-b]thiazole-5-sulfonylamino)-1H-indol-3-yl]-N,N-diethyl-2-oxo-acetamide,
[10] 2-[5-(6-Chloro-imidazo[2,1-b]thiazole-5-sulfonylamino)-1H-indol-3-yl]-N,N-dimethyl-2-oxo-acetamide,
[11] N,N-Dimethyl-2-[4-(naphthalene-1-sulfonylamino)-1H-indol-3-yl]-2-oxo-acetamide,
[12] 2-[4-(5-Chloro-3-methyl-benzo[b]thiophene-2-sulfonylamino)-1H-indol-3-yl]-N,N-dimethyl-2-oxo-acetamide,
[13] 2-[4-(6-Chloro-imidazo[2,1-b]thiazole-5-sulfonylamino)-1H-indol-3-yl]-N,N-dimethyl-2-oxo-acetamide,
[14] N,N-Dimethyl-2-[5-[(4-fluoro-3-methyl-phenyl)-1-sulfonylamino]-1H-indol-3-yl]-2-oxo-acetamide,
[15] 5-(3-Dimethylaminooxalyl-1H-indol-5-ylsulfamoyl)-3-methyl-benzofuran-2-carboxylic acid ethyl ester,
[16] 2-[5-(Biphenyl-4-sulfonylamino)-1H-indol-3-yl]-N,N-dimethyl-2-oxo-acetamide,
[17] N,N-Dimethyl-2-oxo-2-[5-(2-oxo-2,3-dihydro-benzoxazole-6-sulfonylamino)-1 H-indol-3-yl]-acetamide,
[18] N,N-Dimethyl-2-oxo-2-[5-(2-oxo-2,3-dihydrobenzo[d]thiazole-6-sulfonamido)-1 H-indol-3-yl]acetamide,
[19] 2-[5-[(4-Cyclohexyl-phenyl)-1-sulfonylamino)-1H-indol-3-yl]-N,N-dimethyl-2-oxo-acetamide and
[20] N,N-Dimethyl-2-[5-[(4-phenoxy-phenyl)-1-sulfonylamino]-1H-indol-3-yl]-2-oxo-acetamide.

Preferred is the afore mentioned use for the preparation of a medicament for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity, or for the prophylaxis and/or treatment of irritable colon syndrome; disorders of the central nervous system; anxiety; panic attacks; depression; bipolar disorders; cognitive disorders; memory disorders; senile dementia; psychosis; neurodegenerative disorders, preferably selected from the group consisting of Morbus Alzheimer, Morbus Parkinson, Morbus Huntington and Multiple Sclerosis; schizophrenia; psychosis; hyperactivity disorder (ADHD, attention deficit/hyperactivity disorder), or for improvement of cognition (cognitive enhancement).

Particularly preferred is the afore mentioned use for the preparation of a medicament for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

Most particularly preferred is the afore mentioned use for the preparation of a medicament for the prophylaxis and/or treatment of obesity and/or disorders or diseases related thereto.

The substituted indole compounds of general formulas la and lb and corresponding stereoisomers may be obtained analogous to the methods described above for the preparation of the substituted indole compounds of general formulas I and Ic. Any of the substituted indole compounds of general formulas I, Ia, Ib and Ic described herein may also be obtained analogous to the methods described in US 2003/0181482 A1. The respective part of the description is hereby incorporated by reference and forms part of the disclosure.

A mono- or polycyclic ring-system according to the present invention - if not defined otherwise - means a mono- or polycyclic hydrocarbon ring-system that may be saturated, unsaturated or aromatic. If the ring system is polycyclic, e.g. bicyclic, each of its different rings may show a different degree of saturation, i.e. it may be saturated, unsaturated or aromatic. Optionally each of the rings of the mono- or polycyclic ring system may contain one or more, preferably 1, 2 or 3, heteroatoms as ring members, which may be identical or different and which can preferably be selected from the group consisting of N, 0, S and P, more preferably be selected from the group consisting of N, O and S. Preferably the polycyclic ring-system may comprise two rings that are condensed. The rings of the mono- or polycyclic ring-sytem are preferably 5-, 6- or 7-membered.
The term "condensed" according to the present invention means that a ring or ring-system is attached to another ring or ring-system, whereby the terms "annulated" or "annelated" are also used by those skilled in the art to designate this kind of attachment.

Such a mono- or polycyclic ring-system may - if not defined otherwise - be unsubstituted or substituted by one or more substituents, preferably unsubstituted or substituted with 1, 2, 3, 4 or 5 substituents. Said substituents may preferably be selected independently from the group consisting of C₁₋₅-alkyl, -O-C₁₋₅-alkyl, -S-C₁₋₅₋alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl, whereby in each occurence C₁₋₅-alkyl may be linear or branched and whereby said cyclic substituents may be unsubstituted or substituted by 1, 2 or 3 substituents independently selected from the group consisting of methyl, ethyl, n-propyl, iso-propyl, methoxy, ethoxy, F, Cl, Br, -CN, -CF₃, -OCF₃, -SCF₃, -OH, -SH, -NH₂ and NO₂.

If any of the substituents represents or comprises a cycloaliphatic radical (cycloaliphatic group), said cycloaliphatic radical may - if not defined otherwise - be unsubstituted or substituted by one or more substituents, preferably unsubstituted or substituted with 1, 2, 3, 4 or 5 substituents. Said substituents may preferably be selected independently from the group consisting of C₁₋₅-alkyl, -O-C₁₋₅-alkyl, -S-C₁₋₅₋alkyl, -C(=O)-O-C₁₋₅-alkyl, -O-(C=O)-C₁₋₅-alkyl, oxo (=O), F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl, whereby in each occurence C₁₋₅-alkyl may be linear or branched and whereby said cyclic substituents may be unsubstituted or substituted by 1, 2 or 3 substituents independently selected from the group consisting of methyl, ethyl, n-propyl, iso-propyl, methoxy, ethoxy, F, Cl, Br, -CN, -CF₃, -OCF₃, -SCF₃, -OH, -SH, -NH₂ and NO_{2.}

If any of the substituents represents or comprises a cycloaliphatic radical, which contains one or more, preferably 1, 2 or 3, heteroatoms as ring members, unless defined otherwise, each of these heteroatoms may preferably be selected from the group consisting of of N, 0 and S.

Suitable cycloaliphatic radicals, which may be unsubstituted or at least mono-substituted, include cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl.

If any of the substituents represents or comprises an aryl radical (aryl group), including a phenyl or naphthyl group, said aryl radical may - if not defined otherwise - be unsubstituted or substituted by one or more substituents, preferably unsubstituted or substituted with 1, 2, 3, 4 or 5 substituents. Said substituents may preferably be selected independently from the group consisting of C₁₋₅-alkyl, -O-C₁₋₅-alkyl, -S-C₁₋₅₋alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl, whereby in each occurence C₁₋₅-alkyl may be linear or branched and whereby said cyclic substituents may be unsubstituted or substituted by 1, 2 or 3 substituents independently selected from the group consisting of methyl, ethyl, n-propyl, iso-propyl, methoxy, ethoxy, F, Cl, Br, -CN, -CF₃, -OCF₃, -SCF₃, -OH, -SH, -NH₂ and NO_{2.}

Preferred aryl radicals, which may optionally be at least mono-substituted, are phenyl and naphthyl.

If any of the substituents represents or comprises a heteroaryl radical (heteroaryl group), said heteroaryl radical may - if not defined otherwise - be unsubstituted or substituted by one or more substituents, preferably unsubstituted or substituted with 1, 2, 3, 4 or 5 substituents. Said substituents may preferably be selected independently from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl, whereby in each occurence C₁₋₅-alkyl may be linear or branched and whereby said cyclic substituents may be unsubstituted or substituted by 1, 2 or 3 substituents independently selected from the group consisting of methyl, ethyl, n-propyl, iso-propyl, methoxy, ethoxy, F, Cl, Br, -CN, -CF₃, -OCF₃, -SCF₃, -OH, -SH, -NH₂ and NO₂.

The heteroatoms, which are present as ring members in the heteroaryl radical, may, unless defined otherwise, independently be selected from the group consisting of nitrogen, oxygen and sulphur. Preferably the heteroaryl radical comprises 1, 2 or 3 heteroatoms.

Suitable heteroaryl radicals, which may optionally be at least mono-substituted, may preferably be selected from the group consisting of furyl- (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl.

If any of the substituents represents a saturated or unsaturated aliphatic radical (aliphatic group), i.e. an alkyl radical, an alkenyl radical or an alkinyl radical, said aliphatic radical may - if not defined otherwise - be unsubstituted or substituted by one or more substituents, preferably unsubstituted or substituted with 1, 2, 3, 4 or 5 substituents. Said substituents may preferably be selected independently from the group consisting of -O-C₁₋₅-alkyl, -S-C₁₋₅-alkyl, -(C=O)-O-C₁₋₅-alkyl, -O-(C=O)-C₁₋₅₋alkyl, -F, Cl, Br, I, -CN, -CF₃, -OCF₃, -SCF₃, -OH, -SH, -NH₂, -NH(C₁₋₅-alkyl) and -N(C₁₋₅-alkyl)₂, whereby in each occurence C₁₋₅-alkyl may be linear or branched. An alkenyl radical comprises at least one carbon-carbon double bond, an alkinyl radical comprises at least one carbon-carbon triple bond.

Suitable aliphatic radicals, which may be substituted by one or more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl, n-decyl, vinyl, ethinyl, propenyl, propinyl, butenyl and butinyl.

If any of the substituents represents an alkylene group, an alkenylene group or an alkinylene group, which may be substituted, said alkylene group, alkenylene group or alkinylene group may - if not defined otherwise - be unsubstituted or substituted by one or more substituents, preferably unsubstituted or substituted with 1, 2 or 3 substituents. Said substituents may preferably be selected independently from the group consisting of -O-C₁₋₅-alkyl, -S-C₁₋₅-alkyl, -(C=O)-O-C₁₋₅-alkyl, -O-(C=O)-C₁₋₅₋alkyl, -F, Cl, Br, I, -CN, -CF₃, -OCF₃, -SCF₃, -OH, -SH, -NH₂, -NH(C₁₋₅-alkyl) and -N(C₁₋₅-alkyl)₂ , whereby in each occurence C₁₋₅-alkyl may be linear or branched. An alkenylene group comprises at least one carbon-carbon double bond, an alkinylene group comprises at least one carbon-carbon triple bond.

Suitable alkylene groups include -(CH₂)-, -(CH₂)₂-, -(CH₂)₃-,-(CH₂)₄-,-(CH₂)₅-,-(CH₂)₆-, suitable alkenylene groups include -CH=CH-, -CH₂-CH=CH- and -CH=CH-CH₂- and suitable alkinylene groups include -C≡C- , -CH₂-C≡C- and -C≡C-CH₂-.

The substituted indole derivatives of general formulas I, Ia, Ib and Ic and in each case stereoisomers thereof may be obtained in form of a corresponing salt according to methods well known to those skilled in the art, e.g. by reacting said compound with at least one inorganic and/or organic acid, preferably in a suitable reaction medium. Suitable reaction media include, for example, any of the ones given above. Suitable inorganic acids include but are not limited to hydrochloric acid, hydrobromic acid, phosphoric acid, sulfuric acid, nitric acid, suitable organic acids include but are not limited to citric acid, maleic acid, fumaric acid, tartaric acid, or derivatives thereof, p-toluenesulfonic acid, methanesulfonic acid or camphersulfonic acid.

Solvates, preferably hydrates, of the substituted indole compounds of general formulas I, Ia, Ib and Ic or in each case of corresponding stereoisomers may also be obtained by standard procedures known to those skilled in the art.

Any medicament according to the present invention may be in any form suitable for the application to humans and/or animals, preferably humans including infants, children and adults. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may, for example, be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or retarded release. The multiparticulate forms, such as pellets or granules, may e.g. be filled into a capsule, compressed into tablets or suspended in a suitable liquid.

Suitable controlled release formulations, materials and methods for their preparation are are known from the prior art, e.g. from the table of contents of"Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments according to the present invention may also comprise an enteric coating, so that their dissolution is dependent on pH-value. Due to said coating the medicament can pass the stomach undissolved and the respective indole compound is liberated in the intestinal tract. Preferably the enteric coating is soluble at a pH value of 5 to 7.5. Suitable materials and methods for the preparation are are known from the prior art.

Typically, the medicaments according to the present invention may contain 1-60 % by weight of one or more substituted indole compounds as defined herein and 40-99 % by weight of one or more auxiliary substances (additives).

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

In the following methods for determining the pharmacological activity of the substituted indole compounds are described.

### PHARMACOLOGICAL METHODS:

### I) BINDING TO SEROTONIN RECEPTOR 5-HT₆

Cell membranes of HEK-293 cells expressing the 5HT₆-human recombinant receptor were supplied by Receptor Biology. In said membranes the receptor concentration is 2.18 pmol/mg protein and the protein concentration is 9.17 mg/ml. The experimental protocol follows the method of B. L. Roth et al. [B. L. Roth, S. C. Craigo, M. S. Choudhary, A. Uluer, F. J. Monsma, Y. Shen, H. Y. Meltzer, D. R. Sibley: Binding of Typical and Atypical Antipsychotic Agents to 5-Hydroxytryptamine-6 and Hydroxytryptamine-7 Receptors. The Journal of Pharmacology and Experimental Therapeutics, 1994, 268, 1403] with the following slight changes. The respective part of the literature description is hereby incorporated by reference and forms part of the disclosure.

The commercial membrane is diluted (1:40 dilution) with the binding buffer: 50 mM Tris-HCl, 10 mM MgCl₂, 0.5 mM EDTA (pH 7.4). The radioligand used is [³H]-LSD at a concentration of 2.7 nM with a final volume of 200 µl. Incubation is initiated by adding 100 µl of membrane suspension, (≈ 22.9 µg membrane protein), and is prolonged for 60 minutes at a temperature of 37 °C. The incubation is ended by fast filtration in a Brandel Cell Harvester through fiber glass filters made by Schleicher & Schuell GF 3362 pretreated with a solution of polyethylenimine at 0.5 %. The filters are washed three times with three milliliters of buffer Tris-HCl 50 mM pH 7.4. The filters are transferred to flasks and 5 ml of Ecoscint H liquid scintillation cocktail are added to each flask. The flasks are allowed to reach equilibrium for several hours before counting with a Wallac Winspectral 1414 scintillation counter. Non-specific binding is determined in the presence of 100 µM of serotonin. Tests were made in triplicate. The inhibition constants (Kᵢ, nM) were calculated by non-linear regression analysis using the program EBDA/LIGAND described in Munson and Rodbard, Analytical Biochemistry, 1980, 107, 220, the respective part of which is hereby incorporated by reference and forms part of the disclosure.

### II.) FOOD INTAKE MEASUREMENT (BEHAVIOURAL MODEL):

Male W rats (200-270 g) obtained from Harlan, S.A. are used. The animals are acclimatized to the animal facility for at least 5 days before they are subjected to any treatment. During this period the animals are housed (in groups of five) in translucid cages and provided with food and water ad libitum. At least 24 hours before the treatment starts, the animals are adapted to single-housing conditions.

The acute effect of the substituted indole compounds according to the present invention in fasted rats is then determined as follows:

The rats were fasted for 23 hours in their single homecages. After this period, the rats are orally or intraperitoneally dosed with a composition comprising a substituted indole compound or a corresponding composition (vehicle) without said substituted indole compound. Immediately afterwards, the rat is left with preweighed food and cumulative food intake is measured after 1, 2, 4 and 6 hours.

Said method of measuring food intake is also described in the literature publications of Kask et al., European Journal of Pharmacology 414 (2001), 215-224 and Turnbull et al., Diabetes, Vol. 51, August 2002. The respective parts of the descriptions are hereby incorporated by reference and form part of the disclosure.

The present invention is illustrated below with the aid of examples. These illustrations are given solely by way of example and do not limit the general spirit of the present invention.

### Examples:

### Example 1. Preparation of 2-[5-(5-Chloro-3-methyl-benzo[b]thiophene-2-sulfonylamino)-1H-indol-3-yl]-N,N-diethyl-2-oxoacetamide.

155 mg (0.60 mmol) 2-(5-amino-1 H-indol-3-yl)-N,N-diethyl-2-oxoacetamide and 116 mg N-Diisopropylethylamine were dissolved in 2 ml dimethylformamide and 185.5 mg (0.66 mmol) of 5-chloro-3-methylbenzo[b]thiophene-2-sulfonyl chloride were slowly added. The reaction mixture was stirred at ambient temperature (approximately 20 °C) for 3 hours and the solvent was removed in vacuo. The residue was treated with an aqueous NaHCO₃ solution and extracted with chloroform. The combined organic phases were subsequently washed with water and an aqueous NaHCO₃ solution and dried over Na₂SO₄. After removing the solvent a solid residue was obtained that was purified by conventional column chromatography to yield 121 mg (40 % of theory) of the desired product in form of a solid.

### Example 2. Preparation of N,N-Diethyl-2-[5-(naphthalene-2-sulfonylamino)-1H-indol-3-yl]-2-oxo-acetamide.

The reaction was carried out according to the procedure given in Example 1. Starting from 155 mg (0.60 mmol) 2-(5-amino-1H-indol-3-yl)-N,N-diethyl-2-oxoacetamide and 149,5 mg (0.66 mmol) naphthalene-2-sulfonyl chloride, 138 mg (51 % of theory) of the desired product were obtained in form of a solid.

### Example 3. Preparation of N,N-Diethyl-2-[5-(naphtalene-1-sulfonylamino)-1H-indol-3-yl]-2-oxo-acetamide.

The reaction was carried out according to the procedure given in Example 1. Starting from 155 mg (0.60 mmol) 2-(5-amino-1H-indol-3-yl)-N,N-diethyl-2-oxoacetamide and 149,5 mg (0.66 mmol) naphthalene-1-sulfonyl chloride, 120 mg (49 % of theory) of the desired product were obtained in form of a solid.

### Example 4. Preparation of 2-[5-(Biphenyl-4-sulfonylamino)-1H-indol-3-yl]-N,N-diethyl-2-oxo-acetamide.

The reaction was carried out according to the procedure given in Example 1. Starting from 155 mg (0.60 mmol) 2-(5-amino-1H-indol-3-yl)-N,N-diethyl-2-oxoacetamide and 167 mg (0.66 mmol) phenyl-benzene-4-sulfonyl chloride, 126 mg (46 % of theory) of the desired product were obtained in form of a solid.

### Example 5. Preparation of N,N-Diethyl-2-oxo-2-[5-(quinoline-8-sulfonylamino)-1 H-indol-3-yl]-acetamide.

The reaction was carried out according to the procedure given in Example 1. Starting from 155 mg (0.60 mmol) 2-(5-amino-1H-indol-3-yl)-N,N-diethyl-2-oxoacetamide and 168 mg (0.66 mmol) quinoline-8-sulfonyl chloride, 72 mg (26 % of theory) of the desired product were obtained in form of a solid.

### Example 6. Preparation of N,N-Dimethyl-2-[5-(naphthalene-2-sulfonylamino)-1H-indol-3-yl]-2-oxo-acetamide.

The reaction was carried out according to the procedure given in Example 1. Starting from 138 mg (0.60 mmol) 2-(5-amino-1H-indol-3-yl)-N,N-dimethyl-2-oxoacetamide and 149,5 mg (0.66 mmol) naphthalene-2-sulfonyl chloride, 100 mg (40 % of theory) of the desired product were obtained in form of a solid.

### Example 7. Preparation of N,N-Dimethyl-2-[5-(naphtalene-1-sulfonylamino)-1H-indol-3-yl]-2-oxo-acetamide.

The reaction was carried out according to the procedure given in Example 1. Starting from 138 mg (0.60 mmol) 2-(5-amino-1 H-indol-3-yl)-N,N-dimethyl-2-oxoacetamide and 149,5 mg (0.66 mmol) naphthalene-1-sulfonyl chloride, 71 mg (28 % of theory) of the desired product were obtained in form of a solid.

### Example 8. Preparation of 2-[5-(5-Chloro-3-methyl-benzo[b]thiophene-2-sulfonyl-amino)-1 H-indol-3-yl]-N,N-dimethyl-2-oxo-acetamide.

The reaction was carried out according to the procedure given in Example 1. Starting from 138 mg (0.60 mmol) 2-(5-amino-1H-indol-3-yl)-N,N-dimethyl-2-oxoacetamide and 185 mg (0.66 mmol) 5-chloro-3-methyl-benzo[b]thiophene-2-sulfonyl chloride, 120 mg (42 % of theory) of the desired product were obtained in form of a solid.

### Example 9. Preparation of 2-[5-(6-Chloro-imidazo[2,1-b]thiazole-5-sulfonylamino)-1H-indol-3-yl]-N,N-diethyl-2-oxo-acetamide.

The reaction was carried out according to the procedure given in Example 1. Starting from 155 mg (0.60 mmol) 2-(5-amino-1H-indol-3-yl)-N,N-diethyl-2-oxoacetamide and 170 mg (0.66 mmol) 6-chloro-imidazo[2,1-b]thiazole-5-sulfonyl chloride, 71 mg (25 % of theory) of the desired product were obtained in form of a solid.

### Example 10. Preparation of 2-[5-(6-Chloro-imidazo[2,1-b]thiazole-5-sulfonylamino)-1 H-indol-3-yl]-N,N-dimethyl-2-oxo-acetamide.

The reaction was carried out according to the procedure given in Example 1. Starting from 138 mg (0.60 mmol) 2-(5-amino-1H-indol-3-yl)-N,N-dimethyl-2-oxoacetamide and 170 mg (0.66 mmol) 6-chloro-imidazo[2,1-b]thiazole-5-sulfonyl chloride, 35 mg (13 % of theory) of the desired product were obtained in form of a solid.

No attempts have been made to improve the yields of the compounds.

The melting point and ¹H-NMR data for the compounds according to the examples 1 to 10 are given in the following table :

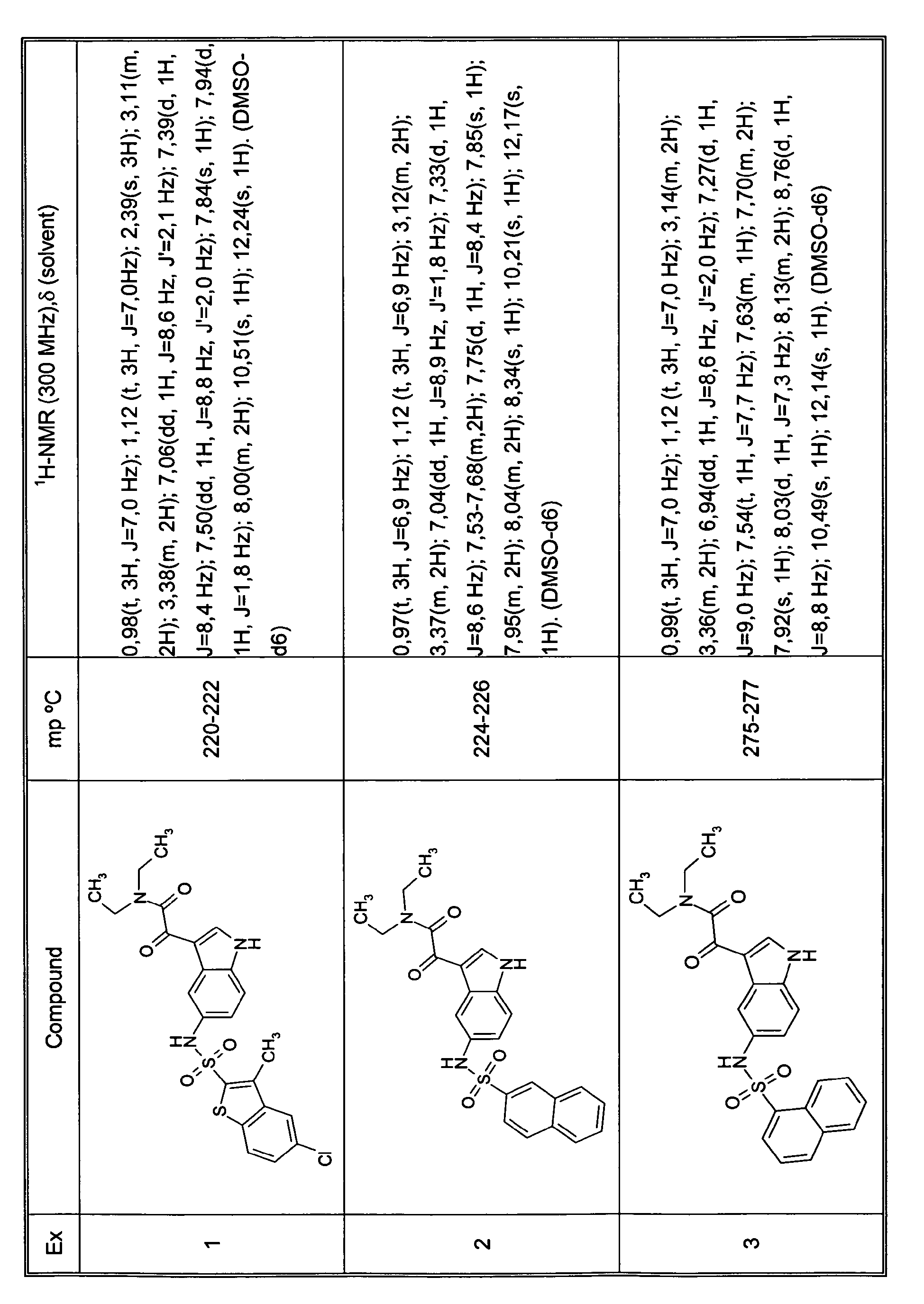

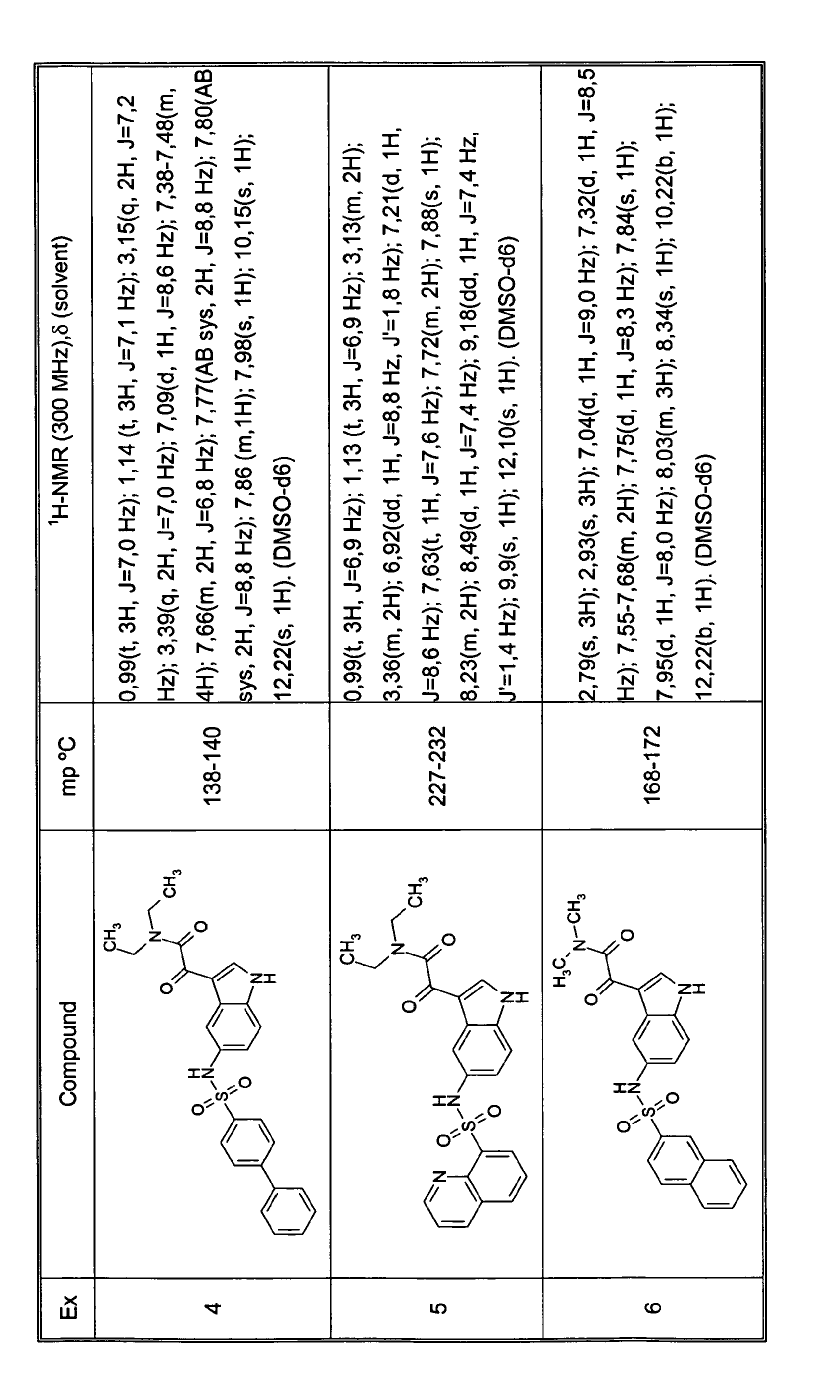

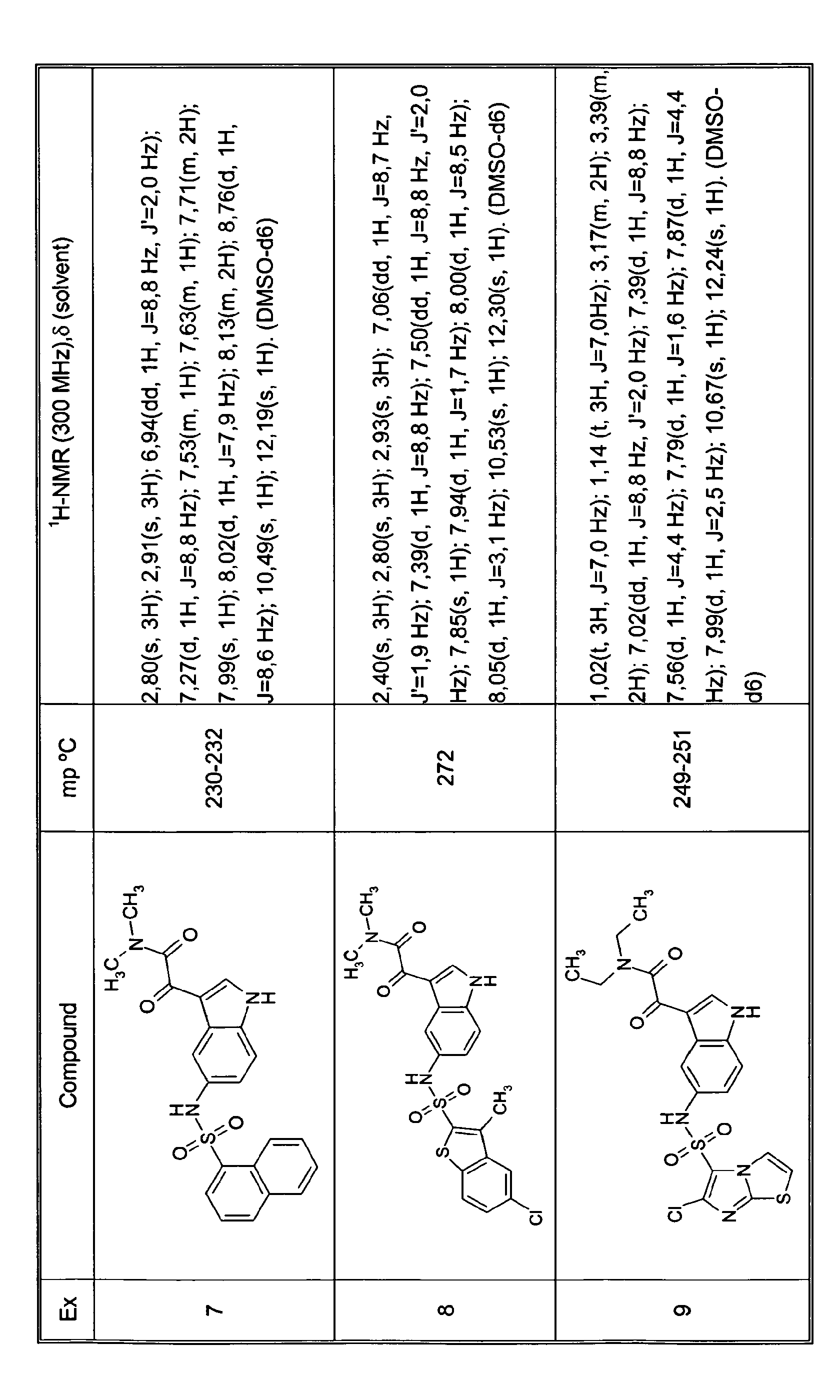

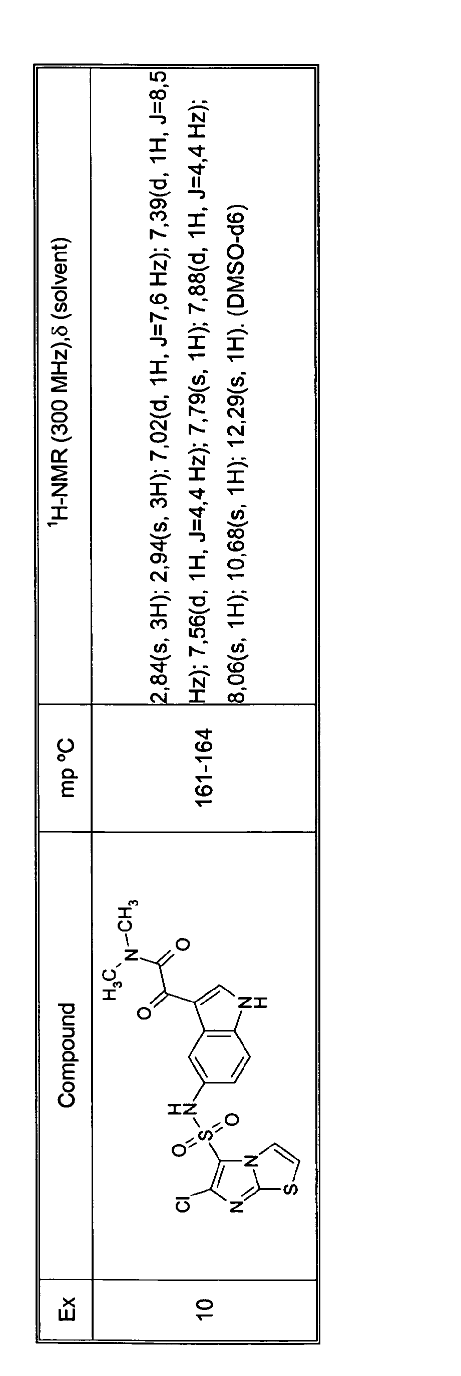

The compounds of the examples 11-20 have been prepared accordingly. Those skilled in the art will realize which starting materials were used to obtain the respective compounds.

### Example 11.

N,N-Dimethyl-2-[4-(naphthalene-1-sulfonylamino)-1H-indol-3-yl]-2-oxo-acetamide.

### Example 12.

2-[4-(5-Chloro-3-methyl-benzo[b]thiophene-2-sulfonylamino)-1H-indol-3-yl]-N,N-dimethyl-2-oxo-acetamide.

### Example 13:

2-[4-(6-Chloro-imidazo[2,1-b]thiazole-5-sulfonylamino)-1H-indol-3-yl]-N,N-dimethyl-2-oxo-acetamide.

### Example 14.

N,N-Dimethyl-2-[5-[(4-fluoro-3-methyl-phenyl)-1-sulfonylamino]-1H-indol-3-yl]-2-oxo-acetamide.

### Example 15:

5-(3-Dimethylaminooxalyl-1 H-indol-5-ylsulfamoyl)-3-methyl-benzofuran-2-carboxylic acid ethyl ester.

### Example 16:

2-[5-(Biphenyl-4-sulfonylamino)-1H-indol-3-yl]-N,N-dimethyl-2-oxo-acetamide.

### Example 17:

N,N-Dimethyl-2-oxo-2-[5-(2-oxo-2,3-dihydro-benzoxazole-6-sulfonylamino)-1 H-indol-3-yl]-acetamide.

### Example 18:

N,N-Dimethyl-2-oxo-2-[5-(2-oxo-2,3-dihydrobenzo[d]thiazole-6-sulfonamido)-1H-indol-3-yl]acetamide.

### Example 19:

2-[5-[(4-Cyclohexyl-phenyl)-1-sulfonylamino]-1 H-indol-3-yl]-N, N-d imethyl-2-oxo-acetamide.

### Example 20:

N,N-Dimethyl-2-[5-[(4-phenoxy-phenyl)-1-sulfonylamino]-1H-indol-3-yl]-2-oxo-acetamide.

### Example 21:

Tablet comprising a substituted indole compound.

### Formula per tablet:

| | |
|---|---|
| Compound according to example 1 | 5 mg |
| Lactose | 60 mg |
| Crystalline cellulose | 25 mg |
| K 90 Povidone (Polyvinylpyrrolidone) | 5 mg |
| Pregelatinised starch | 3 mg |
| Colloidal silicon dioxide | 1 mg |
| Magnesium stearate | 1 mg |
| Total weight per tablet | 100 mg |

### Pharmacological data:

The binding of the substituted indole compounds to the 5-HT₆ receptor was determined as described above.

The binding results for some of these compounds are given in the following table :

| **Compound according to example:** | **K**_{**i**} **(nM)** |
|---|---|
| 9 | 224 |
| 10 | 18.4 |

## Claims

1. A substituted indole compound of general formula I 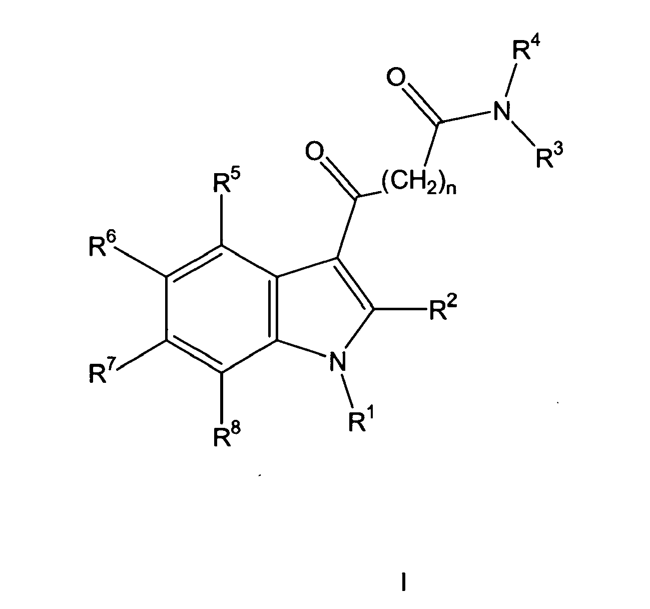 wherein
n represents 0, 1, 2, 3 or 4,
R¹ represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; an optionally at least mono-substituted aryl or heteroaryl radical, which may be bonded via a linear or branched alkylene group; -S(=O)₂-R⁹; or -C(=O)-R¹⁰,
for n = 0: R² represents -NO₂; -NH₂; -SH; -OH; -CN; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
for n = 1, 2, 3 or 4: R² represents -H, -NO₂; -NH₂; -SH; -OH; -CN; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R³ and R⁴, identical or different, represent a hydrogen atom; a linear or branched, saturated or unsaturated aliphatic radical, an optionally at least mono-substituted aryl or heteroaryl radical, which may be bonded via a linear or branched alkylene group; a saturated or unsaturated, optionally at least mono-substituted, optionally at least one heteroatom as a ring member containing cycloaliphatic radical, which may be bonded via a linear or branched alkylene group and/or which may be condensed with an optionally at least mono-substituted mono- or polycyclic ring system, or
R³ and R⁴ together with the bridging nitrogen form an optionally at least mono-substituted, saturated, unsaturated or aromatic heterocyclic ring that may contain at least one further heteroatom as a ring member and/or that may be condensed with an optionally at least mono-substituted mono- or polycyclic ring-system,
R⁵, R⁶, R⁷ and R⁸, identical or different, represent -H; -NO₂; -CN; -N(R¹¹)-S(=O)₂-R¹²; -OR¹³; -SR¹⁴; -C(=O)-OR¹⁵; -NR¹⁶R¹⁷; -C(=O)-R¹⁸; - (C=O)-NR¹⁹R²⁰; -O-(C=O)-R²¹; -S(=O)₂-R²²; -S(=O)₂-NR²³R²⁴; -NR²⁵-C(=O)-NR²⁶R²⁷; -NR²⁸-(C=O)-R²⁹; -NR³⁰-(C=O)-OR³¹; -NR³²-S(=O)NR³³R³⁴; or - S(=O)₂-OR³⁵; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
with the proviso that at least one of the substituents R⁵, R⁶, R⁷ and R⁸ represents an -N(R¹¹)-S(=O)₂-R¹² moiety,
R⁹ and R¹⁰, independent from one another, represent a linear or branched, saturated or unsaturated, optionally at least mono-substituted aliphatic radical; or an optionally at least mono-substituted aryl or heteroaryl radical, which may be bonded via a linear or branched alkylene group,
R¹¹ represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group; an optionally at least mono-substituted aryl or heteroaryl radical, which may be bonded via a linear or branched, optionally at least mono-substituted alkylene, alkenylene or alkinylene group; or an - S(=O)₂-R³⁶ moiety,
R¹² represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system; or a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system,
R¹³-R³⁵, independent from one another, represent a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R³⁶ represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system; or a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system,
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

2. A compound according to claim 1, **characterized in that**
R¹ represents a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆₋alkylene group; an optionally at least mono-substituted 5- to 14- membered aryl or heteroaryl radical, which may be bonded via a linear or branched C₁₋₆₋alkylene group; -S(=O)₂-R⁹; or -C(=O)-R¹⁰,
for n = 0: R² represents -NO₂; -NH₂; -SH; -OH; -CN; a halogen atom; a linear or branched, saturated or unsaturated, optionally at least mono-substituted, optionally at least one heteroatom as a chain member containing C₁₋₁₀ aliphatic radical; a saturated or unsaturated, optionally at least mono-substituted, optionally at least one heteroatom as a ring member containing 3-to 9-membered cycloaliphatic radical, which may be bonded via a linear or branched C₁₋₆-alkylene group; or an optionally at least mono-substituted 5- to 14-membered aryl or heteroaryl radical, which may be bonded via a linear or branched C₁₋₆-alkylene group,
for n = 1, 2, 3 or 4: R² represents -H; -NO₂; -NH₂; -SH; -OH; -CN; a halogen atom; a linear or branched, saturated or unsaturated, optionally at least mono-substituted, optionally at least one heteroatom as a chain member containing C₁₋₁₀ aliphatic radical; a saturated or unsaturated, optionally at least mono-substituted, optionally at least one heteroatom as a ring member containing 3-to 9-membered cycloaliphatic radical, which may be bonded via a linear or branched C₁₋₆-alkylene group; or an optionally at least mono-substituted 5- to 14-membered aryl or heteroaryl radical, which may be bonded via a linear or branched C₁₋₆-alkylene group,
R³ and R⁴, identical or different, represent a hydrogen atom; a linear or branched, saturated or unsaturated C₁₋₁₀ aliphatic radical; an optionally at least mono-substituted 5- to 14-membered aryl or heteroaryl radical, which may be bonded via a linear or branched C₁₋₆ alkylene group; a saturated or unsaturated, optionally at least mono-substituted, optionally at least one heteroatom as a ring member containg 3- to 9-membered cycloaliphatic radical, which may be bonded via a linear or branched C₁₋₆ alkylene group and/or which may be condensed with an optionally at least mono-substituted mono- or polycyclic ring system, or
R³ and R⁴ together with the bridging nitrogen form an optionally at least mono-substituted, saturated, unsaturated or aromatic 4- to 9-membered heterocyclic ring that may contain at least one further heteroatom as a ring member and/or that may be condensed with an optionally at least mono-substituted mono- or polycyclic ring-system,
R⁵, R⁶, R⁷ and R⁸, identical or different, represent -H; -NO₂; -CN; -N(R¹¹)-S(=O)₂-R¹²; -OR¹³; -SR¹⁴; -C(=O)-OR¹⁵; -NR¹⁶R¹⁷; -C(=O)-R¹⁸; -(C=O)-NR¹⁹R²⁰; -O-(C=O)-R²¹; -S(=O)₂-R²²; -S(=O)₂-NR²³R²⁴; -NR²⁵-C(=O)-NR²⁶R²⁷; -NR²⁸-(C=O)-R²⁹; -NR³⁰-(C=O)-OR³¹; -NR³²-S(=O)NR³³R³⁴; or -S(=O)₂-OR³⁵; a halogen atom; a linear or branched, saturated or unsaturated, optionally at least mono-substituted, optionally at least one heteroatom as a chain member containing C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆-alkylene group,
with the proviso that at least one of the substituents R⁵, R⁶, R⁷ and R⁸ represents an -N(R¹¹)-S(=O)₂-R¹² moiety,
R⁹ and R¹⁰, independent from one another, represent a linear or branched, saturated or unsaturated, optionally at least mono-substituted C₁₋₁₀ aliphatic radical; or an optionally at least mono-substituted, 5- to 14-membered aryl or heteroaryl radical, which may be bonded via a linear or branched C₁₋₆-alkylene group,
R¹¹ represents a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group; an optionally at least mono-substituted 5- to 14 membered aryl or heteroaryl radical, which may be bonded via a linear or branched, optionally at least mono-substituted C₁₋₆ alkylene, C₂₋₆ alkenylene or C₂₋₆ alkinylene group; or an -S(=O)₂-R³⁶ moiety,
R¹² represents an optionally at least mono-substituted 5- to 14-membered aryl or heteroaryl radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system; or a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system,
R¹³-R³⁵, independent from one another, represent a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3-to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆ alkylene group,
R³⁶ represents an optionally at least mono-substituted 5- to 14-membered aryl or heteroaryl radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system; or a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system.

3. A compound according to claim 1 or 2, **characterized in that** R¹ represents a hydrogen atom; a linear or branched C₁₋₁₀ alkyl radical; a saturated or unsaturated, optionally at least mono-substituted, 3- to 9-membered cycloaliphatic radical, which may be bonded via a linear or branched C₁₋₆₋alkylene group and/or which may contain 1, 2 or 3 heteroatoms independently selected from the group consisting of nitrogen, oxygen and sulphur as ring members; an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members; -S(=O)₂-R⁹; or -C(=O)-R¹⁰,
preferably R¹ represents a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; -S(=O)₂-R⁹; or -C(=O)-R¹⁰.

4. A compound according to one or more of claims 1-3, **characterized in that**
for n = 0
R² represents -NO₂; -NH₂; -SH; -OH; -CN; -CF₃; -OCH₃; -O-CH₂-CH₃; F; CI; Br; I; a linear or branched, saturated or unsaturated C₁₋₁₀ aliphatic radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10-membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R² represents -NO₂; -NH₂; -SH; -OH; -CN; -CF₃; -OCH₃; -O-CH₂₋CH₃; F; Cl; Br; I; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl, and
for n = 1, 2, 3 or 4
R² represents -H; -NO₂; -NH₂; -SH; -OH; -CN; -CF₃; -OCH₃; -O-CH₂-CH₃; F; Cl; Br; I; a linear or branched, saturated or unsaturated C₁₋₁₀ aliphatic radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10-membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R² represents -H; -NO₂; -NH₂; -SH; -OH; -CN; -CF₃; -OCH₃; -0-CH₂-CH₃; F; Cl; Br; I; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of linear or branched C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R² represents a hydrogen atom.

5. A compound according to one or more of claims 1-4, **characterized in that**
R³ and R⁴, identical or different, represent a hydrogen atom or a linear or branched C₁₋₈ alkyl radical, or
R³ and R⁴ together with the bridging nitrogen form an optionally at least mono-substituted, saturated, unsaturated or aromatic 4- to 9-membered heterocyclic ring that may be condensed with an optionally at least mono-substituted mono- or bicyclic ring-system,
whereby the rings of the ring system are 5- 6- or 7-membered and whereby the heterocyclic ring and one or both of the rings of the ring system may contain 1, 2 or 3 heteroatoms independently selected from the group consisting of nitrogen, oxygen and sulphur,
preferably
R³ and R⁴, identical or different, represent a hydrogen atom or an alkyl radical selected from the group consisting of methyl, ethyl, n-propyl, iso-propyl, n-butyl, sec-butyl, iso-butyl and tert-butyl; or
R³ and R⁴ together with the bridging nitrogen atom form a moiety selected from the group consisting of 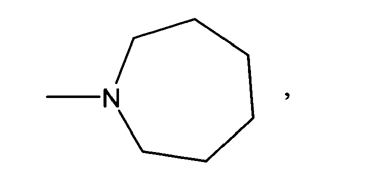 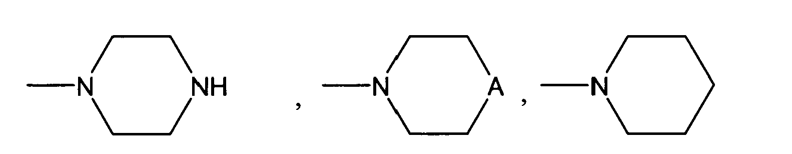 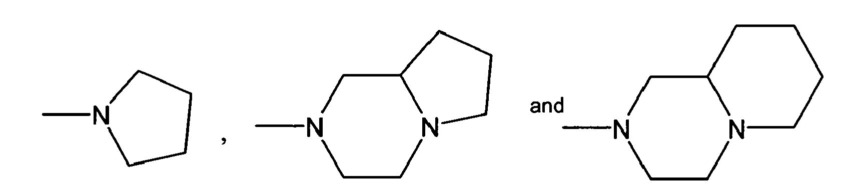 whereby A represents an oxygen atom or a sulphur atom and whereby each of these afore mentioned cyclic moieties may be substituted with 1, 2 or 3 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl in any position including the nitrogen atoms of the piperazine ring,
more preferably R³ and R⁴, identical or different, represent a hydrogen atom or an alkyl radical selected from the group consisting of methyl, ethyl, n-propyl, iso-propyl, n-butyl, sec-butyl, iso-butyl and tert-butyl.

6. A compound according to one or more of claims 1-5, **characterized in that**
R⁵, R⁶, R⁷ and R⁸, identical or different, represent -H; -NO₂; -CN; -N(R¹¹)-S(=O)₂-R¹²; -OR¹³; -SR¹⁴; -C(=O)-OR¹⁵; -NR¹⁶R¹⁷; -C(=O)-R¹⁸; -(C=O)-NR¹⁹R²⁰; -O-(C=O)-R²¹; -S(=O)₂-R²²; -S(=O)₂-NR²³R²⁴; -NR²⁵-C(=O)-NR²⁶R²⁷; -NR²⁸-(C=O)-R²⁹; -NR³⁰-(C=O)-OR³¹; -NR³²-S(=O)NR³³R³⁴; or-S(=O)₂-OR³⁵; F, Cl, Br, I; -CF₃, -CHF₂, -CH₂F, a linear or branched, saturated or unsaturated C₁₋₁₀ aliphatic radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R⁵, R⁶, R⁷ and R⁸, identical or different, represent -H; -NO₂; -CN; -N(R¹¹)-SO₂-R¹²; -OR¹³; -SR ¹⁴; -C(=O)-OR¹⁵; -NR¹⁶R¹⁷; -C(=O)-R¹⁸; -(C=O)-NR¹⁹R²⁰; -O-(C=O)-R²¹; -S(=O)₂-R²²; -S(=O)₂-NR²³R²⁴; -NR²⁵-C(=O)-NR²⁶R²⁷; -NR²⁸-(C=O)-R²⁹; -NR³⁰-(C=O)-OR³¹; -NR³²-S(=O)NR³³R³⁴; or -S(=O)₂-OR³⁵; F, Cl, Br, I; -CF₃, -CHF₂, -CH₂F, an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R⁵, R⁶, R⁷ and R⁸, identical or different, each represent -H; -NO₂; -CN; -N(R¹¹)-SO₂-R¹²; -OR¹³; -SR¹⁴; -C(=O)-OR¹⁵; -NR¹⁶R¹⁷; - F, Cl, Br, I; -CF₃, -CHF₂, -CH₂F, an alkyl radical selected from the group consisting of methyl, ethyl, n-propyl, iso-propyl, n-butyl, sec-butyl, iso-butyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
in each case with the proviso that at least one of the substituents R⁵, R⁶, R⁷ and R⁸ represents an -N(R¹¹)-S(=O)₂-R¹² moiety.

7. A compound according to one or more of claims 1-6, **characterized in that** one of the substituents R⁵, R⁶, R⁷ and R⁸ represents a -N(R¹¹)-S(=O)₂-R¹² moiety.

8. A compound according to claim 7, **characterized in that** one of the substituents R⁵, R⁶, R⁷ and R⁸ represents an -N(R¹¹)-S(=O)₂-R¹² moiety while the other three of these substituents each represent a hydrogen atom.

9. A compound according to one or more of claims 1-8, **characterized in that**
R⁹ and R¹⁰, independent from one another, represent a linear or branched, saturated or unsaturated C₁₋₁₀ aliphatic radical; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R⁹ and R¹⁰, independent from one another, represent an alkyl radical selected from the group consisting of methyl, ethyl, n-propyl, iso-propyl, n-butyl, sec-butyl, iso-butyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅₋alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl.

10. A compound according to one or more of claims 1-9, **characterized in that**
R¹¹ represents a hydrogen atom; a linear or branched, saturated or unsaturated, optionally at least mono-substituted C₁₋₁₀-aliphatic radical; a saturated or unsaturated, optionally at least mono-substituted, 3- to 9-membered cycloaliphatic radical, which may be bonded via a linear or branched C₁₋₆-alkylene, C₂₋₆-alkenylene or C₂₋₆-alkinylene group and/or which may contain 1, 2 or 3 heteroatoms independently selected from the group consisting of nitrogen, oxygen and sulphur as ring members; an optionally at least mono-substituted 5- to 10- membered aryl or heteroaryl radical, which may be bonded via a linear or branched C₁₋₆-alkylene, C₂-₆-alkenylene or C₂₋₆₋alkinylene group and wherein the heteroaryl radical contains 1, 2 or 3 heteroatoms independently selected from the group consisting of nitrogen, oxygen and sulphur as ring members; or an -S(=O)₂-R³⁶ moiety,
preferably R¹¹ represents a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or an -S(=O)₂-R³⁶-moiety,
more preferably R¹¹ represents a hydrogen atom.

11. A compound according to one or more of claims 1-10, **characterized in that** R¹² represents an optionally at least mono-substituted 5- to 10- membered aryl or heteroaryl radical, which may be bonded via a linear or branched, optionally at least mono-substituted C₁₋₆-alkylene, C₂₋₆-alkenylene or C₂₋₆-alkinylene group and/or which may be condensed with an optionally at least mono-substituted, saturated, unsaturated or aromatic, mono- or bicyclic ring system,
whereby the rings of the ring system are 5- 6- or 7-membered and whereby the heteroaryl radical and optionally one or both of the rings of the ring system contain 1, 2 or 3 heteroatoms independently selected from the group consisting of nitrogen, oxygen and sulphur,
or R¹² represents a saturated or unsaturated, optionally at least mono-substituted, 3- to 9-membered cycloaliphatic radical, which may be bonded via a linear or branched C₁₋₆-alkylene, C₂₋₆-alkenylene or C₂₋₆-alkinylene group and/or which may be condensed with an optionally at least mono-substituted mono- or polycyclic ring system,
whereby the rings of the ring system are 5- 6- or 7-membered and whereby the cycloaliphatic radical and optionally one or both of the rings of the ring system may contain 1, 2 or 3 heteroatoms independently selected from the group consisting of nitrogen, oxygen and sulphur,
preferably R¹² represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, 2-oxo-2,3-dihydro-benzooxazolyl, 2-oxo-2,3-dihydrobenzo[d]thiazolyl, benzooxazolinyl, benzothiazolinyl, benzimidazolidinyl, imidazo[2,1-b]thiazolyl, chromenyl and chromanyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R¹² represents an aryl or heteroaryl radical selected from the group consisting of phenyl, 1-naphthyl, 2-naphthyl, benzo[b]-thiophenyl, benzo[b]furanyl, quinolinyl, isoquinolinyl, imidazo[2,1-b]thiazolyl, 2-oxo-2,3-dihydro-benzooxazolyl and 2-oxo-2,3-dihydrobenzo[d]thiazolyl, whereby said aryl or heteroaryl radical may be substituted by 1, 2, 3, 4 or 5 substituents independently selected from the group consisting of methyl, ethyl, n-propyl, iso-propyl, n-butyl, sec-butyl, iso-butyl, tert-butyl, F, Cl, Br, I, -CN, -CF₃, -CF₂H, CFH₂, -C(=O)-O-CH₃, C(=O)-O-CH₂-CH₃, cyclopropyl, cyclobutyl, cyclopentyl, cyclohexyl, phenyl, phenoxy and benzyl.

12. A compound according to one or more of claims 1-11, **characterized in that** R¹³-R³⁵ independent from one another, each represent a hydrogen atom; a linear or branched C₁₋₁₀ alkyl radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members;
preferably R¹³-R³⁵ independent from one another, represent a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl- (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3-}group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl.

13. A compound according to one or more of claims 1-12, **characterized in that** R³⁶ represents an optionally at least mono-substituted 5- to 10- membered aryl or heteroaryl radical, which may be bonded via a linear or branched, optionally at least mono-substituted C₁₋₆-alkylene, C₂₋₆-alkenylene or C₂₋₆-alkinylene group and/or which may be condensed with an optionally at least mono-substituted, saturated, unsaturated or aromatic, mono- or bicyclic ring system,
whereby the rings of the ring system are 5- 6- or 7-membered and whereby the heteroaryl radical and optionally one or both of the rings of the ring system contain 1, 2 or 3 heteroatoms independently selected from the group consisting of nitrogen, oxygen and sulphur,
or R³⁶ represents a saturated or unsaturated, optionally at least mono-substituted, 3- to 9-membered cycloaliphatic radical, which may be bonded via a linear or branched C₁₋₆-alkylene, C₂₋₆-alkenylene or C₂₋₆-alkinylene group and/or which may be condensed with an optionally at least mono-substituted mono- or polycyclic ring system,
whereby the rings of the ring system are 5- 6- or 7-membered and whereby the cycloaliphatic radical and optionally one or both of the rings of the ring system may contain 1, 2 or 3 heteroatoms independently selected from the group consisting of nitrogen, oxygen and sulphur,
preferably R³⁶ represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, 2-oxo-2,3-dihydro-benzooxazolyl, 2-oxo-2,3-dihydrobenzo[d]thiazolyl, benzooxazolinyl, benzothiazolinyl, benzimidazolidinyl, imidazo[2,1-b]thiazolyl, chromenyl and chromanyl, whereby said aryl or heteroaryl radical may be bonded via a -(CH₂)₁, _{2 or 3}- group and/or substituted with 1, 2, 3, 4 or 5 substituents independently selected from the group consisting of C₁₋₅-alkyl, -O-C₁₋₅-alkyl, - S-C₁₋₅-alkyl, oxo (=O), -C(=O)-O-C₁₋₅-alkyl, -O-(C=O)-C₁₋₅-alkyl, F, Cl, Br, I, - CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, - S(=O)₂-C₁₋₅-alkyl, -S(=O)₂-phenyl, cyclopropyl, cyclobutyl, cyclopentyl, cyclohexyl, phenyl, phenoxy and benzyl; or a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting C₁₋₅-alkyl, -0-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl.

14. Process for the preparation of a compound according to one or more of claims 1 to 13, **characterized in that** at least one compound of general formula II, 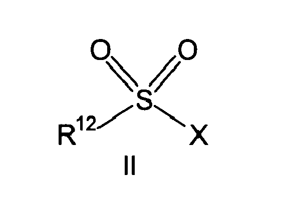 wherein R¹² has the meaning according to one or more of claims 1-13 and X represents a leaving group, preferably a halogen atom, particularly preferably a chlorine atom, is reacted with at least one compound of general formula III, 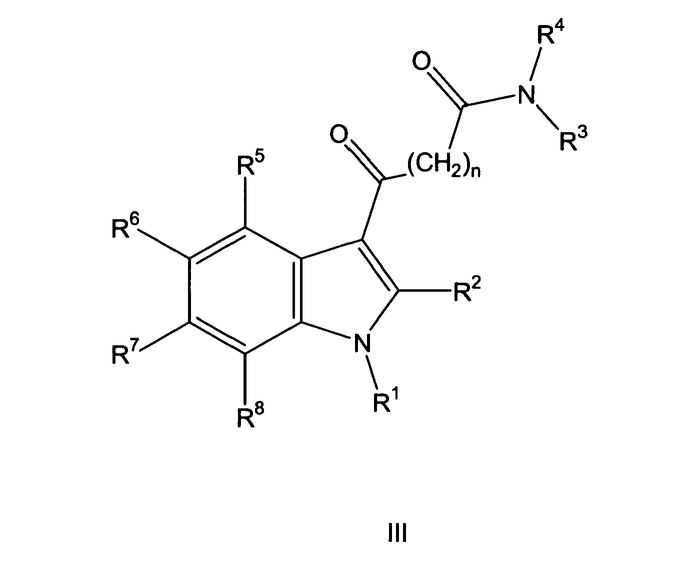 wherein R¹ to R⁴ and n have the meaning according to one or more of claims 1-13 and R⁵, R⁶, R⁷ and R⁸ have the meaning according to one or more of claims 1-13 with the proviso that at least one substituent of the group consisting of R⁵, R⁶, R⁷ and R⁸ is an -N(R¹¹)(H) moiety, in a suitable reaction medium, preferably in the presence of at least one base and/or at least one auxiliary agent.

15. Medicament comprising at least one substituted indole compound of general formula Ia, 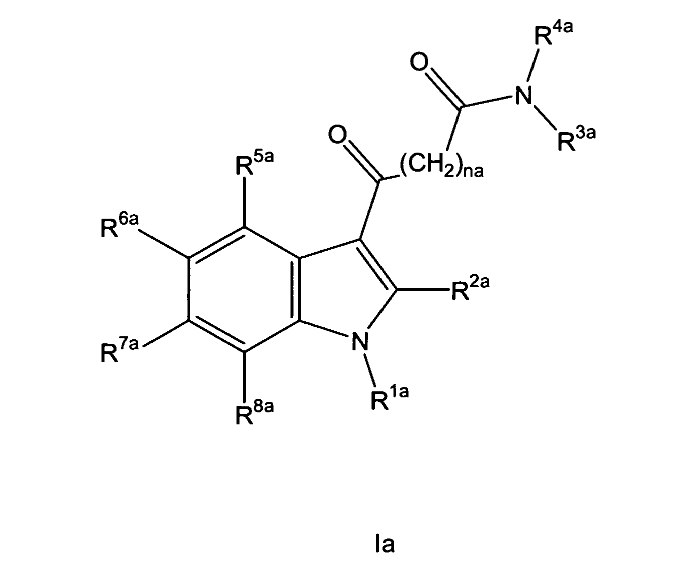 wherein
na represents 0,1, 2, 3 or 4,
for na = 0: R^{1a} represents a hydrogen atom; a saturated or unsaturated, optionally at least mono-substituted, optionally at least one heteroatom as a ring member containing cycloaliphatic radical; an optionally at least mono-substituted aryl or heteroaryl radical; -S(=O)₂-R^{9a}; or -C(=O)-R^{10a},
for na = 1, 2, 3 or 4: R^{1a} represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; an optionally at least mono-substituted aryl or heteroaryl radical, which may be bonded via a linear or branched alkylene group; -S(=O)₂-R^{9a}; or -C(=O)-R^{10a},
R^{2a} represents -H, -NO₂; -NH₂; -SH; -OH; -CN; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R^{3a} and R^{4a}, identical or different, represent a hydrogen atom; a linear or branched, saturated or unsaturated aliphatic radical, an optionally at least mono-substituted aryl or heteroaryl radical, which may be bonded via a linear or branched alkylene group; a saturated or unsaturated, optionally at least mono-substituted, optionally at least one heteroatom as a ring member containing cycloaliphatic radical, which may be bonded via a linear or branched alkylene group and/or which may be condensed with an optionally at least mono-substituted mono- or polycyclic ring system, or
R^{3a} and R^{4a} together with the bridging nitrogen form an optionally at least mono-substituted, saturated, unsaturated or aromatic heterocyclic ring that may contain at least one further heteroatom as a ring member and/or that may be condensed with an optionally at least mono-substituted mono- or polycyclic ring-system,
R^{5a}, R^{6a}, R^{7a} and R^{8a}, identical or different, represent -H; -NO₂; -CN; -N(R^{11a})-S(=O)₂-R^{12a}; -OR^{13a}; -SR^{14a}; -C(=O)-OR^{15a}; -NR^{16a}R^{17a}; -C(=O)-R^{18a}; -(C=O)-NR^{19a}R^{20a}; -O-(C=O)-R^{21a}; -S(=O)₂-R^{22a}; -S(=O)₂-NR^{23a}R^{24a}; -NR^{25a-}C(=O)-NR^{26a}R^{27a}; -NR^{28a}-(C=O)-R^{29a}; -NR^{30a}-(C=O)-OR^{31a}; -NR^{32a-}S(=O)NR^{33a}R^{34a}; or -S(=O)₂-OR^{35a}; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
with the proviso that at least one of the substituents R^{5a}, R^{6a}, R^{7a} and R^{8a} represents an -N(R^{11a})-S(=O)₂-R^{12a} moiety,
R^{9a} and R^{10a}, independent from one another, represent a linear or branched, saturated or unsaturated, optionally at least mono-substituted aliphatic radical; or an optionally at least mono-substituted aryl or heteroaryl radical, which may be bonded via a linear or branched alkylene group,
R^{11a} represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group; an optionally at least mono-substituted aryl or heteroaryl radical, which may be bonded via a linear or branched, optionally at least mono-substituted alkylene, alkenylene or alkinylene group; or an - S(=O)₂-R^{36a} moiety,
R^{12a} represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system; or a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system,
R^{13a}-R^{35a}, independent from one another, represent a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R^{36a} represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system; or a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system,
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

16. Medicament according to claim 15, **characterized in that**
for na = 0: R^{1a} represents a hydrogen atom; a saturated or unsaturated, optionally at least mono-substituted, optionally at least one heteroatom as a ring member containing 3- to 9-membered cycloaliphatic radical; an optionally at least mono-substituted 5- to 14- membered aryl or heteroaryl radical; -S(=O)₂-R^{9a}; or -C(=O)-R^{10a},
for na = 1, 2, 3 or 4: R^{1a} represents a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆-alkylene group; an optionally at least mono-substituted 5- to 14-membered aryl or heteroaryl radical, which may be bonded via a linear or branched C₁₋₆-alkylene group; -S(=O)₂-R^{9a}; or -C(=O)-R^{10a},
R^{2a} represents -H, -NO₂; -NH₂; -SH; -OH; -CN; a halogen atom; a linear or branched, saturated or unsaturated, optionally at least mono-substituted, optionally at least one heteroatom as a chain member containing C₁₋₁₀ aliphatic radical; a saturated or unsaturated, optionally at least mono-substituted, optionally at least one heteroatom as a ring member containing 3-to 9-membered cycloaliphatic radical, which may be bonded via a linear or branched C₁₋₆-alkylene group; or an optionally at least mono-substituted 5- to 14-membered aryl or heteroaryl radical, which may be bonded via a linear or branched C₁₋₆-alkylene group,
R^{3a} and R^{4a}, identical or different, represent a hydrogen atom; a linear or branched, saturated or unsaturated C₁₋₁₀ aliphatic radical; an optionally at least mono-substituted 5- to 14-membered aryl or heteroaryl radical, which may be bonded via a linear or branched C₁₋₆ alkylene group; a saturated or unsaturated, optionally at least mono-substituted, optionally at least one heteroatom as a ring member containg 3- to 9-membered cycloaliphatic radical, which may be bonded via a linear or branched C₁₋₆ alkylene group and/or which may be condensed with an optionally at least mono-substituted mono- or polycyclic ring system, or
R^{3a} and R^{4a} together with the bridging nitrogen form an optionally at least mono-substituted, saturated, unsaturated or aromatic 4- to 9-membered heterocyclic ring that may contain at least one further heteroatom as a ring member and/or that may be condensed with an optionally at least mono-substituted mono- or polycyclic ring-system,
R^{5a}, R^{6a}, R^{7a} and R^{8a}, identical or different, represent -H; -NO₂; -CN; -N(R^{11a})-S(=O)₂-R^{12a}; -OR^{13a}; -SR^{14a}; -C(=O)-OR^{15a}; -NR^{16a}R^{17a}; -C(=O)-R^{18a}; (C=O)-NR^{19a}R^{20a}; -O-(C-O)-R^{21a}; -S(=O)₂-R^{22a}; -S(=O)₂-NR^{23a}R^{24a}; -NR^{25a-}C(=O)-NR^{26a}R^{27a}; -NR^{28a}-(C=O)-R^{29a}; -NR^{30a}-(C=O)-OR^{31a}; -NR^{32a-}S(=O)NR^{33a}R^{34a}; or -S(=O)₂-OR^{35a}; a halogen atom; a linear or branched, saturated or unsaturated, optionally at least mono-substituted, optionally at least one heteroatom as a chain member containing C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆₋alkylene group,
with the proviso that at least one of the substituents R^{5a}, R^{6a}, R^{7a} and R^{8a} represents an -N(R^{11a})-S(=O)₂-R^{12a} moiety,
R^{9a} and R^{10a}, independent from one another, represent a linear or branched, saturated or unsaturated, optionally at least mono-substituted C₁₋₁₀ aliphatic radical; or an optionally at least mono-substituted, 5- to 14-membered aryl or heteroaryl radical, which may be bonded via a linear or branched C₁₋₆-alkylene group,
R^{11a} represents a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group; an optionally at least mono-substituted 5- to 14 membered aryl or heteroaryl radical, which may be bonded via a linear or branched, optionally at least mono-substituted C₁₋₆ alkylene, C₂₋₆ alkenylene or C₂₋₆ alkinylene group; or an -S(=O)₂-R^{36a} moiety, R^{12a} represents an optionally at least mono-substituted 5- to 14-membered aryl or heteroaryl radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system; or a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono-or polycyclic ring system,
R^{13a}-R^{35a}, independent from one another, represent a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3-to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆ alkylene group,
R^{36a} represents an optionally at least mono-substituted 5- to 14-membered aryl or heteroaryl radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system; or a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono-or polycyclic ring system.

17. Medicament according to claim 15 or 16, **characterized in that**
for na = 0
R^{1a} represents a hydrogen atom; a saturated or unsaturated, optionally at least mono-substituted, 3- to 9-membered cycloaliphatic radical, which may contain 1, 2 or 3 heteroatoms independently selected from the group consisting of nitrogen, oxygen and sulphur as ring members; an optionally at least mono-substituted 5- to 10- membered aryl or heteroaryl radical, wherein the heteroaryl radical contains 1, 2 or 3 heteroatoms independently selected from the group consisting of nitrogen, oxygen and sulphur as ring members; -S(=O)₂-R^{9a}; or -C(=O)-R^{10a},
preferably R^{1a} represents a hydrogen atom;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be substituted with 1, 2, 3, 4 or 5 substituents independently selected from the group consisting of C₁₋₅-alkyl, -0-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -CON(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be substituted with 1, 2, 3, 4 or 5 substituents independently selected from the group consisting of C₁₋₅-alkyl, -0-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; -S(=O)₂-R^{9a}; or -C(=O)-R^{10a},
more preferably R^{1a} represents a hydrogen atom,
for na=1,2,3or4
R^{1a} represents a hydrogen atom; a linear or branched C₁₋₁₀ alkyl radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; an optionally at least mono-substituted 5- to 10-membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members; -S(=O)₂-R^{9a}; or -C(=O)-R^{10a},
preferably R^{1a} represents a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3-}group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; -S(=O)₂-R^{9a}; or -C(=O)-R^{10a}.

18. Medicament according to one or more of claims 15-17, **characterized in that**
R^{2a} represents -H, -NO₂; -NH₂; -SH; -OH; -CN; -CF₃; -OCH₃; -O-CH₂-CH₃; F; Cl; Br; I; a linear or branched, saturated or unsaturated C₁₋₁₀ aliphatic radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10-membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R^{2a} represents -H, -NO₂; -NH₂; -SH; -OH; -CN; -CF₃; -OCH₃; -O-CH₂-CH₃; F; Cl; Br; I; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R^{2a} represents a hydrogen atom.

19. Medicament according to one or more of claims 15-18, **characterized in that**
R^{3a} and R^{4a}, identical or different, represent a hydrogen atom or a linear or branched C₁₋₈ alkyl radical, or
R^{3a} and R^{4a} together with the bridging nitrogen form an optionally at least mono-substituted, saturated, unsaturated or aromatic 4- to 9-membered heterocyclic ring that may be condensed with an optionally at least mono-substituted mono- or bicyclic ring-system,
whereby the rings of the ring system are 5- 6- or 7-membered and whereby the heterocyclic ring and one or both of the rings of the ring system may contain 1, 2 or 3 heteroatoms independently selected from the group consisting of nitrogen, oxygen and sulphur,
preferably
R^{3a} and R^{4a}, identical or different, represent a hydrogen atom or an alkyl radical selected from the group consisting of methyl, ethyl, n-propyl, iso-propyl, n-butyl, sec-butyl, iso-butyl and tert-butyl; or
R^{3a} and R^{4a} together with the bridging nitrogen atom form a moiety selected from the group consisting of 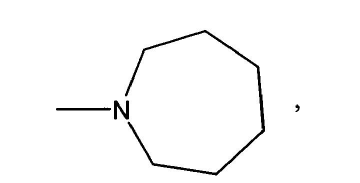 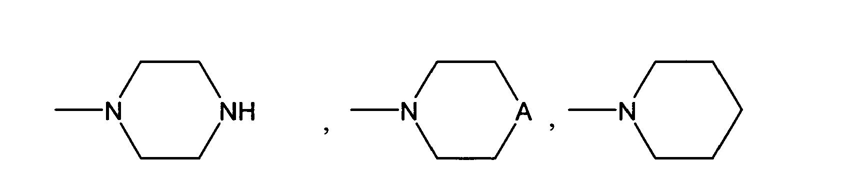 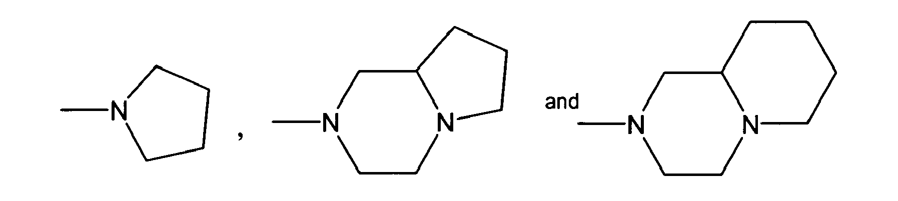 whereby A represents an oxygen atom or a sulphur atom and whereby each of these afore mentioned cyclic moieties may be substituted with 1, 2 or 3 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl in any position including the nitrogen atoms of the piperazine ring,
more preferably R^{3a} and R^{4a}, identical or different, represent a hydrogen atom or an alkyl radical selected from the group consisting of methyl, ethyl, n-propyl, iso-propyl, n-butyl, sec-butyl, iso-butyl and tert-butyl.

20. Medicament according to one or more of claims 15-19, **characterized in that**
R^{5a}, R^{6a}, R^{7a} and R^{8a} identical or different, represent -H; -NO₂; -CN; -N(R^{11a})-S(=O)₂-R^{12a}; -OR^{13a}; -SR^{14a}; -C(=O)-OR^{15a}; -NR^{16a}R^{17a}; -C(=O)-R^{18a}; -(C=O)-NR^{19a}R^{20a}; -O-(C=O)-R^{21a}; -S(=O)₂-R^{22a}; -S(=O)₂-NR^{23a}R^{24a}; -NR^{25a-}C(=O)-NR^{26a}R^{27a}; -NR^{28a}-(C=O)-R^{29a}; -NR^{30a}-(C=O)-OR^{31a}; -NR^{32a-}S(=O)NR^{33a}R^{34a}; or -S(=O)₂-OR^{35a}; F, Cl, Br, I; -CF₃, -CHF₂, -CH₂F, a linear or branched, saturated or unsaturated C₁₋₁₀ aliphatic radical; a saturated or unsaturated, optionally at least mono-substituted, 3- to 9-membered cycloaliphatic radical, which may be bonded via a linear or branched C₁₋₆₋alkylene group and/or which may contain 1, 2 or 3 heteroatoms independently selected from the group consisting of nitrogen, oxygen and sulphur as ring members; or an optionally at least mono-substituted 5- to 10- membered aryl or heteroaryl radical, which may be bonded via a linear or branched C₁₋₆₋alkylene group and wherein the heteroaryl radical contains 1, 2 or 3 heteroatoms independently selected from the group consisting of nitrogen, oxygen and sulphur as ring members,
preferably R^{5a}, R^{6a}, R^{7a} and R^{8a}, identical or different, represent -H; -NO₂; -CN; -N(R^{11a})-S(=O)₂-R^{12a}; -OR^{13a}; -SR^{14a}; -C(=O)-OR^{15a}; -NR^{16a}R^{17a}; -C(=O)-R^{18a}; -(C=O)-NR^{19a}R^{20a}; -O-(C=O)-R^{21a}; -S(=O)₂-R^{22a}; -S(=O)₂-NR^{23a}R^{24a}; -NR^{25a}-C(=O)-NR^{26a}R^{27a}; -NR^{28a}-(C=O)-R^{29a}; -NR^{30a}-(C=O)-OR^{31a}; -NR^{32a-}S(=O)NR^{33a}R^{34a}; or -S(=O)₂-OR^{35a}; F, Cl, Br, I; -CF₃, -CHF₂, -CH₂F, an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R^{5a}, R^{6a}, R^{7a} and R^{8a}, identical or different, each represent -H; -NO₂; -CN; -N(R^{11a})-S(=O)₂-R^{12a}; -OR^{13a}; -SR^{14a}; -C(=O)-OR^{15a}; -NR^{16a}R^{17a}; -F, Cl, Br, I; -CF₃, -CHF₂, -CH₂F, an alkyl radical selected from the group consisting of methyl, ethyl, n-propyl, iso-propyl, n-butyl, sec-butyl, iso-butyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3-}group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -CO-N(C₁₋₅-alkyl)₂, -S(=O)₂-C₁₋₅-alkyl, - S(=O)₂-phenyl, cyclopropyl, cyclobutyl, cyclopentyl, cyclohexyl, phenyl, phenoxy and benzyl,
in each case with the proviso that at least one of the substituents R^{5a}, R^{6a}, R^{7a} and R^{8a} represents an -N(R^{11a})-S(=O)₂-R^{12a} moiety.

21. Medicament according to one or more of claims 15-20, **characterized in that** one of the substituents R^{5a}, R^{6a}, R^{7a} and R^{8a} represents an -N(R^{11a})-S(=O)₂₋R^{12a} moiety.

22. Medicament according to claim 21 **characterized in that** one of the substituents R^{5a}, R^{6a}, R^{7a} and R^{8a} represents an -N(R^{11a})-S(=O)₂-R^{12a} moiety while the other three of these substituents each represent a hydrogen atom.

23. Medicament according to one or more of claims 15-22, **characterized in that**
R^{9a} and R^{10a}, independent from one another, represent a linear or branched, saturated or unsaturated C₁₋₁₀ aliphatic radical; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R^{9a} and R^{10a}, independent from one another, represent an alkyl radical selected from the group consisting of methyl, ethyl, n-propyl, iso-propyl, n-butyl, sec-butyl, iso-butyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅₋alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl.

24. Medicament according to one or more of claims 15-23, **characterized in that**
R^{11a} represents a hydrogen atom; a linear or branched, saturated or unsaturated, optionally at least mono-substituted C₁₋₁₀-aliphatic radical; a saturated or unsaturated, optionally at least mono-substituted, 3- to 9-membered cycloaliphatic radical, which may be bonded via a linear or branched C₁₋₆-alkylene, C₂₋₆-alkenylene or C₂₋₆-alkinylene group and/or which may contain 1, 2 or 3 heteroatoms independently selected from the group consisting of nitrogen, oxygen and sulphur as ring members; an optionally at least mono-substituted 5- to 10- membered aryl or heteroaryl radical, which may be bonded via a linear or branched C₁₋₆-alkylene, C₂₋₆-alkenylene or C₂₋₆₋alkinylene group and wherein the heteroaryl radical contains 1, 2 or 3 heteroatoms independently selected from the group consisting of nitrogen, oxygen and sulphur as ring members; or an -S(=O)₂-R^{36a} moiety,
preferably R^{11a} represents a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or an -S(=O)₂-R^{36a}-moiety,
more preferably R^{11a} represents a hydrogen atom.

25. Medicament according to one or more of claims 15-24, **characterized in that** R^{12a} represents an optionally at least mono-substituted 5- to 10- membered aryl or heteroaryl radical, which may be bonded via a linear or branched, optionally at least mono-substituted C₁₋₆-alkylene, C₂₋₆-alkenylene or C₂₋₆₋alkinylene group and/or which may be condensed with an optionally at least mono-substituted, saturated, unsaturated or aromatic, mono- or bicyclic ring system,
whereby the rings of the ring system are 5- 6- or 7-membered and whereby the heteroaryl radical and optionally one or both of the rings of the ring system contain 1, 2 or 3 heteroatoms independently selected from the group consisting of nitrogen, oxygen and sulphur,
or R^{12a} represents a saturated or unsaturated, optionally at least mono-substituted, 3- to 9-membered cycloaliphatic radical, which may be bonded via a linear or branched C₁₋₆-alkylene, C₂₋₆-alkenylene or C₂₋₆-alkinylene group and/or which may be condensed with an optionally at least mono-substituted mono- or polycyclic ring system,
whereby the rings of the ring system are 5- 6- or 7-membered and whereby the cycloaliphatic radical and optionally one or both of the rings of the ring system may contain 1, 2 or 3 heteroatoms independently selected from the group consisting of nitrogen, oxygen and sulphur,
preferably R^{12a} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, 2-oxo-2,3-dihydro-benzooxazolyl, 2-oxo-2,3-dihydrobenzo[d]thiazolyl, benzooxazolinyl, benzothiazolinyl, benzimidazolidinyl, imidazo[2,1-b]thiazolyl, chromenyl and chromanyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R^{12a} represents an aryl or heteroaryl radical selected from the group consisting of phenyl, 1-naphthyl, 2-naphthyl, benzo[b]-thiophenyl, benzo[b]furanyl, quinolinyl, isoquinolinyl, imidazo[2,1-b]thiazolyl, 2-oxo-2,3-dihydro-benzooxazolyl and 2-oxo-2,3-dihydrobenzo[d]thiazolyl, whereby said aryl or heteroaryl radical may be substituted by 1, 2, 3, 4 or 5 substituents independently selected from the group consisting of methyl, ethyl, n-propyl, iso-propyl, n-butyl, sec-butyl, iso-butyl, tert-butyl, F, Cl, Br, I, -CN, -CF₃, -CF₂H, CFH₂, -C(=O)-O-CH₃, C(=O)-O-CH₂-CH₃, cyclopropyl, cyclobutyl, cyclopentyl, cyclohexyl, phenyl, phenoxy and benzyl.

26. Medicament according to one or more of claims 15-25, **characterized in that** R^{13a}-R^{35a} independent from one another, each represent a hydrogen atom; a linear or branched C₁₋₁₀ alkyl radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members;
preferably R^{13a}-R^{35a} independent from one another, represent a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl- (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)₁, _{2 or 3-}group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl.

27. Medicament according to one or more of claims 15-26, **characterized in that** R^{36a} represents an optionally at least mono-substituted 5- to 10- membered aryl or heteroaryl radical, which may be bonded via a linear or branched, optionally at least mono-substituted C₁₋₆-alkylene, C₂₋₆-alkenylene or C₂₋₆₋alkinylene group and/or which may be condensed with an optionally at least mono-substituted, saturated, unsaturated or aromatic, mono- or bicyclic ring system, whereby the rings of the ring system are 5- 6- or 7-membered and whereby the heteroaryl radical and optionally one or both of the rings of the ring system contain 1, 2 or 3 heteroatoms independently selected from the group consisting of nitrogen, oxygen and sulphur,
or R^{36a} represents a saturated or unsaturated, optionally at least mono-substituted, 3- to 9-membered cycloaliphatic radical, which may be bonded via a linear or branched C₁₋₆-alkylene, C₂₋₆-alkenylene or C₂₋₆-alkinylene group and/or which may be condensed with an optionally at least mono-substituted mono- or polycyclic ring system,
whereby the rings of the ring system are 5- 6- or 7-membered and whereby the cycloaliphatic radical and optionally one or both of the rings of the ring system may contain 1, 2 or 3 heteroatoms independently selected from the group consisting of nitrogen, oxygen and sulphur,
preferably R^{36a} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, 2-oxo-2,3-dihydro-benzooxazolyl, 2-oxo-2,3-dihydrobenzo[d]thiazolyl, benzooxazolinyl, benzothiazolinyl, benzimidazolidinyl, imidazo[2,1-b]thiazolyl, chromenyl and chromanyl, whereby said aryl or heteroaryl radical may be bonded via a -(CH₂)₁, _{2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl.

28. Medicament according to one or more of claims 15-27, **characterized in that** na is 0.

29. Medicament according to one or more of claims 15-28, **characterized in that**
na is 0,
R^{1a} represents a hydrogen atom,
R^{2a} represents a hydrogen atom,
R^{3a} and R^{4a}, identical or different, represent an alkyl radical selected from the group consisting of methyl, ethyl, n-propyl, iso-propyl, n-butyl, sec-butyl, iso-butyl and tert-butyl,
one of the substituents R^{5a}, R^{6a}, R^{7a} and R^{8a} represents an -N(R^{11a})-S(=O)-R^{12a}-moiety while the other three of these substituents each represent a hydrogen atom,
R^{11a} represents a hydrogen atom,
R^{12a} represents an aryl or heteroaryl radical selected from the group consisting of phenyl, 1-naphthyl, 2-naphthyl, benzo[b]-thiophenyl, benzo[b]furanyl, quinolinyl, isoquinolinyl, imidazo[2,1-b]thiazolyl, 2-oxo-2,3-dihydro-benzooxazolyl and 2-oxo-2,3-dihydrobenzo[d]thiazolyl, whereby said aryl or heteroaryl radical may be substituted by 1, 2, 3, 4 or 5 substituents independently selected from the group consisting of methyl, ethyl, n-propyl, iso-propyl, n-butyl, sec-butyl, iso-butyl, tert-butyl, F, Cl, Br, I, -CN, -CF₃, -CF₂H, CFH₂, -C(=O)-O-CH₃, C(=O)-O-CH₂-CH₃, cyclopropyl, cyclobutyl, cyclopentyl, cyclohexyl, phenyl, phenoxy and benzyl.

30. Medicament according to one or more of claims 15-29, **characterized in that** the substituted indole compound is selected from the group consisting of
[1] 2-[5-(5-Chloro-3-methyl-benzo[b]thiophene-2-sulfonylamino)-1H-indol-3-yl]-N,N-diethyl-2-oxoacetamide,
[2] N,N-Diethyl-2-[5-(naphthalene-2-sulfonylamino)-1H-indol-3-yl]-2-oxo-acetamide,
[3] N,N-Diethyl-2-[5-(naphthalene-1-sulfonylamino)-1H-indol-3-yl]-2-oxo-acetamide,
[4] 2-[5-(Biphenyl-4-sulfonylamino)-1H-indol-3-yl]-N,N-diethyl-2-oxo-acetamide,
[5] N,N-Diethyl-2-oxo-2-[5-(quinoline-8-sulfonylamino)-1H-indol-3-yl]-acetamide,
[6] N,N-Dimethyl-2-[5-(naphthalene-2-sulfonylamino)-1H-indol-3-yl]-2-oxo-acetamide,
[7] N,N-Dimethyl-2-[5-(naphthalene-1-sulfonylamino)-1H-indol-3-yl]-2-oxo-acetamide,
[8] 2-[5-(5-Chloro-3-methyl-benzo[b]thiophene-2-sulfonylamino)-1 H-indol-3-yl]-N,N-dimethyl-2-oxo-acetamide,
[9] 2-[5-(6-Chloro-imidazo[2,1-b]thiazole-5-sulfonylamino)-1 H-indol-3-yl]-N, N-diethyl-2-oxo-acetamide,
[10] 2-[5-(6-Chloro-imidazo[2,1-b]thiazole-5-sulfonylamino)-1 H-indol-3-yl]-N,N-dimethyl-2-oxo-acetamide,
[11] N,N-Dimethyl-2-[4-(naphthalene-1-sulfonylamino)-1H-indol-3-yl]-2-oxo-acetamide,
[12] 2-[4-(5-Chloro-3-methyl-benzo[b]thiophene-2-sulfonylamino)-1 H-indole-3-yl]-N,N-dimethyl-2-oxo-acetamide,
[13] 2-[4-(6-Chloro-imidazo[2,1-b]thiazole-5-sulfonylamino)-1H-indol-3-yl]-N, N-dimethyl-2-oxo-acetamide,
[14] N,N-Dimethyl-2-[5-[(4-fluoro-3-methyl-phenyl)-1-sulfonylamino]-1H-indol-3-yl]-2-oxo-acetamide,
[15] 5-(3-Dimethylaminooxalyl-1H-indol-5-ylsulfamoyl)-3-methyl-benzofuran-2-carboxylic acid ethyl ester,
[16] 2-[5-(Biphenyl-4-sulfonylamino)-1H-indol-3-yl]-N,N-dimethyl-2-oxo-acetamide,
[17] N,N-Dimethyl-2-oxo-2-[5-(2-oxo-2,3-dihydro-benzoxazole-6-sulfonylamino)-1 H-indol-3-yl]-acetamide,
[18] N,N-Dimethyl-2-oxo-2-[5-(2-oxo-2,3-dihydrobenzo[d]thiazole-6-sulfonamido)-1H-indol-3-yl]acetamide,
[19] 2-[5-[(4-Cyclohexyl-phenyl)-1-sulfonylamino]-1H-indol-3-yl]-N,N-dimethyl-2-oxo-acetamide and
[20] N,N-Dimethyl-2-[5-[(4-phenoxy-phenyl)-1-sulfonylamino]-1H-indol-3-yl]-2-oxo-acetamide.

31. Medicament according to one or more of claims 15-30 for the prophylaxis and/or treatment of a disorder or a disease that is least partially mediated via 5-HT₆-receptors.

32. Medicament according to one or more of claims 15-31 for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

33. Medicament according to one or more of claims 15-31 for the prophylaxis and/or treatment of irritable colon syndrome; disorders of the central nervous system; anxiety; panic attacks; depression; bipolar disorders; cognitive disorders; memory disorders; senile dementia; psychosis; neurodegenerative disorders, preferably selected from the group consisting of Morbus Alzheimer, Morbus Parkinson, Morbus Huntington and Multiple Sclerosis; schizophrenia; psychosis; hyperactivity disorder (ADHD, attention deficit/hyperactivity disorder) or for improvement of cognition (cognitive enhancement).

34. Use of at least one substituted indole compound of general formula Ib, 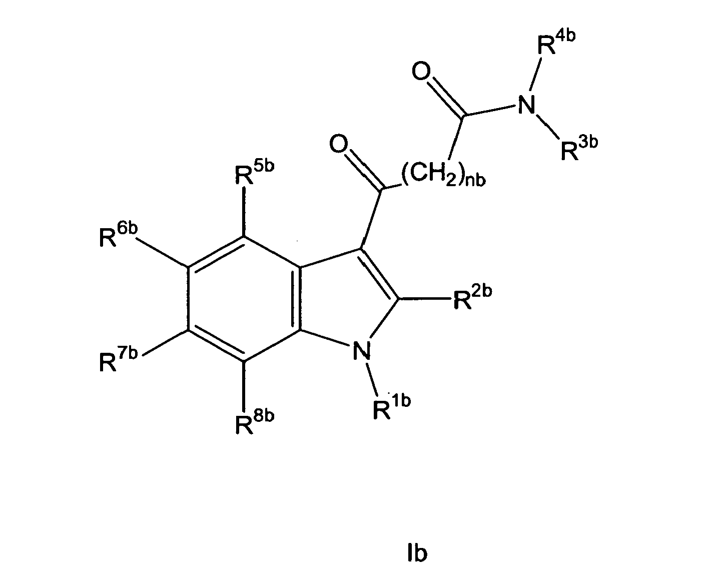 wherein
nb represents 0, 1, 2, 3 or 4,
R^{1b} represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; an optionally at least mono-substituted aryl or heteroaryl radical, which may be bonded via a linear or branched alkylene group; -S(=O)₂-R^{9b}; or -C(=O)-R^{10b},
R^{2b} represents -H, -NO₂; -NH₂; -SH; -OH; -CN; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R^{3b} and R^{4b}, identical or different, represent a hydrogen atom; a linear or branched, saturated or unsaturated aliphatic radical, an optionally at least mono-substituted aryl or heteroaryl radical, which may be bonded via a linear or branched alkylene group; a saturated or unsaturated, optionally at least mono-substituted, optionally at least one heteroatom as a ring member containing cycloaliphatic radical, which may be bonded via a linear or branched alkylene group and/or which may be condensed with an optionally at least mono-substituted mono- or polycyclic ring system, or
R^{3b} and R^{4b} together with the bridging nitrogen form an optionally at least mono-substituted, saturated, unsaturated or aromatic heterocyclic ring that may contain at least one further heteroatom as a ring member and/or that may be condensed with an optionally at least mono-substituted mono- or polycyclic ring-system,
R^{5b}, R^{6b}, R^{7b} and R^{8b}, identical or different, represent -H; -NO₂; -CN; -N(R^{11b})-S(=O)₂-R^{12b}; -OR^{13b}; -SR^{14b}; -C(=O)-OR^{15b}; -NR^{16b}R^{17b}; -C(=O)-R^{18b}; -(C=O)-NR^{19b}R^{20b}; -O-(C=O)-R^{21b}; -S(=O)₂-R^{22b}; -S(=O)₂-NR^{23b}R^{24b}; -NR^{25b-}C(=O)-NR^{26b}R^{27b}; -NR^{28b}-(C=O)-R^{29b}; -NR^{30b}-(C=O)-OR^{31b}; -NR^{32b} -S(=O)NR^{33b}R^{34b}; or -S(=O)₂-OR^{35b}; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
with the proviso that at least one of the substituents R^{5b,} R^{6b}, R^{7b} and R^{8b} represents an -N(R^{11b})-S(=O)₂-R^{12b} moiety,
R^{9b} and R^{10b}, independent from one another, represent a linear or branched, saturated or unsaturated, optionally at least mono-substituted aliphatic radical; or an optionally at least mono-substituted aryl or heteroaryl radical, which may be bonded via a linear or branched alkylene group,
R^{11b} represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group; an optionally at least mono-substituted aryl or heteroaryl radical, which may be bonded via a linear or branched, optionally at least mono-substituted alkylene, alkenylene or alkinylene group; or an -S(=O)₂-R^{36b} moiety,
R^{12b} represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system; or a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system,
R^{13b}-R^{35b}, independent from one another, represent a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R^{36b} represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system; or a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system,
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof,
for the manufacture of a medicament for the prophylaxis and/or treatment of a disorder or disease that is at least partially mediated via 5-HT₆ receptors.

35. Use according to claim 34, **characterized in that**
R^{1b} represents a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆₋alkylene group; an optionally at least mono-substituted 5- to 14- membered aryl or heteroaryl radical, which may be bonded via a linear or branched C₁₋₆₋alkylene group; -S(=O)₂-R^{9b}; or -C(=O)-R^{10b},
R^{2b} represents -H; -NO₂; -NH₂; -SH; -OH; -CN; a halogen atom; a linear or branched, saturated or unsaturated, optionally at least mono-substituted, optionally at least one heteroatom as a chain member containing C₁₋₁₀ aliphatic radical; a saturated or unsaturated, optionally at least mono-substituted, optionally at least one heteroatom as a ring member containing 3-to 9-membered cycloaliphatic radical, which may be bonded via a linear or branched C₁₋₆-alkylene group; or an optionally at least mono-substituted 5- to 14-membered aryl or heteroaryl radical, which may be bonded via a linear or branched C₁₋₆-alkylene group,
R^{3b} and R^{4b}, identical or different, represent a hydrogen atom; a linear or branched, saturated or unsaturated C₁₋₁₀ aliphatic radical; an optionally at least mono-substituted 5- to 14-membered aryl or heteroaryl radical, which may be bonded via a linear or branched C₁₋₆ alkylene group; a saturated or unsaturated, optionally at least mono-substituted, optionally at least one heteroatom as a ring member containg 3- to 9-membered cycloaliphatic radical, which may be bonded via a linear or branched C₁₋₆ alkylene group and/or which may be condensed with an optionally at least mono-substituted mono- or polycyclic ring system, or
R^{3b} and R^{4b} together with the bridging nitrogen form an optionally at least mono-substituted, saturated, unsaturated or aromatic 4- to 9-membered heterocyclic ring that may contain at least one further heteroatom as a ring member and/or that may be condensed with an optionally at least mono-substituted mono- or polycyclic ring-system,
R^{5b}, R^{6b}, R^{7b} and R^{8b}, identical or different, represent -H; -NO₂; -CN; -N(R^{11b})-S(=O)₂-R^{12b}; -OR^{13b}; -SR^{14b}; -C(=O)-OR^{15b}; -NR^{16b}R^{17b}; -C(=O)-R^{18b}; -(C=O)-NR^{19b}R^{20b}; -O-(C=O)-R^{21b}; -S(=O)₂-R^{22b}; -S(=O)₂-NR^{23b}R^{24b}; -NR^{25b-}C(=O)-NR^{26b}R^{27b}; -NR^{28b}-(C=O)-R^{29b}; -NR^{30b}-(C=O)-OR^{31b}; -NR^{32b-}S(=O)NR^{33b}R^{34b}; or -S(=O)₂-OR^{35b}; a halogen atom; a linear or branched, saturated or unsaturated, optionally at least mono-substituted, optionally at least one heteroatom as a chain member containing C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆₋alkylene group,
with the proviso that at least one of the substituents R^{5b}, R^{6b}, R^{7b} and R^{8b} represents an -N(R^{11b})-S(=O)₂-R^{12b} moiety,
R^{9b} and R^{10b}, independent from one another, represent a linear or branched, saturated or unsaturated, optionally at least mono-substituted C₁₋₁₀ aliphatic radical; or an optionally at least mono-substituted, 5- to 14-membered aryl or heteroaryl radical, which may be bonded via a linear or branched C₁₋₆-alkylene group,
R^{11b} represents a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group; an optionally at least mono-substituted 5- to 14 membered aryl or heteroaryl radical, which may be bonded via a linear or branched, optionally at least mono-substituted C₁₋₆ alkylene, C₂₋₆ alkenylene or C₂₋₆ alkinylene group; or an -S(=O)₂-R^{36b} moiety,
R^{12b} represents an optionally at least mono-substituted 5- to 14-membered aryl or heteroaryl radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system; or a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono-or polycyclic ring system,
R^{13b}-R^{35b}, independent from one another, represent a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3-to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆ alkylene group,
R^{36b} represents an optionally at least mono-substituted 5- to 14-membered aryl or heteroaryl radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system; or a saturated or unsaturated, optionally at least mono-substituted, optionally at least one heteroatom as a ring member containing 3- to 9-membered cycloaliphatic radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono-or polycyclic ring system.

36. Use according to claim 34 or 35, **characterized in that** R^{1b} represents a hydrogen atom; a linear or branched C₁₋₁₀ alkyl radical; a saturated or unsaturated, optionally at least mono-substituted, 3- to 9-membered cycloaliphatic radical, which may be bonded via a linear or branched C₁₋₆₋alkylene group and/or which may contain 1, 2 or 3 heteroatoms independently selected from the group consisting of nitrogen, oxygen and sulphur as ring members; an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members; -S(=O)₂-R^{9b}; or -C(=O)-R^{10b},
preferably R^{1b} represents a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3-}group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; -S(=O)₂-R^{9b}; or -C(=O)-R^{10b},
more preferably R^{1b} represents a hydrogen atom.

37. Use according to one or more of claims 34-36, **characterized in that**
R^{2b} represents -H; -NO₂; -NH₂; -SH; -OH; -CN; -CF₃; -OCH₃; -O-CH₂-CH₃; F; Cl; Br; I; a linear or branched, saturated or unsaturated C₁₋₁₀ aliphatic radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10-membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R^{2b} represents -H; -NO₂; -NH₂; -SH; -OH; -CN; -CF₃; -OCH₃; -0-CH₂-CH₃; F; Cl; Br; I; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of linear or branched C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R^{2b} represents a hydrogen atom.

38. Use according to one or more of claims 34-37, **characterized in that** for
R^{3b} and R^{4b}, identical or different, represent a hydrogen atom or a linear or branched C₁₋₈ alkyl radical, or
R^{3b} and R^{4b} together with the bridging nitrogen form an optionally at least mono-substituted, saturated, unsaturated or aromatic 4- to 9-membered heterocyclic ring that may be condensed with an optionally at least mono-substituted mono- or bicyclic ring-system,
whereby the rings of the ring system are 5- 6- or 7-membered and whereby the heterocyclic ring and one or both of the rings of the ring system may contain 1, 2 or 3 heteroatoms independently selected from the group consisting of nitrogen, oxygen and sulphur,
preferably
R^{3b} and R^{4b}, identical or different, represent a hydrogen atom or an alkyl radical selected from the group consisting of methyl, ethyl, n-propyl, iso-propyl, n-butyl, sec-butyl, iso-butyl and tert-butyl; or
R^{3b} and R^{4b} together with the bridging nitrogen atom form a moiety selected from the group consisting of 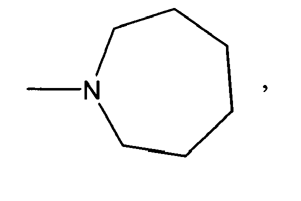 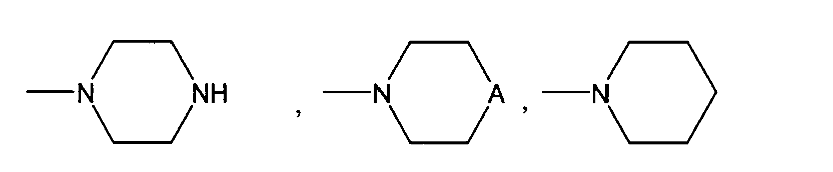 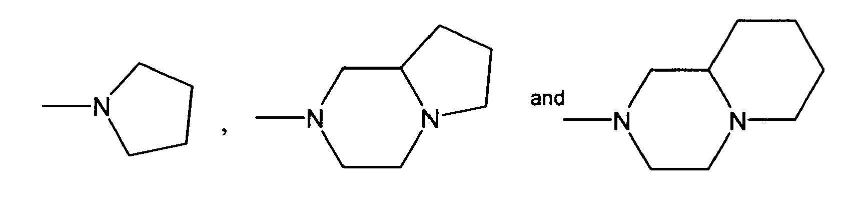 whereby A represents an oxygen atom or a sulphur atom and whereby each of these afore mentioned cyclic moieties may be substituted with 1, 2 or 3 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl in any position including the nitrogen atoms of the piperazine ring,
more preferably R^{3b} and R^{4b}, identical or different, represent a hydrogen atom or an alkyl radical selected from the group consisting of methyl, ethyl, n-propyl, iso-propyl, n-butyl, sec-butyl, iso-butyl and tert-butyl.

39. Use according to one or more of claims 34-38, **characterized in that**
R^{5b}, R^{6b}, R^{7b} and R^{8b}, identical or different, represent -H; -NO₂; -CN; -N(R^{11b})-S(=O)₂-R^{12b}; -OR^{13b}; -SR^{14b}; -C(=O)-OR^{15b}; -NR^{16b}R^{17b}; -C(=O)-R^{18b}; -(C=O)-NR^{19b}R^{20b}; -O-(C=O)-R^{21b}; -S(=O)₂-R^{22b}; -S(=O)₂-NR^{23b}R^{24b}; -NR^{25b-}C(=O)-NR^{26b}R^{27b}; -NR^{28b}-(C=O)-R^{29b}; -NR^{30b}-(C=O)-OR^{31b}; -NR^{32b-}S(=O)NR^{33b}R^{34b}; or -S(=O)₂-OR^{35b}; F, Cl, Br, I; -CF₃, -CHF₂, -CH₂F, a linear or branched, saturated or unsaturated C₁₋₁₀ aliphatic radical; a saturated or unsaturated, optionally at least mono-substituted, 3- to 9-membered cycloaliphatic radical, which may be bonded via a linear or branched C₁₋₆₋alkylene group and/or which may contain 1, 2 or 3 heteroatoms independently selected from the group consisting of nitrogen, oxygen and sulphur as ring members; or an optionally at least mono-substituted 5- to 10- membered aryl or heteroaryl radical, which may be bonded via a linear or branched C₁₋₆₋alkylene group and wherein the heteroaryl radical contains 1, 2 or 3 heteroatoms independently selected from the group consisting of nitrogen, oxygen and sulphur as ring members,
preferably R^{5b}, R^{6b}, R^{7b} and R^{8b}, identical or different, represent -H; -NO₂; -CN; -N(R^{11b})-SO₂-R^{12b}; -OR^{13b}; -SR^{14b}; -C(=O)-OR^{15b}; -NR^{16b}R^{17b}; -C(=O)-R^{18b}; -(C=O)-NR^{19b}R^{20b}; -O-(C=O)-R^{21b}; -S(=O)₂-R^{22b}; -S(=O)₂-NR^{23b}R^{24b}; -NR^{25b}-C(=O)-NR^{26b}R^{27b}; -NR^{28b}-(C=O)-R^{29b}; -NR^{30b}-(C=O)-OR^{31b}; -NR^{32b-}S(=O)NR^{33b}R^{34b}; or -S(=O)₂-OR^{35b}; F, Cl, Br, I; -CF₃, -CHF₂, -CH₂F, an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
more preferably R^{5b}, R^{6b}, R^{7b} and R^{8b}, identical or different, each represent -H; -NO₂; -CN; -N(R^{11b})-SO₂-R^{12b}; -OR^{13b}; -SR^{14b}; -C(=O)-OR^{15b}; -NR^{16b}R^{17b}; - F, Cl, Br, I; -CF₃, -CHF₂, -CH₂F, an alkyl radical selected from the group consisting of methyl, ethyl, n-propyl, iso-propyl, n-butyl, sec-butyl, iso-butyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3-}group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
in each case with the proviso that at least one of the substituents R^{5b}, R^{6b}, R^{7b} and R^{8b} represents a -N(R^{11b})-S(=O)₂-R^{12b} moiety.

40. Use according to one or more of claims 34-39, **characterized in that** one of the substituents R^{5b}, R^{6b}, R^{7b} and R^{8b} represents an -N(R^{11b})-S(=O)₂-R^{12b} moiety.

41. Use according to claim 40, **characterized in that** one of the substituents R^{5b}, R^{6b}, R^{7b} and R^{8b} represents an -N(R^{11b})-S(=O)₂-R^{12b} moiety while the other three of these substituents each represent a hydrogen atom.

42. Use according to one or more of claims 34-41, **characterized in that**
R^{9b} and R^{10b}, independent from one another, represent a linear or branched, saturated or unsaturated C₁₋₁₀ aliphatic radical; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members,
preferably R^{9b} and R^{10b}, independent from one another, represent an alkyl radical selected from the group consisting of methyl, ethyl, n-propyl, iso-propyl, n-butyl, sec-butyl, iso-butyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅₋alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl.

43. Use according to one or more of claims 34-42, **characterized in that**
R^{11b} represents a hydrogen atom; a linear or branched, saturated or unsaturated, optionally at least mono-substituted C₁₋₁₀-aliphatic radical; a saturated or unsaturated, optionally at least mono-substituted, 3- to 9-membered cycloaliphatic radical, which may be bonded via a linear or branched C₁₋₆-alkylene, C₂₋₆-alkenylene or C₂₋₆-alkinylene group and/or which may contain 1, 2 or 3 heteroatoms independently selected from the group consisting of nitrogen, oxygen and sulphur as ring members; an optionally at least mono-substituted 5- to 10- membered aryl or heteroaryl radical, which may be bonded via a linear or branched C₁₋₆-alkylene, C₂₋₆-alkenylene or C₂₋₆₋alkinylene group and wherein the heteroaryl radical contains 1, 2 or 3 heteroatoms independently selected from the group consisting of nitrogen, oxygen and sulphur as ring members; or an -S(=O)₂-R^{36b} moiety,
preferably R^{11b} represents a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or an -S(=O)₂-R^{36b}-moiety,
more preferably R^{11b} represents a hydrogen atom.

44. Use according to one or more of claims 34-43, **characterized in that** R^{12b} represents an optionally at least mono-substituted 5- to 10- membered aryl or heteroaryl radical, which may be bonded via a linear or branched, optionally at least mono-substituted C₁₋₆-alkylene, C₂₋₆-alkenylene or C₂₋₆-alkinylene group and/or which may be condensed with an optionally at least mono-substituted, saturated, unsaturated or aromatic, mono- or bicyclic ring system,
whereby the rings of the ring system are 5- 6- or 7-membered and whereby the heteroaryl radical and optionally one or both of the rings of the ring system contain 1, 2 or 3 heteroatoms independently selected from the group consisting of nitrogen, oxygen and sulphur,
or R^{12b} represents a saturated or unsaturated, optionally at least mono-substituted, 3- to 9-membered cycloaliphatic radical, which may be bonded via a linear or branched C₁₋₆-alkylene, C₂₋₆-alkenylene or C₂₋₆-alkinylene group and/or which may be condensed with an optionally at least mono-substituted mono- or polycyclic ring system,
whereby the rings of the ring system are 5- 6- or 7-membered and whereby the cycloaliphatic radical and optionally one or both of the rings of the ring system may contain 1, 2 or 3 heteroatoms independently selected from the group consisting of nitrogen, oxygen and sulphur,
preferably R^{12b} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, 2-oxo-2,3-dihydro-benzooxazolyl, 2-oxo-2,3-dihydrobenzo[d]thiazolyl, benzooxazolinyl, benzothiazolinyl, benzimidazolidinyl, imidazo[2,1-b]thiazolyl, chromenyl and chromanyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl.
more preferably R^{12b} represents an aryl or heteroaryl radical selected from the group consisting of phenyl, 1-naphthyl, 2-naphthyl, benzo[b]-thiophenyl, benzo[b]furanyl, quinolinyl, isoquinolinyl, imidazo[2,1-b]thiazolyl, 2-oxo-2,3-dihydro-benzooxazolyl and 2-oxo-2,3-dihydrobenzo[d]thiazolyl, whereby said aryl or heteroaryl radical may be substituted by 1, 2, 3, 4 or 5 substituents independently selected from the group consisting of methyl, ethyl, n-propyl, iso-propyl, n-butyl, sec-butyl, iso-butyl, tert-butyl, F, Cl, Br, I, -CN, -CF₃, -CF₂H, CFH₂, -C(=O)-O-CH₃, C(=O)-O-CH₂-CH₃, cyclopropyl, cyclobutyl, cyclopentyl, cyclohexyl, phenyl, phenoxy and benzyl.

45. Use according to one or more of claims 34-44, **characterized in that** R^{13b}-R^{35b} independent from one another, each represent a hydrogen atom; a linear or branched C₁₋₁₀ alkyl radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatoms independently selected from the group consisting of nitrogen, oxygen and sulphur as ring members; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatoms independently selected from the group consisting of nitrogen, oxygen and sulphur as ring members;
preferably R^{13b}-R^{35b} independent from one another, represent a hydrogen atom; an alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl- (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl and imidazo[2,1-b]thiazolyl, whereby said aryl or heteroaryl radical may be bonded via a -(CH₂)_{1, 2 or 3-}group and/or substituted with 1, 2, 3, 4 or 5 substituents independently selected from the group consisting of C₁₋₅-alkyl, -O-C₁₋₅-alkyl, -S-C₁₋₅-alkyl, -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl.

46. Use according to one or more of claims 34-45, **characterized in that** R^{36b} represents an optionally at least mono-substituted 5- to 10- membered aryl or heteroaryl radical, which may be bonded via a linear or branched, optionally at least mono-substituted C₁₋₆-alkylene, C₂₋₆-alkenylene or C₂₋₆-alkinylene group and/or which may be condensed with an optionally at least mono-substituted, saturated, unsaturated or aromatic, mono- or bicyclic ring system,
whereby the rings of the ring system are 5- 6- or 7-membered and whereby the heteroaryl radical and optionally one or both of the rings of the ring system contain 1, 2 or 3 heteroatoms independently selected from the group consisting of nitrogen, oxygen and sulphur,
or R^{36b} represents a saturated or unsaturated, optionally at least mono-substituted, 3- to 9-membered cycloaliphatic radical, which may be bonded via a linear or branched C₁₋₆-alkylene, C₂₋₆-alkenylene or C₂₋₆-alkinylene group and/or which may be condensed with an optionally at least mono-substituted mono- or polycyclic ring system,
whereby the rings of the ring system are 5- 6- or 7-membered and whereby the cycloaliphatic radical and optionally one or both of the rings of the ring system may contain 1, 2 or 3 heteroatoms independently selected from the group consisting of nitrogen, oxygen and sulphur,
preferably R^{36b} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, 2-oxo-2,3-dihydro-benzooxazolyl, 2-oxo-2,3-dihydrobenzo[d]thiazolyl, benzooxazolinyl, benzothiazolinyl, benzimidazolidinyl, imidazo[2,1-b]thiazolyl, chromenyl and chromanyl, whereby said aryl or heteroaryl radical may be bonded via a -(CH₂)_{1, 2 or 3}- group and/or substituted with 1, 2, 3, 4 or 5 substituents independently selected from the group consisting of C₁₋₅-alkyl, -O-C₁₋₅-alkyl, -S-C₁₋₅-alkyl, oxo (=O), -C(=O)-O-C₁₋₅-alkyl, -O-(C=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical may be bonded via a -(CH₂)_{1, 2 or 3}- group and/or substituted with 1, 2, 3, 4 or 5 substituents independently selected from the group consisting C₁₋₅-alkyl, -0-C₁₋₅-alkyl, -S-C₁₋₅-alkyl, oxo (=O), -C(=O)-O-C₁₋₅-alkyl, -O-(C=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl.

47. Use according to one or more of claims 34-46, **characterized in that** nb is 0.

48. Use according to one or more of claims 34-47, **characterized in that**
nb is 0,
R^{1b} represents a hydrogen atom,
R^{2b} represents a hydrogen atom,
R^{3b} and R^{4b}, identical or different, represent an alkyl radical selected from the group consisting of methyl, ethyl, n-propyl, iso-propyl, n-butyl, sec-butyl, iso-butyl and tert-butyl,
one of the substituents R^{5b}, R^{6b}, R^{7b} and R^{8b} represents an -N(R^{11b})-S(=O)₂₋R^{12b}-moiety while the other three of these substituents each represent a hydrogen atom,
R¹¹ represents a hydrogen atom,
R¹² represents an aryl or heteroaryl radical selected from the group consisting of phenyl, 1-naphthyl, 2-naphthyl, benzo[b]-thiophenyl, benzo[b]furanyl, quinolinyl, isoquinolinyl, imidazo[2,1-b]thiazolyl, 2-oxo-2,3-dihydro-benzooxazolyl and 2-oxo-2,3-dihydrobenzo[d]thiazolyl, whereby said aryl or heteroaryl radical may be substituted by 1, 2, 3, 4 or 5 substituents independently selected from the group consisting of methyl, ethyl, n-propyl, iso-propyl, n-butyl, sec-butyl, iso-butyl, tert-butyl, F, Cl, Br, I, -CN, -CF₃, -CF₂H, CFH₂, -C(=O)-O-CH₃, C(=O)-O-CH₂-CH₃, cyclopropyl, cyclobutyl, cyclopentyl, cyclohexyl, phenyl, phenoxy and benzyl.

49. Use according to one or more of claims 34-48, **characterized in that** the substituted indole compound is selected from the group consisting of
[1] 2-[5-(5-Chloro-3-methyl-benzo[b]thiophene-2-sulfonylamino)-1H-indol-3-yl]-N,N-diethyl-2-oxoacetamide,
[2] N,N-Diethyl-2-[5-(naphthalene-2-sulfonylamino)-1H-indol-3-yl]-2-oxo-acetamide,
[3] N,N-Diethyl-2-[5-(naphthalene-1-sulfonylamino)-1H-indol-3-yl]-2-oxo-acetamide,
[4] 2-[5-(Biphenyl-4-sulfonylamino)-1H-indol-3-yl]-N,N-diethyl-2-ox-acetamide,
[5] N,N-Diethyl-2-oxo-2-[5-(quinoline-8-sulfonylamino)-1H-indol-3-yl]-acetamide,
[6] N,N-Dimethyl-2-[5-(naphthalene-2-sulfonylamino)-1H-indol-3-yl]-2-oxo-acetamide,
[7] N,N-Dimethyl-2-[5-(naphthalene-1-sulfonylamino)-1H-indol-3-yl]-2-oxo-acetamide,
[8] 2-[5-(5-Chloro-3-methyl-benzo[b]thiophene-2-sulfonylamino)-1H-indol-3-yl]-N,N-dimethyl-2-oxo-acetamide,
[9] 2-[5-(6-Chloro-imidazo[2,1-b]thiazole-5-sulfonylamino)-1H-indol-3-yl]-N,N-diethyl-2-oxo-acetamide,
[10] 2-[5-(6-Chloro-imidazo[2,1-b]thiazole-5-sulfonylamino)-1H-indol-3-yl]-N,N-dimethyl-2-oxo-acetamide,
[11] N,N-Dimethyl-2-[4-(naphthalene-1-sulfonylamino)-1H-indol-3-yl]-2-oxo-acetamide,
[12] 2-[4-(5-Chloro-3-methyl-benzo[b]thiophene-2-sulfonylamino)-1H-indol-3-yl]-N,N-dimethyl-2-oxo-acetamide,
[13] 2-[4-(6-Chloro-imidazo[2,1-b]thiazole-5-sulfonylamino)-1H-indol-3-yl]-N,N-dimethyl-2-oxo-acetamide,
[14] N,N-Dimethyl-2-[5-[(4-fluoro-3-methyl-phenyl)-1-sulfonylamino]-1H-indol-3-yl]-2-oxo-acetamide,
[15] 5-(3-Dimethylaminooxalyl-1H-indol-5-ylsulfamoyl)-3-methyl-benzofuran-2-carboxylic acid ethyl ester,
[16] 2-[5-(Biphenyl-4-sulfonylamino)-1H-indol-3-yl]-N,N-dimethyl-2-oxo-acetamide,
[17] N,N-Dimethyl-2-oxo-2-[5-(2-oxo-2,3-dihydro-benzoxazole-6-sulfonylamino)-1 H-indol-3-yl]-acetamide,
[18] N,N-Dimethyl-2-oxo-2-[5-(2-oxo-2,3-dihydrobenzo[d]thiazole-6-sulfonamido)-1 H-indol-3-yl]-acetamide,
[19] 2-[5-[(4-Cyclohexyl-phenyl)-1-sulfonylamino]-1H-indol-3-yl]-N,N-dimethyl-2-oxo-acetamide and
[20] N,N-Dimethyl-2-[5-[(4-phenoxy-phenyl)-1-sulfonylamino]-1H-indol-3-yl]-2-oxo-acetamide.

50. Use according to one or more of claims 34-49 for the manufacture of a medicament for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

51. Use according to one or more of claims 34-49 for the manufacture of a medicament for the prophylaxis and/or treatment of irritable colon syndrome; disorders of the central nervous system; anxiety; panic attacks; depression; bipolar disorders; cognitive disorders; memory disorders; senile dementia; psychosis; neurodegenerative disorders, preferably selected from the group consisting of Morbus Alzheimer, Morbus Parkinson, Morbus Huntington and Multiple Sclerosis; schizophrenia; psychosis; hyperactivity disorder (ADHD, attention deficit/hyperactivity disorder) or for improvement of cognition (cognitive enhancement).

52. Use of at least one compound according to one or more of claims 34-49 for the manufacture of a medicament for the prophylaxis and/or treatment of a disorder or a disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

53. Use of at least one compound according to one or more of claims 34-49 for the manufacture of a medicament for the prophylaxis and/or treatment of disorders of the intestinal tract, preferably for the treatment of irritable colon syndrome; disorders of the central nervous system; anxiety; panic attacks; depression; bipolar disorders; cognitive disorders; memory disorders; senile dementia; psychosis; neurodegenerative disorders, preferably selected from the group consisting of Morbus Alzheimer, Morbus Parkinson, Morbus Huntington and Multiple Sclerosis; schizophrenia; psychosis; hyperactivity disorder (ADHD, attention deficit/hyperactivity disorder) or for improvement of cognition (cognitive enhancement).

54. A substituted indole compound of general formula Ic, 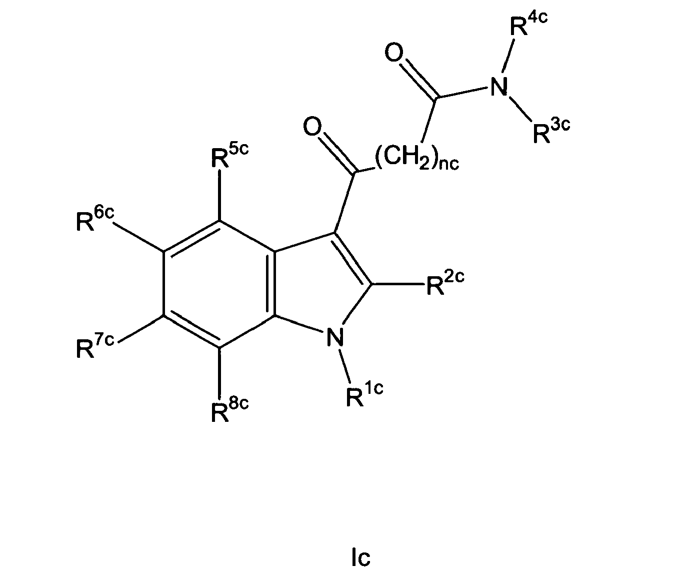 wherein
nc represents 0, 1, 2, 3 or 4,
R^{1c} represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; an optionally at least mono-substituted aryl or heteroaryl radical, which may be bonded via a linear or branched alkylene group; -S(=O)₂-R^{9c}; or -C(=O)-R^{10c},
R^{2c} represents -H, -NO₂; -NH₂; -SH; -OH; -CN; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R^{3c} and R^{4c}, identical or different, represent a hydrogen atom; a linear or branched, saturated or unsaturated aliphatic radical, an optionally at least mono-substituted aryl or heteroaryl radical, which may be bonded via a linear or branched alkylene group; a saturated or unsaturated, optionally at least mono-substituted, optionally at least one heteroatom as a ring member containing cycloaliphatic radical, which may be bonded via a linear or branched alkylene group and/or which may be condensed with an optionally at least mono-substituted mono- or polycyclic ring system, or
R^{3c} and R^{4c} together with the bridging nitrogen form an optionally at least mono-substituted, saturated, unsaturated or aromatic heterocyclic ring that may contain at least one further heteroatom as a ring member and/or that may be condensed with an optionally at least mono-substituted mono- or polycyclic ring-system,
R^{5c}, R^{6c} , R^{7c} and R^{8c}, identical or different, represent -H; -NO₂; -CN; -N(R^{11c})-S(=O)₂-R^{12c}; -OR^{13c}; -SR^{14c}; -C(=O)-OR^{15c}; -NR^{16c}R^{17c}; -C(=O)-R^{18c}; -(C=O)-NR^{19c}R^{20c}; -O-(C=O)-R^{21c}; -S(=O)₂-R^{22c}; -S(=O)₂-NR^{23c}R^{24c}; -NR^{25c-}C(=O)-NR^{26c}R^{27c}; -NR^{28c}-(C=O)-R^{29c}; -NR^{30c}-(C=O)-OR^{31c}; -NR^{32c-}S(=O)NR^{33c}R^{34c}; or -S(=O)₂-OR^{35c}; a halogen atom; a linear or branched, saturated or unsaturated, optionally at least mono-substituted, optionally at least one heteroatom as a chain member containing aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
with the proviso that
one of the substituents R^{5c}, R^{6c}, R^{7c} and R^{8c} represents an -N(R^{11c})-S(=O)₂₋R^{12c} moiety,
R^{9c} and R^{10c}, independent from one another, represent a linear or branched, saturated or unsaturated, optionally at least mono-substituted aliphatic radical; or an optionally at least mono-substituted aryl or heteroaryl radical, which may be bonded via a linear or branched alkylene group,
R^{11c} represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group; an optionally at least mono-substituted aryl or heteroaryl radical, which may be bonded via a linear or branched, optionally at least mono-substituted alkylene, alkenylene or alkinylene group; or an -S(=O)₂-R^{36c} moiety,
R^{12c} represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system; or a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system,
R^{13c}-R^{35c}, independent from one another, represent a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R^{36c} represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system; or a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system,
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

55. A compound according to claim 54, **characterized in that** R^{5c} represents an -N(R^{11c})-SO₂-R^{12c}-moiety.

56. A compound according to claim 54, **characterized in that** R^{6c} represents an -N(R^{11c})-SO₂-R^{12c}-moiety.

57. A compound according to claim 54, **characterized in that** R^{7c} represents an -N(R^{11c})-SO₂-R^{12c}-moiety.

58. A compound according to claim 54, **characterized in that** R^{8c} represents an -N(R^{11c})-SO₂-R^{12c}-moiety.

59. A compound according to one or more of claims 54-58, **characterized in that** nc is 0.

60. Medicament comprising at least one compound according to one or more of claims 54-59 and optionally at least one auxiliary substance.

61. Use of at least one compound according to one or more of claims 54-59 for the preparation of a medicament for the prophylaxis and/or prevention of a disorder or disease that is at least partially mediated via 5-HT₆ receptors.

62. Use according to claim 61 for the manufacture of a medicament for the prophylaxis and/or treatment of a disorder or disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

63. Use according to claim 61 for the manufacture of a medicament for the prophylaxis and/or treatment of irritable colon syndrome; disorders of the central nervous system; anxiety; panic attacks; depression; bipolar disorders; cognitive disorders; memory disorders; senile dementia; psychosis; neurodegenerative disorders, preferably selected from the group consisting of Morbus Alzheimer, Morbus Parkinson, Morbus Huntington and Multiple Sclerosis; schizophrenia; psychosis; hyperactivity disorder (ADHD, attention deficit/hyperactivity disorder) or for improvement of cognition (cognitive enhancement).

64. Use of at least one compound according to one or more of claims 54-59 for the prophylaxis and/or treatment of a disorder or disease that is related to food intake, preferably for the regulation of appetite, for the maintenance, increase or reduction of body weight, for the prophylaxis or treatment of obesity, bulimia, anorexia, cachexia or type II diabetes (non insulin dependent diabetes mellitus), preferably type II diabetes that is caused by obesity.

65. Use of at least one compound according to one or more of claims 54-59 for the manufacture of a medicament for the prophylaxis and/or treatment of irritable colon syndrome; disorders of the central nervous system; anxiety; panic attacks; depression; bipolar disorders; cognitive disorders; memory disorders; senile dementia; psychosis; neurodegenerative disorders, preferably selected from the group consisting of Morbus Alzheimer, Morbus Parkinson, Morbus Huntington and Multiple Sclerosis; schizophrenia; psychosis; hyperactivity disorder (ADHD, attention deficit/hyperactivity disorder) or for improvement of cognition (cognitive enhancement).
